# EUROPEAN PATENT APPLICATION

(11) **EP 4 406 546 A1**
(43) Date of publication of application: **31.07.2024**
(21) Application number: 23799441.3
(22) Date of filing: 19.04.2023
(51) Int. Cl.: A61K 38/12, A61K 9/08, A61K 38/00, A61K 47/12, A61K 47/14, A61K 47/18, A61K 47/20

(54) **COMPOSITION CONTAINING PEPTIDE COMPOUND FOR USE WITH SURFACTANT**

(30) Priority: 02.05.2022 JP 2022075833; 29.08.2022 EP 22192611; 12.10.2022 EP 22200995; 12.10.2022 TW 111138589; 12.10.2022 WO PCT/JP2022/038128
(71) Applicant: CHUGAI SEIYAKU KABUSHIKI KAISHA, Tokyo 115-8543 (JP)
(72) Inventor: NAKAE, Shinichi, Yokohama City, Kanagawa 244-8602 (JP); TAKANO, Ryusuke, Yokohama City, Kanagawa 244-8602 (JP); TANG, Hengmin, Yokohama City, Kanagawa 244-8602 (JP); SAKURAI, Yuji, Yokohama City, Kanagawa 244-8602 (JP)
(74) Representative: Vossius & Partner Patentanwälte Rechtsanwälte mbB
(86) International application number: PCT/JP2023/015658
(87) International publication number: WO 2023/214509

(57) **Abstract**

The present invention relates to a composition comprising a component (1) below, for combined use of a component (2) below:
(1) at least one or more peptide compounds selected from the group consisting of (i), (ii), and (iii) below:
(i) a peptide compound containing one or more N-substituted amino acid residues,
(ii) a peptide compound having ClogP of 4 or more and 25 or less, and
(iii) a peptide compound having a solubility of 10 mg/mL or less at 37°C and 1 atm in 50 mM phosphate buffer (pH 6.5); and

(2) at least one or more surfactants selected from the group consisting of (iv) and (v) below:
(iv) a surfactant having a linear alkylene structure and having 5 or more and 13 or less carbon atoms in the alkylene structure, and
(v) a surfactant having a carnitine residue.

## Description

### Technical Field

The present invention relates to a composition containing a peptide compound, for combined use of a surfactant.

### Background Art

In recent years, the development of drug discovery technology that makes it possible to discover drugs toward tough targets such as protein-protein interaction inhibition, agonists, and molecular chaperones by using medium molecular compounds (e.g., having molecular weights of 500 to 2,000 g/mol) has gained attention.

It is generally believed that compounds having a molecular weight of 500 g/mol or more have low membrane permeability and problems in absorbability. The same holds for the peptide compound, which is an example of the medium molecular weight compounds. As strategies to improve the membrane permeability of peptide compounds, a peptide compound containing an N-substituted amino acid residue (e.g., an N-methylamino acid residue) as a component, a peptide compound containing a cyclic structure, or the like, have been created (e.g., Patent Literature 1). In addition, a combination of a peptide compound with a surfactant has been attempted to improve absorbability. For example, Patent Literature 2 discloses a finished medicament adapted for oral delivery, containing a physiologically active peptide agent, at least one pharmaceutically acceptable pH-lowering agent, and at least one absorption-promoting agent effective to enhance the bioavailability of the active agent.

### Citation List

### Patent Literature

Patent Literature 1: International Publication No. WO 2013/100132
Patent Literature 2: Japanese Translation of PCT International Application Publication No. JP 2009-518437

### Summary of Invention

### Technical Problem

Since the method of improving the absorbability by combining a peptide compound with a surfactant, such as lauroylcarnitine, as disclosed in Patent Literature 2, does not need to change the structure of the peptide compound itself, it can avoid affecting the function of the peptide compound, such as the binding ability to the target protein. Meanwhile, a combination of a peptide compound described in (1) below or the like and a surfactant, which enhances the absorbability, has not been known to date.

It is an object of the present invention to provide a composition containing a peptide compound and a surfactant, having enhanced membrane permeability and/or absorbability of the peptide compound. It is another object of the present invention to provide a composition containing a peptide compound, for combined use of a surfactant. It is a further object of the present invention to provide a method of using a surfactant for improving membrane permeability and/or absorbability of a peptide compound.

### Solution to Problem

The present invention relates to, for example, each of the following inventions.
[1] A composition comprising a component (1) below, for combined use of a component (2) below:
   (1) at least one or more peptide compounds selected from the group consisting of (i), (ii), and (iii) below:
      (i) a peptide compound containing one or more N-substituted amino acid residues,
      (ii) a peptide compound having ClogP of 4 or more and 25 or less, and
      (iii) a peptide compound having a solubility of 10 mg/mL or less at 37°C and 1 atm in 50 mM phosphate buffer (pH 6.5); and
   (2) at least one or more surfactants selected from the group consisting of (iv) and (v) below:
      (iv) a surfactant having a linear alkylene structure and having 5 or more and 13 or less carbon atoms in the alkylene structure, and
      (v) a surfactant having a carnitine residue.
[2] A composition comprising a component (2) below, for combined use of a component (1) below:
   (1) at least one or more peptide compounds selected from the group consisting of (i), (ii), and (iii) below:
      (i) a peptide compound containing one or more N-substituted amino acid residues,
      (ii) a peptide compound having ClogP of 4 or more and 25 or less, and
      (iii) a peptide compound having a solubility of 10 mg/mL or less at 37°C and 1 atm in 50 mM phosphate buffer (pH 6.5); and
   (2) at least one or more surfactants selected from the group consisting of (iv) and (v) below:
      (iv) a surfactant having a linear alkylene structure and having 5 or more and 13 or less carbon atoms in the alkylene structure, and
      (v) a surfactant having a carnitine residue.
[3] The composition according to [1] or [2], further comprising (3) a solubility improver.
[4] The composition according to [3], wherein a content of the solubility improver is 0.05% by volume or more and 100% by volume or less based on 100% by volume of a liquid component contained in the composition.
[5] The composition according to [3] or [4], wherein a content of the solubility improver is 0.3% by volume or more and 30% by volume or less, preferably 0.5% by volume or more and 15% by volume or less, more preferably 0.8% by volume or more and 10% by volume or less based on 100% by volume of a liquid component contained in the composition.
[6] The composition according to any one of [3] to [5], wherein the solubility improver contains a polyoxyethylene structure.
[7] The composition according to [6], wherein an average number of moles of ethylene oxide added in the solubility improver is 2 or more and 100 or less.
[8] The composition according to any one of [3] to [7], wherein the solubility improver is a polyoxyethylene castor oil or a polyoxyethylene sorbitan fatty acid ester.
[9] The composition according to any one of [3] to [8], wherein the solubility improver comprises a polymer.
[10] The composition according to [9], wherein the polymer forms a solid dispersion with the peptide compound.
[11] The composition according to [9] or [10], wherein the polymer is selected from the group consisting of polyethylene glycol, polyvinyl pyrrolidone, copovidone, polyvinyl alcohol, a cellulose-based polymer, and a methacrylic acid methacrylic acid copolymer.
[12] The composition according to [9] or [10], wherein the polymer is selected from the group consisting of polyvinyl alcohol, a polyvinyl alcohol polyvinyl acetate copolymer, polyvinyl pyrrolidone, copovidone, a copolymer of acrylate and methacrylic acid, a polyethylene polyvinyl alcohol copolymer, a polyoxyethylene-polyoxypropylene block copolymer (also called as poloxamer), polyethylene glycol, hydroxypropyl methyl cellulose acetate (HPMCA), hydroxypropyl methyl cellulose (HPMC), hydroxypropyl cellulose (HPC), methylcellulose, hydroxyethyl methyl cellulose, hydroxyethyl cellulose, hydroxyethyl cellulose acetate, hydroxyethyl ethyl cellulose, cellulose acetate phthalate, cellulose acetate trimellitate, cellulose acetate succinate, methyl cellulose phthalate, hydroxymethyl cellulose ethyl phthalate, hydroxypropyl methyl cellulose phthalate, hydroxypropyl methyl cellulose acetate succinate (HPMCAS), hydroxypropyl methyl acetate maleate, hydroxypropyl methyl trimellitate, carboxymethyl ethyl cellulose, polyvinyl butylate phthalate, polyvinyl alcohol acetate phthalate, a methacrylic acid/ethyl acrylate copolymer (preferably in a mass ratio of 1 : 99 to 99 : 1), a methacrylic acid/methyl methacrylate copolymer (preferably in a mass ratio of 1 : 99 to 99 : 1), a methacrylic acid copolymer, aminoalkyl methacrylic acid copolymer E, and polyvinyl acetal diethyl aminoacetate.
[13] The composition according to [9] or [10], wherein the polymer is hydroxypropyl methyl cellulose acetate (HPMCA).
[14] The composition according to any one of [1] to [13], wherein an amount of the surfactant to be used in combination is 0.05 parts by mass or more and 300 parts by mass or less based on 1 part by mass of the peptide compound.
[15] The composition according to any one of [1] to [14], wherein an amount of the surfactant to be used in combination is 0.075 parts by mass or more and 80 parts by mass or less, preferably 0.1 parts by mass or more and 60 parts by mass or less, more preferably 0.2 parts by mass or more and 40 parts by mass or less, and further preferably 0.3 parts by mass or more and 30 parts by mass or less based on 1 part by mass of the peptide compound.
[16] The composition according to any one of [1] to [15], wherein a substituent on a nitrogen atom of the N-substituted amino acid residue is a C₁-C₆ alkyl group.
[17] The composition according to [16], wherein the substituent on the nitrogen atom of the N-substituted amino acid residue is at least one selected from the group consisting of a methyl group and an ethyl group.
[18] The composition according to [16], wherein the substituent on the nitrogen atom of the N-substituted amino acid residue is a methyl group.
[19] The composition according to any one of [1] to [18], wherein the peptide compound is a cyclic peptide compound.
[20] The composition according to [19], wherein the number of amino acid residues constituting a cyclic portion of the cyclic peptide compound is 5 or more and 15 or less.
[21] The composition according to [19] or [20], wherein the number of amino acid residues constituting a cyclic portion of the cyclic peptide compound is 6 or more and 14 or less, preferably 7 or more and 14 or less, more preferably 8 or more and 12 or less, and further preferably 9 or more and 11 or less.
[22] The composition according to any one of [1] to [21], wherein a molecular weight of the peptide compound is 5,000 g/mol or less.
[23] The composition according to any one of [1] to [22], wherein a molecular weight of the peptide compound is 2000 g/mol or less.
[24] The composition according to any one of [1] to [23], wherein a molecular weight of the peptide compound is 500 g/mol or more.
[25] The composition according to any one of [1] to [24], wherein a molecular weight of the peptide compound is 1,000 g/mol or more, preferably 1,100 g/mol or more, and more preferably 1,200 g/mol or more.
[26] The composition according to any one of [1] to [25], wherein the surfactant is represented by any one of (a1) to (a3) below: 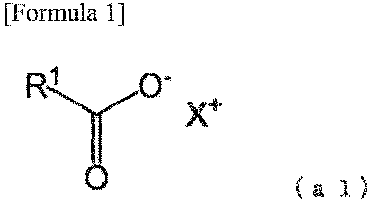 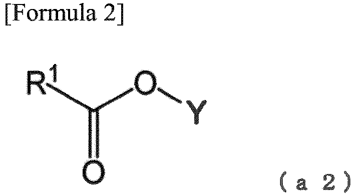 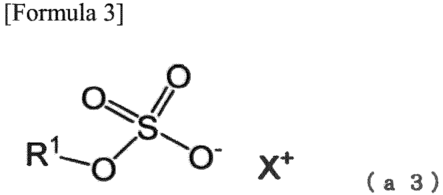
   wherein R¹ represents an optionally substituted, saturated or unsaturated, linear alkyl group having 5 or more and 13 or less carbon atoms, X represents sodium or potassium, and Y represents a group represented by formula (a4) below or a stereoisomer thereof: 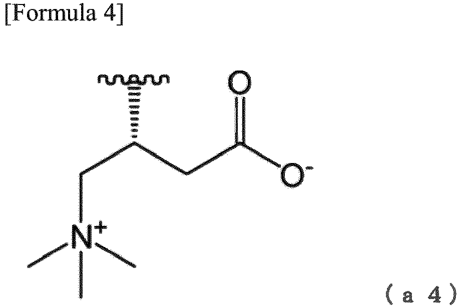
   Wherein 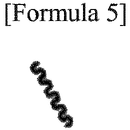 represents a bond.
[27] The composition according to [26], wherein the R¹ is a saturated linear alkyl group having 7 or more and 13 or less carbon atoms.
[28] The composition according to [26], wherein the R¹ is an unsubstituted linear alkyl group.
[29] The composition according to [26], wherein the R¹ represents a linear alkyl group having 8 or more and 12 or less carbon atoms.
[30] The composition according to [26], wherein the R¹ represents a linear alkyl group having 10 or more and 12 or less carbon atoms.
[31] The composition according to any one of [1] to [30], wherein the surfactant is a medium-chain fatty acid ester, a sodium salt of medium-chain fatty acid, or a potassium salt of medium-chain fatty acid.
[32] The composition according to any one of [1] to [31], wherein the surfactant is acylcarnitine.
[33] The composition according to any one of [1] to [32], wherein the surfactant is lauroyl-L-carnitine.
[34] The composition according to any one of [1] to [33], wherein a value of Caco-2 Papp (cm/sec) of the peptide compound is 1.0E-9 or more as measured in a system in which the surfactant is present.
[35] The composition according to any one of [1] to [34], wherein a value of Caco-2 Papp (cm/sec) of the peptide compound is 1.0E-7 or more as measured in a system in which the surfactant is present.
[36] The composition according to any one of [1] to [35], which is a pharmaceutical composition.
[37] The composition according to [36], which contains the peptide compound as an active ingredient.
[38] The composition according to any one of [1] to [37], further comprising a pharmaceutically acceptable excipient.
[39] The composition according to any one of [1] to [38], further comprising a pharmaceutically acceptable carrier.
[40] The composition according to any one of [1] to [39], wherein the N-substituted amino acid residue is a non-natural N-substituted amino acid residue.
[41] The composition according to any one of [1] to [40], wherein the number of amino acid residues of the peptide compound is 5 or more and 30 or less.
[42] The composition according to any one of [1] to [41], wherein the number of amino acid residues of the peptide compound is 7 or more and 25 or less, preferably 8 or more and 15 or less, and more preferably 9 or more and 13 or less.
[43] The composition according to any one of [1] to [42], wherein a ClogP/number of amino acid residues of the peptide compound is 1.0 or more.
[44] The composition according to any one of [1] to [43], wherein the surfactant is lauroylcamitine.
[45] The composition according to any one of [1] to [44], wherein the surfactant is a cationic surfactant.
[46] The composition according to any one of [1] to [45], wherein the surfactant is an isolated component.
[47] The composition according to any one of [1] to [46], wherein a value of Caco-2 Papp (cm/sec) of the peptide compound as measured in a system in which the surfactant is present is 2-fold or more the value as measured in a system in which the surfactant is not present.
[48] The composition according to any one of [1] to [47], wherein a value of Caco-2 Papp (cm/sec) of the peptide compound as measured in a system in which the surfactant is present is 3-fold or more, preferably 5-fold or more, and more preferably 10-fold or more the value as measured in a system in which the surfactant is not present.
[49] The composition according to any one of [1] to [48], which is a composition for administration.
[50] The composition according to any one of [1] to [49], which is a composition for oral administration.
[51] The composition according to any one of [1] to [50], which is a composition for promoting absorption of a peptide compound.
[52] The composition according to any one of [1] to [51], wherein a pharmacologically active substance is the peptide compound.
[53] The composition according to any one of [1] to [52], wherein the peptide compound contains 2 or more, preferably 3 or more, more preferably 4 or more, and further preferably 5 or more N-substituted amino acids.
[54] The composition according to any one of [1] to [53], wherein ClogP of the peptide compound is 6 or more and 23 or less, preferably 8 or more and 21 or less, and more preferably 9 or more and 20 or less.
[55] The composition according to any one of [1] to [54], wherein the ClogP/number of amino acid residues is 1.0 or more and 1.8 or less, and preferably 1.1 or more and 1.6 or less.
[56] The composition according to any one of [1] to [55], wherein a solubility of the peptide compound at 37°C and 1 atm in 50 mM phosphate buffer (pH 6.5) is 5 mg/mL or less, preferably 2.5 mg/mL or less, and more preferably 2 mg/mL or less.
[57] The composition according to any one of [1] to [56], wherein the surfactant has a linear alkylene structure of 6 or more and 13 or less, preferably 8 or more and 12 or less, more preferably 10 or more and 12 or less, and more preferably 11 carbon atoms.
[58] A kit comprising a combination of the composition according to any one of [1] to [57] and a surfactant.
[59] The kit according to [58], further comprising an instruction manual or a package insert.

The composition according to [1] described above includes the compositions of the following aspects.
[1-1] A composition comprising a component (1) below, for combined use of a component (2) below:
   (1) a peptide compound containing one or more N-substituted amino acid residues;
   (2) a surfactant having a linear alkylene structure and having 5 or more and 13 or less carbon atoms in the alkylene structure.
[1-2] The composition according to [1-1], wherein ClogP of the peptide compound is 4 or more and 25 or less.
[1-3] The composition according to [1-1] or [1-2], wherein a solubility of the peptide compound at 37°C and 1 atm in 50 mM phosphate buffer (pH 6.5) is 10 mg/mL or less.
[1-4] The composition according to any one of [1-1] to [1-3], wherein the surfactant has a carnitine residue.
[2-1] A composition comprising a component (1) below, for combined use of a component (2) below:
   (1) a peptide compound having ClogP of 4 or more and 25 or less;
   (2) a surfactant having a linear alkylene structure and having 5 or more and 13 or less carbon atoms in the linear alkylene structure.
[2-2] The composition according to [2-1], wherein the peptide compound contains one or more N-substituted amino acid residues.
[2-3] The composition according to [2-1] or [2-2], wherein a solubility of the peptide compound at 37°C and 1 atm in 50 mM phosphate buffer (pH 6.5) is 10 mg/mL or less.
[2-4] The composition according to any one of [2-1] to [2-3], wherein the surfactant has a carnitine residue.
[3-1] A composition comprising a component (1) below, for combined use of a component (2) below:
   (1) a peptide compound having a solubility of 10 mg/mL or less at 37°C and 1 atm in 50 mM phosphate buffer (pH 6.5);
   (2) a surfactant having a linear alkylene structure and having 5 or more and 13 or less carbon atoms in the linear alkylene structure.
[3-2] The composition according to [3-1], wherein the peptide compound contains one or more N-substituted amino acid residues.
[3-3] The composition according to [3-1] or [3-2], wherein ClogP of the peptide compound is 4 or more and 25 or less.
[3-4] The composition according to any one of [3-1] to [3-3], wherein the surfactant has a carnitine residue.
[4-1] A composition comprising a component (1) below, for combined use of a component (2) below:
   (1) a peptide compound containing one or more N-substituted amino acid residues;
   (2) a surfactant having a carnitine residue.
[4-2] The composition according to [4-1], wherein ClogP of the peptide compound is 4 or more and 25 or less.
[4-3] The composition according to [4-1] or [4-2], wherein a solubility of the peptide compound at 37°C and 1 atm in 50 mM phosphate buffer (pH 6.5) is 10 mg/mL or less.
[4-4] The composition according to any one of [4-1] to [4-3], wherein the surfactant has a linear alkylene structure and the number of carbon atoms in the alkylene structure is 5 or more and 13 or less.
[5-1] A composition comprising a component (1) below, for combined use of a component (2) below:
   (1) a peptide compound having ClogP of 4 or more and 25 or less,
   (2) a surfactant having a carnitine residue.
[5-2] The composition according to [5-1], wherein the peptide compound contains one or more N-substituted amino acid residues.
[5-3] The composition according to [5-1] or [5-2], wherein a solubility of the peptide compound at 37°C and 1 atm in 50 mM phosphate buffer (pH 6.5) is 10 mg/mL or less.
[5-4] The composition according to any one of [5-1] to [5-3], wherein the surfactant has a linear alkylene structure and the number of carbon atoms in the alkylene structure is 5 or more and 13 or less.
[6-1] A composition comprising a component (1) below, for combined use of a component (2) below:
   (1) a peptide compound having a solubility of 10 mg/mL or less at 37°C and 1 atm in 50 mM phosphate buffer (pH 6.5);
   (2) a surfactant having a carnitine residue.
[6-2] The composition according to [6-1], wherein the peptide compound contains one or more N-substituted amino acid residues.
[6-3] The composition according to [6-1] or [6-2], wherein ClogP of the peptide compound is 4 or more and 25 or less.
[6-4] The composition according to any one of [6-1] to [6-3], wherein the surfactant has a linear alkylene structure and the number of carbon atoms in the alkylene structure is 5 or more and 13 or less.

The present invention also relates to, for example, each of the following inventions.
[A1] A method for improving absorbability of a peptide compound (1) below, comprising
   using in combination a composition containing the peptide compound (1) below and a surfactant (2) below:
   (1) at least one or more peptide compounds selected from the group consisting of (i), (ii), and (iii) below:
      (i) a peptide compound containing one or more N-substituted amino acid residues,
      (ii) a peptide compound having ClogP of 4 or more and 25 or less, and
      (iii) a peptide compound having a solubility of 10 mg/mL or less at 37°C and 1 atm in 50 mM phosphate buffer (pH 6.5); and
   (2) at least one or more surfactants selected from the group consisting of (iv) and (v) below:
      (iv) a surfactant having a linear alkylene structure and having 5 or more and 13 or less carbon atoms in the alkylene structure, and
      (v) a surfactant having a carnitine residue.
[A2] A method for improving absorbability of a peptide compound (1) below, comprising using in combination the peptide compound (1) below and a surfactant (2) below:
   (1) at least one or more peptide compounds selected from the group consisting of (i), (ii), and (iii) below:
      (i) a peptide compound containing one or more N-substituted amino acid residues,
      (ii) a peptide compound having ClogP of 4 or more and 25 or less, and
      (iii) a peptide compound having a solubility of 10 mg/mL or less at 37°C and 1 atm in 50 mM phosphate buffer (pH 6.5); and
   (2) at least one or more surfactants selected from the group consisting of (iv) and (v) below:
      (iv) a surfactant having a linear alkylene structure and having 5 or more and 13 or less carbon atoms in the alkylene structure, and
      (v) a surfactant having a carnitine residue.
[A3] The method according to [A1], wherein the composition further comprises (3) a solubility improver.
[A4] The method according to [A3], wherein a content of the solubility improver is 0.05% by volume or more and 100% by volume or less based on 100% by volume of a liquid component contained in the composition.
[A5] The method according to [A3] or [A4], wherein a content of the solubility improver is 0.3% by volume or more and 30% by volume or less, preferably 0.5% by volume or more and 15% by volume or less, more preferably 0.8% by volume or more and 10% by volume or less based on 100% by volume of a liquid component contained in the composition.
[A6] The method according to any one of [A3] to [A5], wherein the solubility improver contains a polyoxyethylene structure.
[A7] The method according to [A6], wherein an average number of moles of ethylene oxide added in the solubility improver is 2 or more and 100 or less.
[A8] The method according to any one of [A3] to [A7], wherein the solubility improver is a polyoxyethylene castor oil or a polyoxyethylene sorbitan fatty acid ester.
[A9] The method according to any one of [A3] to [A8], wherein the solubility improver comprises a polymer.
[A10] The method according to [A9], wherein the polymer forms a solid dispersion with the peptide compound.
[A11] The method according to [A9] or [A10], wherein the polymer is selected from the group consisting of polyethylene glycol, polyvinyl pyrrolidone, copovidone, polyvinyl alcohol, a cellulose-based polymer, and a methacrylic acid methacrylic acid copolymer.
[A12] The method according to [A9] or [A10], wherein the polymer is selected from the group consisting of polyvinyl alcohol, a polyvinyl alcohol polyvinyl acetate copolymer, polyvinyl pyrrolidone, copovidone, a copolymer of acrylate and methacrylic acid, a polyethylene polyvinyl alcohol copolymer, a polyoxyethylene-polyoxypropylene block copolymer (also called as poloxamer), polyethylene glycol, hydroxypropyl methyl cellulose acetate (HPMCA), hydroxypropyl methyl cellulose (HPMC), hydroxypropyl cellulose (HPC), methylcellulose, hydroxyethyl methyl cellulose, hydroxyethyl cellulose, hydroxyethyl cellulose acetate, hydroxyethyl ethyl cellulose, cellulose acetate phthalate, cellulose acetate trimellitate, cellulose acetate succinate, methyl cellulose phthalate, hydroxymethyl cellulose ethyl phthalate, hydroxypropyl methyl cellulose phthalate, hydroxypropyl methyl cellulose acetate succinate (HPMCAS), hydroxypropyl methyl acetate maleate, hydroxypropyl methyl trimellitate, carboxymethyl ethyl cellulose, polyvinyl butylate phthalate, polyvinyl alcohol acetate phthalate, a methacrylic acid/ethyl acrylate copolymer (preferably in a mass ratio of 1 : 99 to 99 : 1), a methacrylic acid/methyl methacrylate copolymer (preferably in a mass ratio of 1 : 99 to 99 : 1), a methacrylic acid copolymer, aminoalkyl methacrylic acid copolymer E, and polyvinyl acetal diethyl aminoacetate.
[A13] The method according to [A9] or [A10], wherein the polymer is hydroxypropyl methyl cellulose acetate (HPMCA).
[A14] The method according to any one of [A1] to [A13], wherein an amount of the surfactant to be used in combination is 0.05 parts by mass or more and 300 parts by mass or less based on 1 part by mass of the peptide compound.
[A15] The method according to any one of [A1] to [A14], wherein an amount of the surfactant to be used in combination is 0.075 parts by mass or more and 80 parts by mass or less, preferably 0.1 parts by mass or more and 60 parts by mass or less, more preferably 0.2 parts by mass or more and 40 parts by mass or less, and further preferably 0.3 parts by mass or more and 30 parts by mass or less based on 1 part by mass of the peptide compound.
[A16] The method according to any one of [A1] to [A15], wherein a substituent on a nitrogen atom of the N-substituted amino acid residue is a C₁-C₆ alkyl group.
[A17] The method according to [A16], wherein the substituent on the nitrogen atom of the N-substituted amino acid residue is at least one selected from the group consisting of a methyl group and an ethyl group.
[A18] The method according to [A16], wherein the substituent on the nitrogen atom of the N-substituted amino acid residue is a methyl group.
[A19] The method according to any one of [A1] to [A18], wherein the peptide compound is a cyclic peptide compound.
[A20] The method according to [A19], wherein the number of amino acid residues constituting a cyclic portion of the cyclic peptide compound is 5 or more and 15 or less.
[A21] The method according to [A19] or [A20], wherein the number of amino acid residues constituting a cyclic portion of the cyclic peptide compound is 6 or more and 14 or less, preferably 7 or more and 14 or less, more preferably 8 or more and 12 or less, and further preferably 9 or more and 11 or less.
[A22] The method according to any one of [A1] to [A21], wherein a molecular weight of the peptide compound is 5,000 g/mol or less.
[A23] The method according to any one of [A1] to [A22], wherein a molecular weight of the peptide compound is 2,000 g/mol or less.
[A24] The method according to any one of [A1] to [A23], wherein a molecular weight of the peptide compound is 500 g/mol or more.
[A25] The method according to any one of [A1] to [A24], wherein a molecular weight of the peptide compound is 1,000 g/mol or more, preferably 1,100 g/mol or more, and more preferably 1,200 g/mol or more.
[A26] The method according to any one of [A1] to [A25], wherein the surfactant is represented by any one of (a1) to (a3) below: 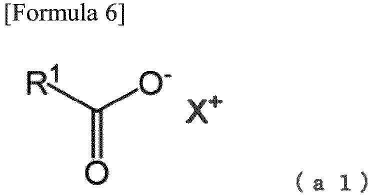 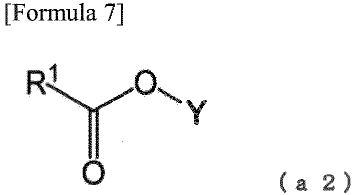 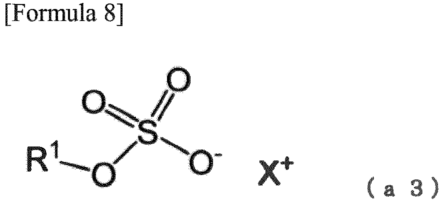
   wherein R¹ represents an optionally substituted, saturated or unsaturated, linear alkyl group having 5 or more and 13 or less carbon atoms, X represents sodium or potassium, and Y represents a group represented by formula (a4) below or a stereoisomer thereof 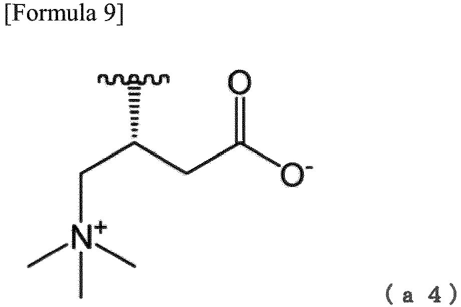
   wherein 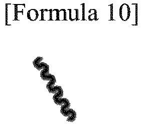 represents a bond.
[A27] The method according to [A26], wherein the R¹ is a saturated linear alkyl group having 7 or more and 13 or less carbon atoms.
[A28] The method according to [A26], wherein the R¹ is an unsubstituted linear alkyl group.
[A29] The method according to [A26], wherein the R¹ represents a linear alkyl group having 8 or more and 12 or less carbon atoms.
[A30] The method according to [A26], wherein the R¹ represents a linear alkyl group having 10 or more and 12 or less carbon atoms.
[A31] The method according to any one of [A1] to [A30], wherein the surfactant is a medium-chain fatty acid ester, a sodium salt of medium-chain fatty acid, or a potassium salt of medium-chain fatty acid.
[A32] The method according to any one of [A1] to [A31], wherein the surfactant is acylcarnitine.
[A33] The method according to any one of [A1] to [A32], wherein the surfactant is lauroyl-L-carnitine.
[A34] The method according to any one of [A1] to [A33], wherein a value of Caco-2 Papp (cm/sec) of the peptide compound is 1.0E-9 or more as measured in a system in which the surfactant is present.
[A35] The method according to any one of [A1] to [A34], wherein a value of Caco-2 Papp (cm/sec) of the peptide compound is 1.0E-7 or more as measured in a system in which the surfactant is present.
[A36] The method according to any one of [A1] and [A3] to [A35], wherein the composition is a pharmaceutical composition.
[A37] The method according to [A36], wherein the pharmaceutical composition contains the peptide compound as an active ingredient.
[A38] The method according to any one of [A1] and [A3] to [A37], wherein the composition further comprises a pharmaceutically acceptable excipient.
[A39] The method according to any one of [A1] and [A3] to [A38], wherein the composition further comprises a pharmaceutically acceptable carrier.
[A40] The method according to any one of [A1] to [A39], wherein the N-substituted amino acid residue is a non-natural N-substituted amino acid residue.
[A41] The method according to any one of [A1] to [A40], wherein the number of amino acid residues of the peptide compound is 5 or more and 30 or less.
[A42] The method according to any one of [A1] to [A41], wherein the number of amino acid residues of the peptide compound is 7 or more and 25 or less, preferably 8 or more and 15 or less, and more preferably 9 or more and 13 or less.
[A43] The method according to any one of [A1] to [A42], wherein a ClogP/number of amino acid residues of the peptide compound is 1.0 or more.
[A44] The method according to any one of [A1] to [A43], wherein the surfactant is lauroylcamitine.
[A45] The method according to any one of [A1] to [A44], wherein the surfactant is a cationic surfactant.
[A46] The method according to any one of [A1] to [A45], wherein the surfactant is an isolated component.
[A47] The method according to any one of [A1] to [A46], wherein a value of Caco-2 Papp (cm/sec) of the peptide compound as measured in a system in which the surfactant is present is 2-fold or more the value as measured in a system in which the surfactant is not present.
[A48] The method according to any one of [A1] to [A47], wherein a value of Caco-2 Papp (cm/sec) of the peptide compound as measured in a system in which the surfactant is present is 3-fold or more, preferably 5-fold or more, and more preferably 10-fold or more the value as measured in a system in which the surfactant is not present.
[A49] The method according to any one of [A1] and [A3] to [A48], wherein the composition is a composition for administration.
[A50] The method according to any one of [A1] and [A3] to [A49], wherein the composition is a composition for oral administration.
[A51] The method according to any one of [A1] and [A3] to [A50], wherein the composition is a composition for promoting absorption of a peptide compound.
[A52] The method according to any one of [A1] to [A51], wherein the peptide compound contains 2 or more, preferably 3 or more, more preferably 4 or more, and further preferably 5 or more N-substituted amino acids.
[A53] The method according to any one of [A1] to [A52], wherein ClogP of the peptide compound is 6 or more and 23 or less, preferably 8 or more and 21 or less, and more preferably 9 or more and 20 or less.
[A54] The method according to any one of [A1] to [A53], wherein the ClogP/number of amino acid residues is 1.0 or more and 1.8 or less, and preferably 1.1 or more and 1.6 or less.
[A55] The method according to any one of [A1] to [A54], wherein a solubility of the peptide compound at 37°C and 1 atm in 50 mM phosphate buffer (pH 6.5) is 5 mg/mL or less, preferably 2.5 mg/mL or less, and more preferably 2 mg/mL or less.
[A56] The method according to any one of [A1] to [A55], wherein the surfactant has a linear alkylene structure of 6 or more and 13 or less, preferably 8 or more and 12 or less, more preferably 10 or more and 12 or less, and more preferably 11 carbon atoms.
[A57] The method according to any one of [A1] and [A3] to [A56], wherein the composition and the surfactant are administered to a mammal.
[A58] The method according to [A57], wherein any one of the composition and the surfactant is administered within 24 hours after administration of the other.
[A59] The method according to [A57], wherein any one of the composition and the surfactant is administered within 12 hours after administration of the other.
[A60] The method according to [A57], wherein any one of the composition and the surfactant is administered within 6 hours after administration of the other.
[A61] The method according to [A57], wherein any one of the composition and the surfactant is administered within 1 hour after administration of the other.
[A62] The method according to any one of [A2] to [A56], further comprising administering the peptide compound and the surfactant to a mammal.
[A63] The method according to [A62], wherein any one of the peptide compound and the surfactant is administered within 24 hours after administration of the other.
[A64] The method according to [A62], wherein any one of the peptide compound and the surfactant is administered within 12 hours after administration of the other.
[A65] The method according to [A62], wherein any one of the peptide compound and the surfactant is administered within 6 hours after administration of the other.
[A66] The method according to [A62], wherein any one of the peptide compound and the surfactant is administered within 1 hour after administration of the other.

The method according to [A1] described above includes the methods of the following aspects.
[A1-1] A method for improving absorbability of a peptide compound (1) below, comprising using in combination a composition containing the peptide compound (1) below and a surfactant (2) below:
   (1) a peptide compound containing one or more N-substituted amino acid residues; and
   (2) a surfactant having a linear alkylene structure and having 5 or more and 13 or less carbon atoms in the alkylene structure.
[A1-2] The method according to [A1-1], wherein ClogP of the peptide compound is 4 or more and 25 or less.
[A1-3] The method according to [A1-1] or [A1-2], wherein a solubility of the peptide compound at 37°C and 1 atm in 50 mM phosphate buffer (pH 6.5) is 10 mg/mL or less.
[A1-4] The method according to any one of [A1-1] to [A1-3], wherein the surfactant has a carnitine residue.
[A2-1] A method for improving absorbability of a peptide compound (1) below, comprising
   using in combination a composition containing the peptide compound (1) below and a surfactant (2) below:
   (1) a peptide compound having ClogP of 4 or more and 25 or less; and
   (2) a surfactant having a linear alkylene structure and having 5 or more and 13 or less carbon atoms in the linear alkylene structure.
[A2-2] The method according to [A2-1], wherein the peptide compound contains one or more N-substituted amino acid residues.
[A2-3] The method according to [A2-1] or [A2-2], wherein a solubility of the peptide compound at 37°C and 1 atm in 50 mM phosphate buffer (pH 6.5) is 10 mg/mL or less.
[A2-4] The method according to any one of [A2-1] to [A2-3], wherein the surfactant has a carnitine residue.
[A3-1] A method for improving absorbability of a peptide compound (1) below, comprising using in combination a composition containing the peptide compound (1) below and a surfactant (2) below:
   (1) a peptide compound having a solubility of 10 mg/mL or less at 37°C and 1 atm in 50 mM phosphate buffer (pH 6.5); and
   (2) a surfactant having a linear alkylene structure and having 5 or more and 13 or less carbon atoms in the linear alkylene structure.
[A3-2] The method according to [A3-1], wherein the peptide compound contains one or more N-substituted amino acid residues.
[A3-3] The method according to [A3-1] or [A3-2], wherein ClogP of the peptide compound is 4 or more and 25 or less.
[A3-4] The method according to any one of [A3-1] to [A3-3], wherein the surfactant has a carnitine residue.
[A4-1] A method for improving absorbability of a peptide compound (1) below, comprising
   using in combination a composition containing the peptide compound (1) below and a surfactant (2) below:
   (1) a peptide compound containing one or more N-substituted amino acid residues; and
   (2) a surfactant having a carnitine residue.
[A4-2] The method according to [A4-1], wherein ClogP of the peptide compound is 4 or more and 25 or less.
[A4-3] The method according to [A4-1] or [A4-2], wherein a solubility of the peptide compound at 37°C and 1 atm in 50 mM phosphate buffer (pH 6.5) is 10 mg/mL or less.
[A4-4] The method according to any one of [A4-1] to [A4-3], wherein the surfactant has a linear alkylene structure and the number of carbon atoms in the alkylene structure is 5 or more and 13 or less.
[A5-1] A method for improving absorbability of a peptide compound (1) below, comprising
   using in combination a composition containing the peptide compound (1) below and a surfactant (2) below:
   (1) a peptide compound having ClogP of 4 or more and 25 or less, and
   (2) a surfactant having a carnitine residue.
[A5-2] The method according to [A5-1], wherein the peptide compound contains one or more N-substituted amino acid residues.
[A5-3] The method according to [A5-1] or [A5-2], wherein a solubility of the peptide compound at 37°C and 1 atm in 50 mM phosphate buffer (pH 6.5) is 10 mg/mL or less.
[A5-4] The method according to any one of [A5-1] to [A5-3], wherein the surfactant has a linear alkylene structure and the number of carbon atoms in the alkylene structure is 5 or more and 13 or less.
[A6-1] A method for improving absorbability of a peptide compound (1) below, comprising
   using in combination a composition containing the peptide compound (1) below and a surfactant (2) below:
   (1) a peptide compound having a solubility of 10 mg/mL or less at 37°C and 1 atm in 50 mM phosphate buffer (pH 6.5); and
   (2) a surfactant having a carnitine residue.
[A6-2] The method according to [A6-1], wherein the peptide compound contains one or more N-substituted amino acid residues.
[A6-3] The method according to [A6-1] or [A6-2], wherein ClogP of the peptide compound is 4 or more and 25 or less.
[A6-4] The method according to any one of [A6-1] to [A6-3], wherein the surfactant has a linear alkylene structure and the number of carbon atoms in the alkylene structure is 5 or more and 13 or less.

### Advantageous Effects of Invention

According to the present invention, a composition containing a peptide compound and a surfactant, having enhanced membrane permeability and absorbability of the peptide compound can be provided. According to the present invention, a composition containing a peptide compound, for combined use of a surfactant can also be provided. According to the present invention, a method of using a surfactant for improving membrane permeability and/or absorbability of a peptide compound can be further provided.

### Brief Description of Drawings

[Figure 1] Figure 1 is a graph showing changes in plasma concentration of each formulation of compound 1 (rat, 30 mg/kg).
[Figure 2] Figure 2 is a graph showing changes in plasma concentration of each formulation of compound 2 (rat, 30 mg/kg).
[Figure 3] Figure 3 is a graph showing changes in plasma concentration of each formulation of compound 3 (rat, 30 mg/kg).

### Description of Embodiments

Hereinafter, the embodiments for carrying out the present invention are described in detail. However, the present invention is not limited to the following embodiments.

As used herein, the term "one or more" means one or two or more in number. When the term "one or more" is used in the context relating to a substituent of a group, the term means the number of from one to the maximum number of the substituents the group can have. Specific examples of the term "one or more" include 1, 2, 3, 4, 5, 6, 7, 8, 9, 10, and/or greater numbers.

As used herein, the term "to" indicating a range includes values at both ends of the range. For example, the term "A to B" means a range that is greater than or equal to A and less than or equal to B.

The term "about", as used herein in combination with a numerical value, means a value range of +10% and -10% of the numerical value.

In the present invention, the meaning of the term "and/or" includes any combination in which "and" and "or" are appropriately combined. Specifically, for example, the term "A, B and/or C" includes the following seven variations; (i) A, (ii) B, (iii) C, (iv) A and B, (v) A and C, (vi) B and C, (vi) B and C, and (vii) A, B and C.

In a non-limiting aspect, the composition according to the present embodiments is a composition including a component (1) below, for combined use of a component (2) below.
(1) at least one or more peptide compounds selected from the group consisting of (i), (ii), and (iii) below:
   (i) a peptide compound containing one or more N-substituted amino acid residues;
   (ii) a peptide compound having ClogP of 4 or more and 25 or less; and
   (iii) a peptide compound having a solubility of 10 mg/mL or less at 37°C and 1 atm in 50 mM phosphate buffer (pH 6.5).
(2) at least one or more surfactants selected from the group consisting of (iv) and (v) below:
   (iv) a surfactant having a linear alkylene structure and having 5 or more and 13 or less carbon atoms in the linear alkylene structure; and
   (v) a surfactant having a carnitine residue.

### [Peptide compounds]

As used herein, the term "peptide compound" is not particularly limited as long as amino acid residues in the peptide compound are linked by an amide bond or an ester bond. The peptide compound is preferably a compound in which two or more amino acid residues in the peptide compound are linked by an amide bond. In this case, a portion of the main chain may have an ester bond, as in depsipeptide. The number of amino acid residues in the peptide compound is not particularly limited, but may be, for example, 5 or more, 7 or more, 8 or more, or 9 or more. The number of amino acid residues of the peptide compound may also be, for example, 30 or less, 25 or less, 15 or less, or 13 or less. The number of amino acid residues of the peptide compound may be, for example, 5 or more and 30 or less, 7 or more and 25 or less, 8 or more and 15 or less, and 9 or more and 13 or less. The number of amino acid residues of the peptide compound may be, for example, 8 or more and 20 or less, and is preferably 9 or more and 15 or less, more preferably 10 or more and 14 or less, and most preferably 11. The peptide compound may have a branched structure.

All terms are used in meanings generally understood in the technical field. The meaning of each term is, but not limited to, as illustrated below.

As used herein, the term "amino acid" includes a natural amino acid and a non-natural amino acid (sometimes also referred to as an amino acid derivative). Also, as used herein, the term "amino acid residue" includes a natural amino acid residue and a non-natural amino acid (amino acid derivative) residue.

Natural amino acids refer to glycine (Gly), L-alanine (Ala), L-serine (Ser), L-threonine (Thr), L-valine (Val), L-leucine (Leu), L-isoleucine (Ile), L-phenylalanine (Phe), L-tyrosine (Tyr), L-tryptophan (Trp), L-histidine (His), L-glutamic acid (Glu), L-aspartic acid (Asp), L-glutamine (Gln), L-asparagine (Asn), L-cysteine (Cys), L-methionine (Met), L-lysine (Lys), L-arginine (Arg), and L-proline (Pro).

Non-natural amino acids (amino acid derivatives) are not particularly limited, and examples thereof include a β-amino acid, a D-type amino acid, an N-substituted amino acid (excluding Pro), an α,α-disubstituted amino acid, an amino acid having a side chain different from that of natural amino acids, and a hydroxycarboxylic acid. As used herein, non-natural N-substituted amino acids mean N-substituted amino acids other than Pro.

As the amino acids herein, amino acids having any conformation are acceptable. The selection of a side chain of an amino acid is not particularly limited, and the side chain is freely selected from, in addition to a hydrogen atom, an alkyl group, an alkenyl group, an alkynyl group, an aryl group, a heteroaryl group, an aralkyl group, a heteroaralkyl group, a cycloalkyl group, a spiro-bonded cycloalkyl group, and the like. Each of the side chains may have a substituent. The substituent is also not limited, and one or two or more substituents may be freely selected independently from any substituents including, for example, a halogen atom, an O atom, a S atom, a N atom, a B atom, a Si atom, or a P atom. That is, examples of the side chain include an alkyl group, an alkoxy group, an alkenyl group, an alkynyl group, an aryl group, a heteroaryl group, an aralkyl group, or a cycloalkyl group which may be substituted, oxo, aminocarbonyl, and a halogen atom. The amino acid according to one embodiment may be a compound having a carboxy group and an amino group in the same molecule, and even in this case, imino acids such as proline and hydroxyproline are also included in the amino acid.

Examples of halogen-derived substituents include fluoro (-F), chloro (-Cl), bromo (-Br), and iodo (-I).

Examples of O-atom-derived substituents include hydroxy (-OH), oxy (-OR), carbonyl (-C(=O)-R), carboxy (-CO₂H), oxycarbonyl(-C(=O)-OR), carbonyloxy (-O-C(=O)-R), thiocarbonyl (-C(=O)-SR), a carbonylthio group (-S-C(=O)-R), aminocarbonyl (-C(=O)-NHR), carbonylamino (-NH-C(=O)-R), oxycarbonylamino (-NH-C(=O)-OR), sulfonylamino (-NH-SO₂-R), aminosulfonyl (-SO₂-NHR), sulfamoylamino (-NH-SO₂-NHR), thiocarboxy (-C(=O)-SH), and carboxycarbonyl (-C(=O)-CO₂H).

Examples of oxy (-OR) include alkoxy, cycloalkoxy, alkenyloxy, alkynyloxy, aryloxy, heteroaryloxy, and aralkyloxy.

Examples of carbonyl (-C(=O)-R) include formyl (-C(=O)-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C(=O)-OR) include alkyloxycarbonyl, cycloalkyloxycarbonyl, alkenyloxycarbonyl, alkynyloxycarbonyl, aryloxycarbonyl, heteroaryloxy carbonyl, and aralkyloxycarbonyl.

Examples of carbonyloxy (-O-C(=O)-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C(=O)-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C(=O)-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C(=O)-NHR) include alkylaminocarbonyl, cycloalkylaminocarbonyl, alkenylaminocarbonyl, alkynylaminocarbonyl, arylaminocarbonyl, heteroarylaminocarbonyl, and aralkylaminocarbonyl. In addition to these, examples include compounds in which an H atom bonded to the N atom in -C(=O)-NHR is further substituted with alkyl, cycloalkyl, alkenyl, alkynyl, aryl, heteroaryl, or aralkyl.

Examples of carbonylamino (-NH-C(=O)-R) include alkylcarbonylamino, cycloalkylcarbonylamino, alkenylcarbonylamino, alkynylcarbonylamino, arylcarbonylamino, heteroarylcarbonylamino, and aralkylcarbonylamino. In addition to these, examples include compounds in which an H atom bonded to the N atom in -NH-C(=O)-R is further substituted with alkyl, cycloalkyl, alkenyl, alkynyl, aryl, heteroaryl, or aralkyl.

Examples of oxycarbonylamino (-NH-C(=O)-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. In addition to these, examples include compounds in which an H atom bonded to the N atom in - NH-C(=O)-OR is further substituted with alkyl, cycloalkyl, alkenyl, alkynyl, aryl, heteroaryl, or aralkyl.

Examples of sulfonylamino (-NH-SO₂-R) include alkylsulfonylamino, cycloalkylsulfonylamino, alkenylsulfonylamino, alkynylsulfonylamino, arylsulfonylamino, heteroarylsulfonylamino, and aralkylsulfonylamino. In addition to these, examples include compounds in which an H atom bonded to the N atom in -NH-SO₂-R is further substituted with alkyl, cycloalkyl, alkenyl, alkynyl, aryl, heteroaryl, or aralkyl.

Examples of aminosulfonyl (-SO₂-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. In addition to these, examples include compounds in which an H atom bonded to the N atom in -SO₂-NHR is further substituted with alkyl, cycloalkyl, alkenyl, alkynyl, aryl, heteroaryl, or aralkyl.

Examples of sulfamoylamino (-NH-SO₂-NHR) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. Further, the two H atoms bonded to the N atom in -NH-SO₂-NHR may be substituted with substituents independently selected from the group consisting of alkyl, cycloalkyl, alkenyl, alkynyl, aryl, heteroaryl, and aralkyl, or the two substituents may form a ring.

Examples of S-atom-derived substituents include thiol (-SH), thio (-S-R), sulfinyl (-S(=O)-R), sulfonyl (-S(O)₂-R), sulfo (-SO₃H), and pentafluorosulfanyl (-SF₅).

Examples of thio (-S-R) include alkylthio, cycloalkylthio, alkenylthio, alkynylthio, arylthio, heteroarylthio, and aralkylthio.

Examples of sulfinyl (-S(=O)-R) include alkylsulfinyl, cycloalkylsulfinyl, alkenylsulfinyl, alkynylsulfinyl, arylsulfinyl, heteroarylsulfinyl, and aralkylsulfinyl.

Examples of sulfonyl (-S(O)₂-R) include alkylsulfonyl, cycloalkylsulfonyl, alkenylsulfonyl, alkynylsulfonyl, arylsulfonyl, heteroarylsulfonyl, and aralkylsulfonyl.

Examples of N-atom-derived substituents include azido (-N₃, also referred to as "azide group"), cyano (-CN), primary amino (-NH₂), secondary amino (-NH-R), tertiary amino (-NR(R')), amidino (-C(=NH)-NH₂), substituted amidino (-C(=NR)-NR'R"), guanidino (-NH-C(=NH)-NH₂), substituted guanidino (-NR-C(=NR‴)-NR'R"), and aminocarbonylamino (-NR-CO-NR'R").

Examples of secondary amino (-NH-R) include alkylamino, cycloalkylamino, alkenylamino, alkynylamino, arylamino, heteroarylamino, and aralkylamino.

Examples of tertiary amino (-NR(R')) include an amino group having two substituents each independently selected from alkyl, cycloalkyl, alkenyl, alkynyl, aryl, heteroaryl, aralkyl, and the like, such as alkyl(aralkyl)amino, and the two substituents may form a ring.

Examples of substituted amidino (-C(=NR)-NR'R") include groups in which the three substituents R, R', and R" on the N atom are each independently selected from alkyl, cycloalkyl, alkenyl, alkynyl, aryl, heteroaryl, and aralkyl, such as alkyl(aralkyl)(aryl)amidino.

Examples of substituted guanidino (-NR-C(=NR‴)-NR'R") include a group in which R, R', R", and R‴ are each independently selected from alkyl, cycloalkyl, alkenyl, alkynyl, aryl, heteroaryl, and aralkyl, and a group in which they form a ring.

Examples of aminocarbonylamino (-NR-CO-NR'R") include a group in which R, R', and R" are each independently selected from a hydrogen atom, alkyl, cycloalkyl, alkenyl, alkynyl, aryl, heteroaryl, and aralkyl, and a group in which they form a ring.

Examples of B-atom-derived substituents include boryl (-BR(R')), and dioxyboryl (-B(OR)(OR')). The two substituents R and R' may be groups each independently selected from alkyl, cycloalkyl, alkenyl, alkynyl, aryl, heteroaryl, aralkyl, or the like, or may form a ring. Specific examples include a cyclic boryl group, and more specifically include a pinacolate boryl group, a neopentanediolate boryl group, and a catecholate boryl group.

The amino group of the main chain of the amino acid may be unsubstituted (-NH₂) or substituted (i.e., -NHR, wherein R represents, for example, an alkyl group, an alkenyl group, an alkynyl group, an aryl group, a heteroaryl group, an aralkyl group, or a cycloalkyl group, which is optionally substituted, or a carbon chain attached to the N atom and a carbon atom at position α may form a ring, like proline).

As used herein, an amino acid in which the amino group of the main chain is substituted is referred to as an "N-substituted amino acid". Examples of the "N-substituted amino acid" as used herein include preferably, but are not limited to, N-alkylamino acid, N-C₁-C₆ alkylamino acid, N-C₁-C₅ alkylamino acid, N-C₁-C₄ alkylamino acid, N-C₁-C₃ alkylamino acid, N-ethylamino acid, N-methylamino acid, N-C₇-C₁₄ aralkylamino acid, N-benzylamino acid, and N-phenethylamino acid.

Specific examples of the substituent on the nitrogen atom of the N-substituted amino acid (R of -NHR described above) herein include an alkyl group (preferably a C₁-C₆ alkyl group, more preferably a C₁-C₄ alkyl group, further preferably a C₁-C₃ alkyl group, still more preferably an ethyl group or a methyl group), a C₇-C₁₄ aralkyl group, a benzyl group, and a phenethyl group. As the substituent on the nitrogen atom of the N-substituted amino acid, an ethyl group or a methyl group is more preferred, and a methyl group is particularly preferred (i.e., N-methylamino acid is particularly preferred as the N-substituted amino acid).

The "amino acid" herein includes all corresponding isotopes to each. The isotope of "amino acid" is one in which at least one atom is substituted with an atom having the same atomic number (the same number of protons) and a different mass number (different sum of numbers of protons and neutrons). Examples of the isotope included in the "amino acid" herein include a hydrogen atom, a carbon atom, a nitrogen atom, an oxygen atom, a phosphorus atom, a sulfur atom, a fluorine atom, a chlorine atom, and the like, and they include ²H, ³H; ¹³C, ¹⁴C; ¹⁵N; ¹⁷O, ¹⁸O; ³²P; ³⁵S; ¹⁸F; ³⁶Cl; and the like, respectively.

The peptide compound according to the present embodiments may be a cyclic peptide compound. As used herein, the "cyclic peptide compound" is not particularly limited as long as it is a peptide compound having a cyclic portion constituted of 5 or more amino acid residues. The number of amino acid residues constituting the cyclic portion of the cyclic peptide compound may be 5 or more and 15 or less, 6 or more and 15 or less, 6 or more and 14 or less, 7 or more and 14 or less, 8 or more and 14 or less, 7 or more and 13 or less, 7 or more and 12 or less, 8 or more and 12 or less, 8 or more and 11 or less, 9 or more and 11 or less, 10, or 11. The number of amino acid residues constituting the cyclic portion of the cyclic peptide compound may be, for example, 8 or more and 20 or less, and is preferably 9 or more and 15 or less, more preferably 10 or more and 14 or less, and most preferably 11. The cyclic portion is preferably formed via a covalent bond such as an amide bond, a carbon-carbon bond formation reaction, an S-S bond, a thioether bond, and a triazole bond. The cyclization may take any form, such as cyclization with a carbon-nitrogen bond such as an amide bond, cyclization with a carbon-oxygen bond such as an ester bond or ether bond, cyclization with a carbon-sulfur bond such as a thioether bond, cyclization with a carbon-carbon bond, or cyclization by heterocyclic construction. Among these, cyclization through covalent bonds such as amide bonds and carbon-carbon bonds is preferred, and cyclization through an amide bond by a carboxy group of the side chain and an amino group of the main chain is more preferred. The positions of the carboxy group, the amino group, and the like used for cyclization may be on the main chain or the side chain, and are not particularly limited as long as the positions allow the groups to be cyclized.

The cyclic peptide compound may have a linear portion in addition to the cyclic portion. The specific aspects of the number of amino acid residues of the cyclic peptide compound are the same as the specific aspects of the number of amino acid residues of the peptide compound described above. When the cyclic peptide compound has a linear portion, the sum of the numbers of amino acid residues of the cyclic portion and the linear portion preferably falls within the range of the number of amino acid residues of the peptide compound described above. When the cyclic peptide compound has a linear portion, the number of amino acid residues constituting the cyclic portion may be 5 or more and 15 or less, 6 or more and 15 or less, 6 or more and 14 or less, 7 or more and 14 or less, 8 or more and 14 or less, 7 or more and 13 or less, 7 or more and 12 or less, 8 or more and 11 or less, 9 or more and 11 or less, 10, or 11, and the number of amino acid residues constituting the linear portion may be 1 or more and 8 or less, 1 or more and 7 or less, 1 or more and 6 or less, 1 or more and 5 or less, 1 or more and 4 or less, or 1 or more and 3 or less. When the cyclic peptide compound has a linear portion, the number of amino acid residues constituting the cyclic portion may be, for example, 8 or more and 20 or less, and is preferably 9 or more and 15 or less, more preferably 10 or more and 14 or less, and most preferably 11, and the number of amino acid residues constituting the linear portion may be, for example, 1 or more and 8 or less, and is preferably 1 or more and 6 or less, more preferably 1 or more and 4 or less, and most preferably 1 or more and 3 or less.

The molecular weight of the peptide compound according to the present embodiments is not particularly limited, but may be, for example, 500 g/mol or more, 550 g/mol or more, 600 g/mol or more, 650 g/mol or more, 700 g/mol or more, 750 g/mol or more, 800 g/mol or more, 850 g/mol or more, 900 g/mol or more, 950 g/mol or more, 1000 g/mol or more, 1100 g/mol or more, 1200 g/mol or more, 1300 g/mol or more, or 1400 g/mol or more. The upper limit of the molecular weight of the peptide compound according to the present embodiments is not particularly limited, but may be 5000 g/mol or less, 4000 g/mol or less, 3000 g/mol or less, 2500 g/mol or less, or 2000 g/mol or less. The range of the molecular weight of the peptide compound according to the present embodiments is, for example, 500 or more and 2,000 or less, preferably 1,000 or more and 1,800 or less, more preferably 1,300 or more and 1,600 or less, and most preferably 1,400 or more and 1,500 or less. The molecular weight as used herein means the sum (unit: "g/mol") of the atomic weights of atoms constituting the compound molecule and is obtained by calculating the sum of the atomic weights of the atoms included in the molecular structural formula (unit "g/mol"). The unit of the molecular weight as used herein may be omitted. The molecular weight of the peptide compound according to the present embodiments can be measured by any method known in the art, preferably can be measured by liquid chromatography, and more preferably can be measured by liquid chromatography-mass spectrometry (LC/MS) described in Examples.

The peptide compound according to the present embodiments may have ClogP of, for example, 4 or more and 25 or less. ClogP is a computer calculated partition coefficient and can be calculated in conformity with the principles of "CLOGP Reference Manual Daylight Version 4.9 (date of release: August 1, 2011, https://www.daylight.com/dayhtml/doc/clogp/)". One example of a method for calculating ClogP includes calculation using Daylight Version 4.95 from Daylight Chemical Information Systems, Inc. (date of release: August 1, 2011, ClogP algorithm version 5.4, database version 28,
https://www.daylight.com/dayhtml/doc/release_notes/index.html).

The ClogP of the peptide compound according to the present embodiments is more preferably 24 or less, further preferably 23 or less, still more preferably 22 or less, still even more preferably 21 or less, and particularly preferably 20 or less. The lower limit of ClogP of the peptide compound according to the present embodiments is more preferably 5 or more, further preferably 6 or more, still more preferably 7 or more, still even more preferably 8 or more, and particularly preferably 10 or more. Examples of the range of ClogP of the peptide compound according to the present embodiments include 5 or more and 24 or less, 6 or more and 23 or less, 7 or more and 22 or less, 8 or more and 21 or less, 9 or more and 20 or less, 10 or more and 20 or less, 11 or more and 18 or less, and 11.2 or more and 16.1 or less. The range of ClogP of the peptide compound according to the present embodiments is, for example, 4 or more and 25 or less, preferably 6 or more and 23 or less, more preferably 8 or more and 21 or less, and most preferably 9 or more and 20 or less.

The percentage of ClogP of the peptide compound according to the present embodiments based on cyclosporin A (ClogP: 14.36) may be 174% or less, may be 167% or less, may be 160% or less, may be 153% or less, may be 146% or less, or may be 139% or less. The percentage of ClogP may be 28% or more, may be 35% or more, may be 42% or more, may be 49% or more, may be 56% or more, may be 63% or more, or may be 70% or more. The percentage of ClogP of the peptide compound according to the present embodiments based on cyclosporin A (ClogP: 14.36) may be 28% or more and 174% or less, may be 35% or more and 174% or less, may be 42% or more and 167% or less, may be 49% or more and 160% or less, may be 56% or more and 153 or less, may be 63% or more and 146% or less, or may be 70% or more and 139% or less.

Thus, ClogP of the peptide compound according to the present embodiments can be indicated by the value of ClogP or the percentage based on ClogP of cyclosporin A, and these can be used interchangeably with each other. It is preferred that ClogP of the peptide compound according to the present embodiments is equal to or more than ClogP of a compound having a structure below, which is compound 4 described in Examples.

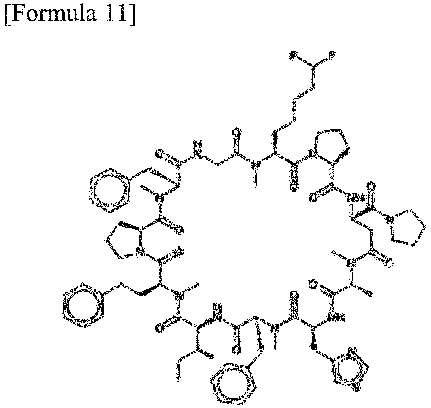

The peptide compound according to the present embodiments may have ClogP/number of amino acid residues of 1.0 or more. As used herein, the "number of amino acid residues" means a total number of amino acid residues constituting a peptide compound. For example, the number of amino acid residues of the cyclic peptide compound in which the cyclic portion is constituted of 10 amino acid residues and the linear portion is constituted of 1 amino acid residue is 11. The ClogP/number of amino acid residues is a value calculated by dividing ClogP of a peptide compound by the number of amino acid residues contained in the peptide compound. For example, when ClogP of a peptide compound is 14.0 and the number of amino acid residues contained in the peptide compound is 7, ClogP/number of amino acid residues of the peptide compound is calculated as 2.0.

The ClogP/number of amino acid residues of the peptide compound according to the present embodiments may be 1.1 or more or may be 1.2 or more. The upper limit of the ClogP/number of amino acid residues of the peptide compound according to the present embodiments may be 1.8 or less, 1.7 or less, 1.6 or less, or 1.5 or less. The range of the ClogP/number of amino acid residues of the peptide compound according to the present embodiments may be, for example, 1.0 or more and 1.8 or less, and is preferably 1.0 or more and 1.7 or less, more preferably 1.1 or more and 1.6 or less, and most preferably 1.1 or more and 1.5 or less.

The peptide compound according to the present embodiments may have a solubility of 10 mg/mL or less at 37°C and 1 atm in 50 mM phosphate buffer (pH 6.5). The solubility of the peptide compound according to the present embodiments at 37°C and 1 atm in 50 mM phosphate buffer (pH 6.5) may be 5 mg/ml or less, 2.5 mg/ml or less, 2.0 mg/ml or less, 1 mg/ml or less, 0.5 mg/ml or less, 0.25 mg/ml or less, 0.1 mg/ml or less, 0.05 mg/ml or less, 0.025 mg/ml or less, 0.01 mg/ml or less, 0.005 mg/ml or less, 0.0025 mg/ml or less, or 0.001 mg/ml or less. The solubility of the peptide compound according to the present embodiments at 37°C and 1 atm in 50 mM phosphate buffer (pH 6.5) may be, for example, more than 0 µg/mL and 10,000 µg/mL or less, and is preferably 0.1 µg/mL or more and 10,00 µg/mL or less, more preferably 0.5 µg/mL or more and 200 µg/mL or less, and most preferably 1.0 µg/mL or more and 100 µg/mL or less. It should be noted that the "solubility" means a solubility under the conditions of 37°C, 1 atm.

The solubility can be measured by the following procedures.

To an excessive amount of a lyophilized powder of the compound, 50 mM phosphate buffer (PPB, pH 6.5) is added, and after shaking (37°C, 1 atm, 1800 rpm, for 22 to 24 hours), the mixture is filtered with a filter, and the compound concentration of the filtrate is measured by LC/MS/MS. Solubility (µg/mL) is calculated from the measured compound concentration.

It is preferred that the peptide compound according to the present embodiments is a peptide compound classified into class IV by the Biopharmaceutics Classification System (BCS). BCS is a guideline for predicting the gastrointestinal absorption properties of a drug product by classifying the drug product into four classes (classes I to IV) based on its solubility and absorption rate. As used herein, the term "absorption" may mean gastrointestinal absorption.

The solubility in BCS (D₀: Dose Number) is determined with D₀ = 1 as the boundary, and the solubility is determined to be high (high solubility) when D₀ ≤ 1 and determined to be low (low solubility) when D₀ ≥ 1. The absorption rate (Fₐ: a rate of absorption from the gastrointestinal tract) in BCS is determined with an absorbance of 90% as the boundary, and the absorption rate is determined to be low (low absorption rate) when Fₐ ≤ 0.9, and determined to be high (high absorption rate) when Fₐ ≥ 0.9. Classes I to IV of BCS are defined as follows:
Class I: high solubility (D₀ ≤ 1) and high absorption rate (Fₐ ≥ 0.9)
Class II: low solubility (D₀ ≥ 1) and high absorption rate (Fₐ ≥ 0.9)
Class III: high solubility (D₀ ≤ 1) and low absorption rate (Fₐ ≤ 0.9)
Class IV: low solubility (D₀ ≥ 1)and low absorption rate (Fₐ ≤ 0.9).

The value of Caco-2 Papp (cm/sec) of the peptide compound according to the present embodiments, as measured in a system in which the surfactant (2) according to the present embodiments is not present, may be 1.0E-5 or less, 9.0E-6 or less, 8.0E-6 or less, 7.0E-6 or less, 6.0E-6 or less, 5.0E-6 or less, 4.0E-6 or less, 3.0E-6 or less, 2.0E-6 or less, 1.8E-6 or less, 1.6E-6 or less, 1.4E-6 or less, 1.2E-6 or less, 1.0E-6 or less, 9.8E-7 or less, 9.6E-7 or less, 9.4E-7 or less, 9.2E-7 or less, 9.0E-7 or less, 8.8E-7 or less, 8.6E-7 or less, 8.4E-7 or less, 8.2E-7 or less, 8.0E-7 or less, 7.8E-7 or less, 7.6E-7 or less, 7.4E-7 or less, 7.2E-7 or less, 7.0E-7 or less, 6.8E-7 or less, 6.6E-7 or less, 6.4E-7 or less, 6.2E-7 or less, 6.0E-7 or less, 5.8E-7 or less, 5.6E-7 or less, 5.4E-7 or less, 5.2E-7 or less, 5.0E-7 or less, 4.8E-7 or less, 4.6E-7 or less, 4.4E-7 or less, 4.2E-7 or less, 4.0E-7 or less, 3.8E-7 or less, 3.6E-7 or less, 3.4E-7 or less, 3.2E-7 or less, 3.0E-7 or less, 2.8E-7 or less, 2.6E-7 or less, 2.4E-7 or less, 2.2E-7 or less, 2.0E-7 or less, 1.8E-7 or less, 1.6E-7 or less, 1.4E-7 or less, 1.2E-7 or less, or 1.0E-7 or less. The value of Caco-2 Papp (cm/sec) of the peptide compound according to the present embodiments, as measured in a system in which the surfactant (2) according to the present embodiments is not present, may be, for example, 1.0E-10 or more and 1.0E-5 or less, and is preferably 1.0E-9 or more and 5.0E-7 or less, more preferably 3.0E-9 or more and 1.0E-7 or less, and most preferably 5.0E-9 or more and 5.0E-8 or less. It should be noted that E-n (n is a natural number) means 10⁻ⁿ (e.g., 1.0E-5 = 1.0 × 10⁻⁵).

The value of Caco-2 Papp (cm/sec) of the peptide compound according to the present embodiments, as measured in the system in which the surfactant (2) according to the present embodiments is present, may be 1.0E-9 or more, 1.0E-8 or more, 2.0E-8 or more, 3.0E-8 or more, 4.0E-8 or more, 5.0E-8 or more, 6.0E-8 or more, 7.0E-8 or more, 8.0E-8 or more, 9.0E-8 or more, 1.0E-7 or more, 1.1E-7 or more, 1.2E-7 or more, 1.3E-7 or more, 1.4E-7 or more, 1.5E-7 or more, 1.6E-7 or more, 1.7E-7 or more, 1.8E-7 or more, 1.9E-7 or more, or 2.0E-7 or more. The value of Caco-2 Papp (cm/sec) of the peptide compound according to the present embodiments, as measured in the system in which the surfactant (2) according to the present embodiments is present, may be, for example, 1.0E-9 or more and 1.0E-5 or less, and is preferably 5.0E-9 or more and 5.0E-6 or less, more preferably 1.0E-8 or more and 1.0E-6 or less, and most preferably 3.0E-8 or more and 5.0E-7 or less.

The value of Caco-2 Papp (cm/sec) of the peptide compound according to the present embodiments, as measured in the system in which the surfactant (2) according to the present embodiments is present, may be 2-fold or more, 3-fold or more, 5-fold or more, 10-fold or more, or 15-fold or more the value as measured in the system in which the surfactant (2) is not present. The value of Caco-2 Papp (cm/sec) of the peptide compound according to the present embodiments, as measured in the system in which the surfactant (2) according to the present embodiments is present, may be, for example, 1.1-fold or more and 100-fold or less, and is preferably 1.3-fold or more and 60-fold or less, more preferably 1.5-fold or more and 40-fold or less, and most preferably 2.0-fold or more and 30-fold or less the value as measured in the system in which the surfactant (2) is not present.

The value of Caco-2 Papp (cm/sec) is a value that serves as an indicator of the membrane permeability in the cell membrane and can be measured by the following methods.
(step 1) Caco-2 cells are cultured on a plate (e.g., 96-well Transwell and Falcon(R)96) for 3 weeks, and then the composition to be evaluated and a FaSSIF/HBSS buffer (pH 6.5) are added to the Apical side, and an HBSS buffer (pH 7.4) containing 4% BSA is added to the Basal side (the start of the permeability test).
(step 2) Each well is shaken at 5%CO₂, 37°C, 80 rpm, and at 180 minutes after the start, a sample on the Basal side is taken and the permeation amount of the peptide compound is measured by liquid chromatography-mass spectrometry (LC/MS/MS).
(step 3) From the measured permeability amount, the permeability coefficient (Caco-2 Papp (cm/sec)) is calculated.

In the above measurements, a Pgp inhibitor, such as Zosquidar, can be added to the FaSSIF/HBSS buffer and the HBSS buffer, respectively.

After step (step 1) and before step (step 2) of the above, pre-incubation can be performed by allowing each well to stand at 5% CO₂, 37°C, 80 rpm for 20 to 24 hours.

After pre-incubation, the solution at the Basal side can be removed and washed, and a new solution of the same composition can be added. A Pgp inhibitor can also be added to the new solution. When the pre-incubation is performed, it is preferable that a DMEM solution (pH 7.4) containing 4% BSA is used instead of the HBSS buffer (pH 7.4).

In addition, as the concentration of the substance to be evaluated on the donor side used in the calculation of the permeability coefficient in step (step 3) above, the concentration when initially added can be used, or the concentration measured by collecting the solution at the Apical side before the start of pre-incubation or the start of shaking in the above step (step 2) can be used. In particular, when the preincubation is performed, it is preferable to use the concentration of the solution at the Apical side collected before pre-incubation.

Specifically, the concentration can be measured by the method described in Examples.

In this manner, when measuring Caco-2 Papp (cm/sec) of the composition according to the present embodiments, the substance to be measured is a peptide compound contained in the composition.

The composition according to the present embodiments contains a peptide compound. The peptide compound contained in the composition according to the present embodiments is at least one or more selected from the group consisting of (i) a peptide compound containing one or more N-substituted amino acid residues, (ii) a peptide compound having ClogP of 4 or more and 25 or less, and (iii) a peptide compound having a solubility of 10 mg/mL or less at 37°C and 1 atm in 50 mM phosphate buffer (pH 6.5).

According to the present embodiments, (i) the peptide compound containing one or more N-substituted amino acid residues can be applied without limitation to the specific aspects of the peptide compound described above as long as the peptide compound is a peptide compound containing one or more N-substituted amino acid residues.

According to the present embodiments, (i) the peptide compound containing one or more N-substituted amino acid residues is, for example, a peptide compound containing one or more N-substituted amino acid residues, preferably containing at least three N-substituted amino acid residues, more preferably containing at least four N-substituted amino acid residues, and most preferably containing at least five N-substituted amino acid residues. The N-substituted amino acid residues may be present continuously or discontinuously in the N-substituted cyclic peptide compound.

Specific examples of (i) the peptide compound containing one or more N-substituted amino acid residues according to the present embodiments include, for example, compounds 1 to 12 described in Examples below.

According to the present embodiments, (ii) the peptide compound having ClogP of 4 or more and 25 or less can be applied without limitation to the specific aspects of the peptide compound described above as long as ClogP of the peptide compound is 4 or more and 25 or less.

According to the present embodiments, (iii) the peptide compound having a solubility of 10 mg/mL or less at 37°C and 1 atm in 50 mM phosphate buffer (pH 6.5) can be applied without limitation to the specific aspects of the peptide compound described above as long as the solubility of the peptide compound at 37°C and 1 atm in 50 mM phosphate buffer (pH 6.5) is 10 mg/mL or less.

Specific examples of (iii) the peptide compound having a solubility of 10 mg/mL or less at 37°C and 1 atm in 50 mM phosphate buffer (pH 6.5) according to the present embodiments can include cyclosporin A.

In the present embodiments, preferred examples of the peptide compound having all of the features above include compounds 1 to 12 described in Examples below.

### [Surfactant]

The composition according to the present embodiments may be a composition for combined use of a surfactant (2). The surfactant according to the present embodiments is at least one or more selected from the group consisting of (iv) a surfactant having a linear alkylene structure and having 5 or more and 13 or less carbon atoms in the alkylene structure, and (v) a surfactant having a carnitine residue. The surfactant may be used singly or in combination of two or more. Specific examples of the surfactant described below may be used in the form of a salt (e.g., hydrochloride or sodium salt).

The surfactant according to one embodiment may be a component that promotes the emulsification and dispersion of the peptide compound, a component that promotes absorption via the transcellular pathway, or a component that promotes absorption via the paracellular pathway.

The surfactant according to one embodiment has a linear alkylene structure, and the number of carbon atoms contained in the linear alkylene structure is 5 or more and 13 or less. The number of carbon atoms may be 6 or more, 7 or more, 8 or more, 10 or more, or 11. The number of carbon atoms contained in the linear alkylene structure may be 6 or more and 13 or less, 7 or more and 13 or less, 8 or more and 12 or less, 10 or more and 12 or less, or 11. The number of carbon atoms contained in the linear alkylene structure may be, for example, 5 or more and 13 or less, and is preferably 7 or more and 11 or less, more preferably 9 or more and 11 or less, and most preferably 11.

Preferred examples of the surfactant according to the present invention are shown below (the numbers in the parentheses each represent the number of carbon atoms in the linear alkylene structure).
(a) caproic acid (5)
(b) caprylic acid (7)
(c) capric acid (9)
(d) lauric acid (11)
(e) lauroylcarnitine (11)
(f) lauroyl-L-carnitine (11)
(g) carnitine palmitate (15)

It is also preferred that the surfactant according to one embodiment is a compound represented by any of the following general formulae (a1) to (a3):

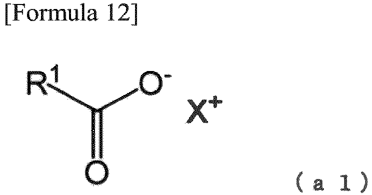

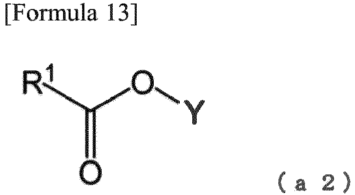

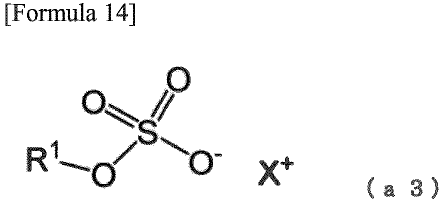

In the general formulae (a1) to (a3), R¹ represents an optionally substituted, saturated or unsaturated, linear alkyl group having 5 or more and 13 or less carbon atoms, X represents sodium or potassium, and Y represents a group represented by the following formula (a4) or a stereoisomer thereof.

The unsaturated alkyl group can also be referred to as an unsaturated hydrocarbon group.

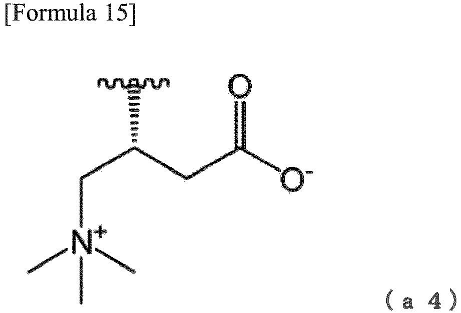

In the general formulae (a1) to (a3), R¹ is preferably an alkyl group having 5 or more and 13 or less carbon atoms, more preferably an alkyl group having 7 or more and 13 or less carbon atoms, further preferably an alkyl group having 8 or more and 12 or less carbon atoms, still more preferably an alkyl group having 10 or more and 12 or less carbon atoms, and particularly preferably an alkyl group having 11 carbon atoms. It is also preferred that the alkyl group is a linear alkyl group. It is also preferred that the alkyl group is a saturated alkyl group. It is also preferred that the alkyl group is an unsubstituted alkyl group. In the formula (a4), 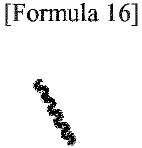 represents a bond.

The surfactant according to one embodiment also contains a medium-chain fatty acid structure. The medium-chain fatty acid refers to a fatty acid having 6 or more and 12 or less carbon atoms. The number of carbon atoms in the medium-chain fatty acid structure may be 8 or more, 10 or more, or 12. The number of carbon atoms in the medium-chain fatty acid structure may be, for example, 6 or more and 12 or less, and is preferably 8 or more and 12 or less, more preferably 10 or more and 12 or less, and most preferably 12.

The surfactant according to one embodiment may be a medium-chain fatty acid ester, a sodium salt of medium-chain fatty acid, or a potassium salt of medium-chain fatty acid. The medium-chain fatty acid ester is a compound in which an ester bond is formed between a carboxy group of a medium-chain fatty acid and a hydroxy group of a hydroxy group-containing compound. Examples of the medium-chain fatty acid include, but are not limited to, caproic acid, enanthic acid, caprylic acid, pelargonic acid, capric acid, undecylic acid, and lauric acid, and among them, more preferably caprylic acid, capric acid, and lauric acid, and further preferably lauric acid. Examples of the hydroxy group-containing compound include, but are not limited to, aliphatic alcohol, polyhydric alcohol, and hydroxy group-containing betaine, such as carnitine, trimethylglycine, or proline betaine.

The surfactant according to one embodiment is preferably acylcarnitine, further preferably lauroylcarnitine or carnitine palmitate, still more preferably lauroylcarnitine, and particularly preferably lauroyl-L-carnitine.

The surfactant according to one embodiment has a carnitine residue. The surfactant having a carnitine residue may preferably be acylcarnitine. Acylcamitine is a compound in which an ester bond is formed between a hydroxy group of carnitine and a carboxy group of a carboxy group-containing compound. The carnitine may be in a D-form or an L-form. The carboxy group-containing compound may be an organic acid, preferably a medium-chain fatty acid such as a saturated fatty acid or an unsaturated fatty acid, more preferably a saturated fatty acid. The number of carbon atoms of the medium-chain fatty acid may be 6 or more, 8 or more, 10 or more, or 12. The number of carbon atoms of the medium-chain fatty acid may be, for example, 6 or more and 12 or less, and is preferably 8 or more and 12 or less, more preferably 10 or more and 12 or less, and most preferably 12. The number of carbon atoms of the medium-chain fatty acid may be 6 or more, 8 or more, 10 or more, or 12. The number of carbon atoms of the medium-chain fatty acid may be, for example, 6 or more and 12 or less, and is preferably 8 or more and 12 or less, more preferably 10 or more and 12 or less, and most preferably 12. Examples of the saturated fatty acid according to the present invention include caproic acid, caprylic acid, capric acid, and lauric acid. The acylcarnitine according to the present invention is more preferably lauroylcarnitine or carnitine palmitate, further preferably lauroylcarnitine, and particularly preferably lauroyl-L-carnitine.

The surfactant according to one embodiment may be an anionic surfactant, a cationic surfactant, an amphoteric surfactant, or a nonionic surfactant. The surfactant is preferably an anionic surfactant or a cationic surfactant, more preferably a cationic surfactant. Examples of the anionic surfactant include a carboxylate, a sulfonate, and a sulfate, preferably a sulfonate, and further preferably sodium lauryl sulfate (sodium dodecyl sulfate).

The surfactant according to one embodiment may be used in combination as an isolated component or as a composition including this surfactant with the composition according to the present embodiments. In one embodiment, the surfactant may be used as an absorption-promoting agent for the peptide compound (1).

### [Solubility Improver]

The composition according to the present embodiments may further include a solubility improver that improves the solubility of a peptide compound. Examples of the component that improves the solubility of a peptide compound include various oily components, a polymer that forms a solid dispersion (Amorphous Solid Dispersion, hereinafter referred to as "ASD") with a peptide compound, and a component that adjusts pH. The solubility improver may be used singly or in combination of two or more.

Preferred specific examples of the oily component can include fatty acids such as oleic acid, stearic acid, linoleic acid, palmitic acid, linolenic acid, and myristic acid, olive oil, almond oil, coconut oil, cocoa butter, macadamia nut oil, avocado oil, safflower oil, soybean oil, flaxseed oil, rapeseed oil, castor oil, palm oil, high oleic sunflower oil, high oleic safflower oil, sunflower oil, cottonseed oil, corn oil, sesame oil, peanut oil, almond oil, Aleurites Moluccanus seed oil, grape seed oil, pistachio seed oil, sunflower oil, hazelnut oil, jojoba oil, meadowfoam oil, rosehip oil, Tricaproin, Tricaprylin, Tricaprin, Tripalmitolein, Triolein, Trilinolein, Trilinolenin, Trieicosenoin, and Trierucin. Examples of the oily component include, in addition to those listed above, vegetable oils collected from plants, those partially decomposed by a hydrolysis treatment thereof, and those separated and purified. The oily component may also be obtained by synthesizing using a synthesis method.

Regarding the oily component, it may be further preferable to use a compound having a polyoxyethylene structure added to an oily component as a solubility improver. The polyoxyethylene structure is represented by -(CH₂-CH₂-O)ₙ-. In this compound, the average number of moles of ethylene oxide added is preferably 2 or more and 100 or less, more preferably 3 or more and 80 or less, further preferably 3 or more and 60 or less, and still more preferably 3 or more and 50 or less. The average molar number of ethylene oxide added is also preferably 5 or more and 40 or less, more preferably 10 or more and 40 or less, further preferably 20 or more and 40 or less, and still more preferably 30 or more and 40 or less.

Specific examples of the compound having the polyoxyethylene structure added include polyoxyethylene castor oil, polyoxyethylene hydrogenated castor oil, and a polyoxyethylene sorbitan fatty acid ester. Among these, polyoxyethylene castor oil, polyoxyethylene hydrogenated castor oil, and a polyoxyethylene sorbitan fatty acid ester are preferred, polyoxyethylene castor oil and a polyoxyethylene sorbitan fatty acid ester are more preferred, polyoxyethylene castor oil having an average number of moles of ethylene oxide added of 30 or more and 40 or less and a polyoxyethylene sorbitan fatty acid ester having an average number of moles of ethylene oxide added of 10 or more and 40 or less are further preferred, and polyoxyethylene castor oil 35 and polyoxyethylene (20) sorbitan monooleate (Tween 80) are still more preferred.

Examples of the polymer that forms ASD with a peptide compound include polyethylene glycol, polyvinylpyrrolidone, copovidone, polyvinyl alcohol, a cellulose-based polymer, and a methacrylic acid methacrylic acid copolymer. Specific examples include a vinyl polymer or copolymer having at least one substituent selected from hydroxyl, alkylacyloxy, and a group containing a cyclic amide; a vinyl copolymer of at least one hydrophilic hydroxyl-containing repeating unit and at least one hydrophobic alkyl- or aryl-containing repeating unit; polyvinyl alcohol; polyvinyl alcohol having at least a portion of repeating units of a non-hydrolyzed (vinyl acetate) form; a polyvinyl alcohol polyvinyl acetate copolymer; polyvinyl pyrrolidone; copovidone; a copolymer of acrylate and methacrylic acid; a polyethylene polyvinyl alcohol copolymer; a polyoxyethylene-polyoxypropylene block copolymer (also called as poloxamer), polyethylene glycol, hydroxypropyl methyl cellulose acetate (HPMCA), hydroxypropyl methyl cellulose (HPMC), hydroxypropyl cellulose (HPC), methylcellulose, hydroxyethyl methyl cellulose, hydroxyethyl cellulose, hydroxyethyl cellulose acetate, hydroxyethyl ethyl cellulose, cellulose acetate phthalate, cellulose acetate trimellitate, cellulose acetate succinate, methyl cellulose phthalate, hydroxymethyl cellulose ethyl phthalate, hydroxypropyl methyl cellulose phthalate, hydroxypropyl methyl cellulose acetate succinate (HPMCAS), hydroxypropyl methyl acetate maleate, hydroxypropyl methyl trimellitate, carboxymethyl ethyl cellulose, polyvinyl butylate phthalate, polyvinyl alcohol acetate phthalate, a methacrylic acid/ethyl acrylate copolymer (preferably in a mass ratio of 1 : 99 to 99 : 1), a methacrylic acid/methyl methacrylate copolymer (preferably in a mass ratio of 1 : 99 to 99 : 1), a methacrylic acid copolymer, aminoalkyl methacrylic acid copolymer E, and polyvinyl acetal diethyl aminoacetate.

Specific examples of the pH adjusting agent include lactic acid, succinic acid, gluconic acid, citric acid, citric acid hydrate, trisodium citrate, phosphoric acid, potassium carbonate, sodium hydrogen carbonate, tartaric acid, malic acid, ascorbic acid, fumaric acid, aspartic acid, glutamic acid, glutamic acid hydrochloride, malonic acid, maleic acid, meglumine, arginine, lysine, glycine, sodium carbonate, and sodium hydrogen phosphate.

### [Self-emulsifying formulation]

The composition according to the present embodiments can also include a component in a state in which (3) a solubility improver is pre-mixed, such as a Self-Emulsifying Drug Delivery System (hereinafter referred to as "SEDDS").

### [Composition]

The composition according to the present embodiments includes at least (1) a peptide compound. The amount of the surfactant (2) to be used in combination with the composition according to the present embodiments may be 0.05 parts by mass or more, 0.075 parts by mass or more, 0.1 parts by mass or more, 0.2 parts by mass or more, or 0.3 parts by mass or more of (2) the surfactant based on 1 part by mass of (1) the peptide compound. Also, (2) the surfactant may be 300 parts by mass or less, 200 parts by mass or less, 150 parts by mass or less, 100 parts by mass or less, 80 parts by mass or less, 60 parts by mass or less, 40 parts by mass or less, or 30 parts by mass or less based on 1 part by mass of (1) the peptide compound. Further, the amount of (2) the surfactant may be 0.05 parts by mass or more and 300 parts by mass or less, 0.05 parts by mass or more and 200 parts by mass or less, 0.05 parts by mass or more and 150 parts by mass or less, 0.05 parts by mass or more and 100 parts by mass or less, 0.075 parts by mass or more and 80 parts by mass or less, 0.1 parts by mass or more and 60 parts by mass or less, 0.2 parts by mass or more and 40 parts by mass or less, or 0.3 parts by mass or more and 30 parts by mass or less based on 1 part by mass of (1) the peptide compound.

The amount of (2) the surfactant based on 1 part by mass of (1) the peptide compound may be, for example, 0.05 parts by mass or more and 300 parts by mass or less, and is preferably 0.1 parts by mass or more and 100 parts by mass or less, more preferably 1.0 part by mass or more and 50 parts by mass or less, and most preferably 1.5 parts by mass or more and 30 parts by mass or less. When (1) the peptide compound contains two or more peptide compounds, the above range is to the total amount of the two or more peptide compounds. The same holds for (2) the surfactant. The components (1) and (2) can be quantified by liquid chromatography-mass spectrometry (LC-MS), a liquid chromatography-charged aerosol detector, or a nuclear magnetic resonance apparatus (NMR).

When the composition according to the present embodiments contains (3) the solubility improver, regarding the contents of the component (1) and the component (3) in the composition, (3) the solubility improver may be 0.1 parts by mass or more, 0.2 parts by mass or more, 0.3 parts by mass or more, 0.4 parts by mass or more, 0.5 parts by mass or more, 1 part by mass or more, 3 parts by mass or more, 4 parts by mass or more, 5 parts by mass or more, 6 parts by mass or more, or 7 parts by mass or more based on 1 part by mass of (1) the peptide compound. Also, (3) the solubility improver may be 100 parts by mass or less, 80 parts by mass or less, 60 parts by mass or less, 40 parts by mass or less, and 20 parts by mass or less. When (1) the peptide compound contains two or more peptide compounds, the above range is to the total amount of the two or more peptide compounds.

In addition, when (3) the solubility improver is a liquid at 25°C, the content of (3) the solubility improver based on 100% by volume of the liquid component contained in the composition including (3) the solubility improver itself may be 0.05% by volume or more, 0.075% by volume or more, 0.1% by volume or more, 0.2% by volume or more, 0.3% by volume or more, 0.5% by volume or more, or 1.0% by volume or more. The content of (3) the solubility improver may be 100% by volume or less, 85% by volume or less, 50% by volume or less, 40% by volume or less, 30% by volume or less, 20% by volume or less, 15% by volume or less, or 10% by volume or less. When (3) the solubility improver is a liquid at 25°C, the content of (3) the solubility improver based on 100% by volume of the liquid component contained in the composition including (3) the solubility improver itself is, for example, 0.05% by volume or more and 100% by volume or less, preferably 0.1% by volume or more and 30% by volume or less, more preferably 0.5% by volume or more and 20% by volume or less, and most preferably 1.0% by volume or more and 10% by volume or less.

The content of the (1) peptide compound in the composition according to the present embodiments may be appropriately set according to the type of the peptide compound, the application of the composition, and the like. Examples of the content of the (1) peptide compound in the composition according to the present embodiments are, but are not limited to, 0.01 mg/ml or more and 300 mg/ml or less, 0.03 mg/ml or more and 200 mg/ml or less, 0.1 mg/ml or more and 100 mg/ml or less, 0.3 mg/ml or more and 50 mg/ml or less, 1 mg/ml or more and 25 mg/ml or less, and 3 mg/ml or more and 10 mg/ml or less per 1 ml of the liquid component contained in the composition according to the present embodiments.

The composition according to the present embodiments may contain a pharmaceutically acceptable carrier. Examples of the carrier include saline, buffered saline, water, an isotonic aqueous buffer, and a combination of these.

The composition according to the present embodiments may contain pharmaceutically acceptable other components to the extent that the effect according to the present invention is not impaired. Examples of the other components include a stabilizer, a preservative, an antioxidant, a disintegrant, an excipient, a binder, and a fluidizer or lubricant. Examples of the stabilizer include phosphatidic acid, ascorbic acid, glycerin, and cetanol. Examples of the preservative include ethyl paraoxybenzoate and propyl paraoxybenzoate. Examples of the antioxidant include butylated hydroxytoluene, butylated hydroxyanisole, propyl gallate, and gallic acid propyl ester. Examples of the disintegrant include calcium carmellose, sodium croscarmellose, crospopidone, and low-substituted hydroxypropyl cellulose. Examples of the excipient include starches such as corn starch, lactose, glucose, and D-mannitol. Examples of the binder include sucrose, gelatin, gum arabic powder, and methylcellulose. Examples of the fluidizer or lubricant include light anhydrous silicic acid, aqueous silicic acid dioxide, magnesium stearate, and talc.

The surfactant according to the present embodiments can be used as an absorption-promoting agent of a peptide compound, since the surfactant promotes oral absorption of a peptide compound having low membrane permeability.

The composition according to the present embodiments may also be used as a pharmaceutical composition for tough targets such as protein-protein interaction inhibition, agonists, molecular chaperones, or the like, depending on the type of peptide compound used. The pharmaceutical composition according to the present embodiments may include the peptide compound (1) as an active ingredient (pharmacologically active substance).

Furthermore, the composition according to the present embodiments may be used as a composition for administration, especially as a composition for oral administration, to a living organism. Examples of the subject to be administered include a mammal, specifically mouse, rat, rabbit, dog, monkey, and human. The composition according to the present embodiments may be used particularly for administration to human. Thus, the composition according to the present embodiments may be used as a pharmaceutical composition. Further, the present invention provides a treatment and/or prevention method comprising administering an effective amount of the composition according to the present invention to a subject in need thereof.

The order of administration of the composition according to the present embodiments and the surfactant (2) to a subject is not particularly limited, and the composition may be administered first and the surfactant may then be administered, or the surfactant may be administered first and the composition may then be administered. Any one of these components may be administered, for example, within 24 hours, preferably within 12 hours, more preferably within 6 hours, and most preferably within 1 hour after administration of the other.

### [Kit including composition according to present embodiments and surfactant]

The composition according to the present embodiments and the surfactant (2) can be combined to prepare a kit. The kit may include an instruction manual or a package insert.

### [Other aspects of present invention]

In a non-limiting aspect, the composition according to the present embodiments includes a peptide compound (1) below:
(1) at least one or more peptide compounds selected from the group consisting of (i), (ii), and (iii) below:
   (i) a peptide compound containing one or more N-substituted amino acid residues,
   (ii) a peptide compound having ClogP of 4 or more and 25 or less, and
   (iii) a peptide compound having a solubility of 10 mg/mL or less at 37°C and 1 atm in 50 mM phosphate buffer (pH 6.5).

In this aspect, the composition is used in combination with a surfactant and can thereby improve the absorbability of the peptide compound according to the present embodiments. The composition can be applied without limitation to each aspect described above. The amount of the surfactant for the combined use of the composition may be 0.05 parts by mass or more, 0.075 parts by mass or more, 0.1 parts by mass or more, 0.2 parts by mass or more, or 0.3 parts by mass or more based on 1 part by mass of the peptide compound (1) in the composition according to the present embodiments. The amount of the surfactant based on 1 part by mass of the peptide compound (1) may be 300 parts by mass or less, 200 parts by mass or less, 150 parts by mass or less, 100 parts by mass or less, 80 parts by mass or less, 60 parts by mass or less, 40 parts by mass or less, or 30 parts by mass or less. The amount of the surfactant based on 1 part by mass of the peptide compound (1) may further be 0.05 parts by mass or more and 300 parts by mass or less, 0.05 parts by mass or more and 200 parts by mass or less, 0.05 parts by mass or more and 150 parts by mass or less, 0.05 parts by mass or more and 100 parts by mass or less, 0.075 parts by mass or more and 80 parts by mass or less, 0.1 parts by mass or more and 60 parts by mass or less, 0.2 parts by mass or more and 40 parts by mass or less, or 0.3 parts by mass or more and 30 parts by mass or less. The amount of the surfactant based on 1 part by mass of the peptide compound (1) may be, for example, 0.05 parts by mass or more and 300 parts by mass or less, and is preferably 0.1 parts by mass or more and 100 parts by mass or less, more preferably 1.0 part by mass or more and 50 parts by mass or less, and most preferably 1.5 parts by mass or more and 30 parts by mass or less. When the component (1) contains two or more peptide compounds, etc., the above range is to the total amount of the two or more peptide compounds, etc. The same holds for the surfactant.

In a non-limiting aspect, the present invention provides a method for improving absorbability of the peptide compound (1) above, and this method can also be regarded as a method including using in combination a composition including the peptide compound (1) and a surfactant. Specific aspects of the method according to the present embodiments can be applied without limitation to the aspects described about the composition according to the present invention. In the method according to the present embodiments, the surfactant can be applied without limitation to the surfactants described herein, but is preferably at least one member selected from the group consisting of lauroyl-L-carnitine, sodium caprylate, sodium lauryl sulfate, and sodium N-(8-[2-hydroxybenzoyl]amino)caprylate, and more preferably lauroyl-L-carnitine. In the present aspects, the peptide compound (1) itself may be used instead of the composition including the peptide compound (1).

In a non-limiting aspect, the present invention may provide use of a surfactant for improving absorption of the peptide compound (1) above. Specific aspects of the method according to the present embodiments can be applied without limitation to the aspects described about the composition according to the present invention. In the method according to the present embodiments, the surfactant can be applied without limitation to the surfactants described herein, but is preferably at least one member selected from the group consisting of lauroyl-L-carnitine, sodium caprylate, sodium lauryl sulfate, and sodium N-(8-[2-hydroxybenzoyl]amino)caprylate, and more preferably lauroyl-L-carnitine.

In a non-limiting aspect, the present invention may provide a composition including a surfactant, for combined use of the peptide compound (1) above itself or a composition including the peptide compound (1). In the present embodiments, the surfactant can be applied without limitation to the surfactants described herein, but is preferably at least one member selected from the group consisting of lauroyl-L-carnitine, sodium caprylate, sodium lauryl sulfate, and sodium N-(8-[2-hydroxybenzoyl]amino)caprylate, and more preferably lauroyl-L-carnitine. In the present aspects, the peptide compound (1) itself may be used instead of the composition including the peptide compound (1).

### Examples

Hereinafter, preferred specific aspects of the present invention are described by way of Examples, but the present invention is not limited thereto.

### [Synthesis Example] Synthesis of cyclic peptide compounds

Cyclic peptide compounds 1 to 12 (also referred to simply as compounds 1 to 12) having the amino acid sequence shown in Table 1 were synthesized by the same method as described in WO2013/100132, WO2018/225864 or WO2021/90855, and the final products were obtained as dried products. The portion present at the right end in Table 1 forms the C-terminus. Tables 2-1 to 2-3 provides descriptions of the abbreviations of amino acids. Furthermore, the structural formulae of compounds 1 to 12 and cyclosporin A are shown in Tables 3-0 to 3-4.

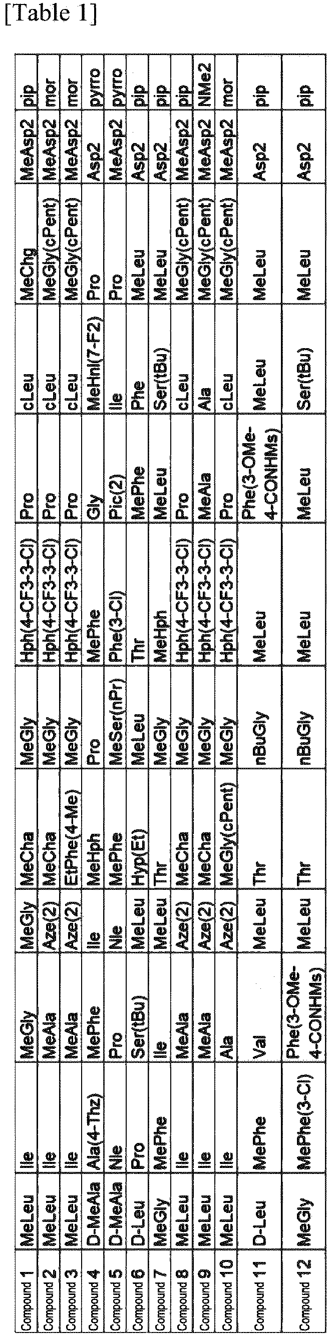

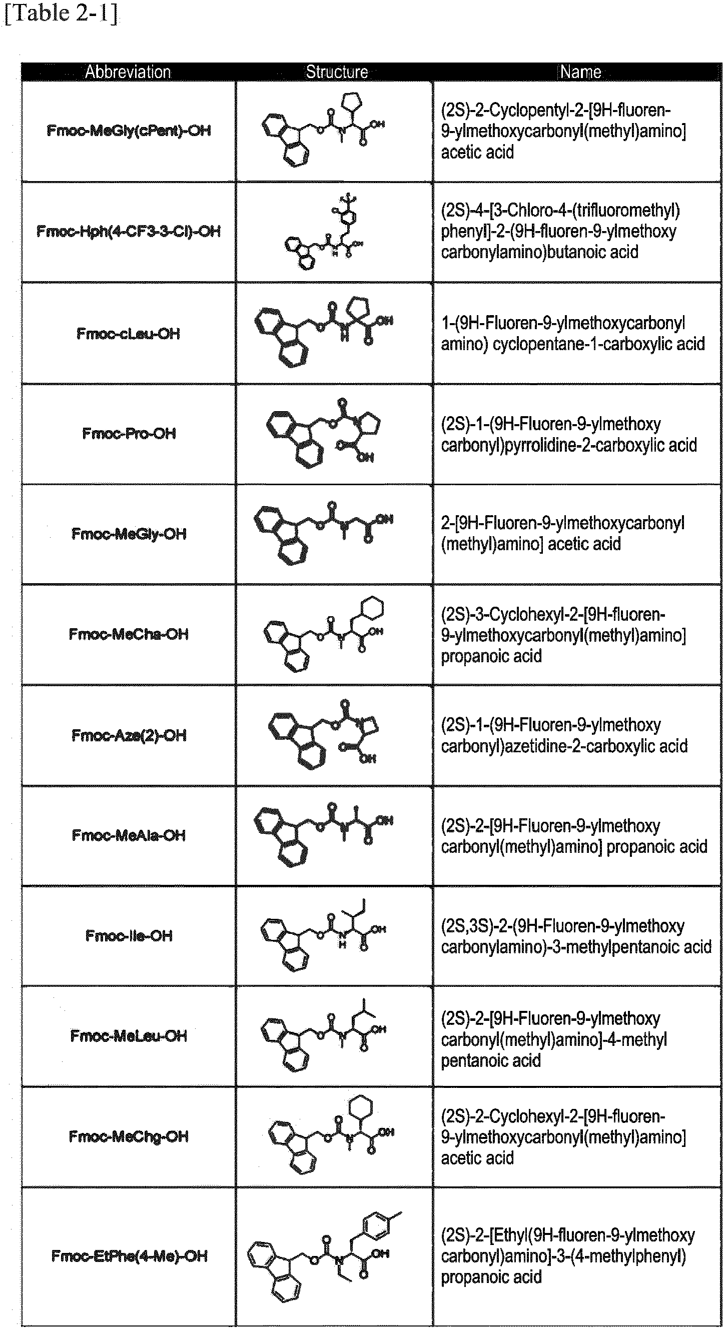

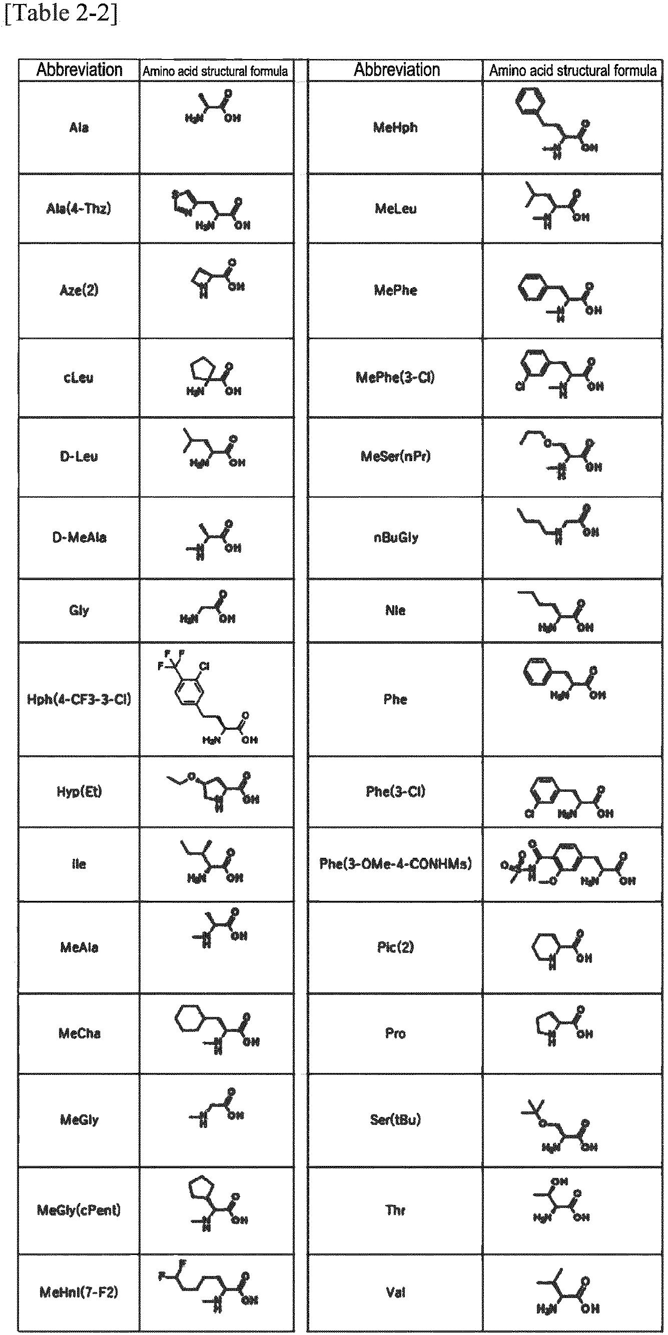

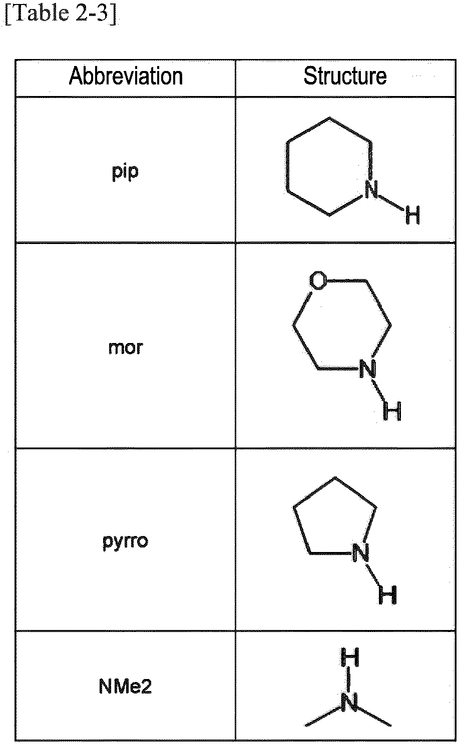

**[Table 3-0]**

| | |
|---|---|
| Cyclosporin A | 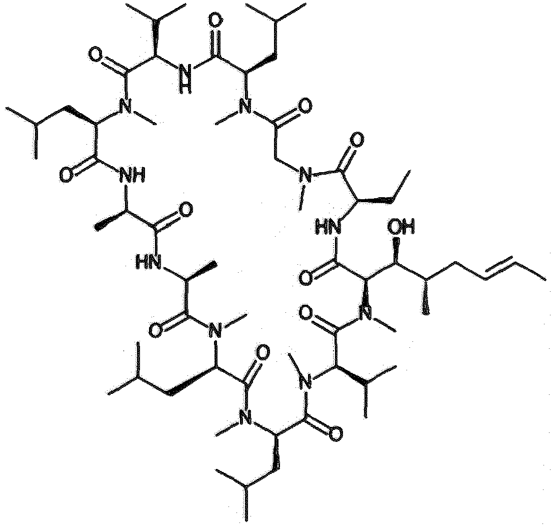 |

**[Table 3-1]**

| | |
|---|---|
| Compound **1** | 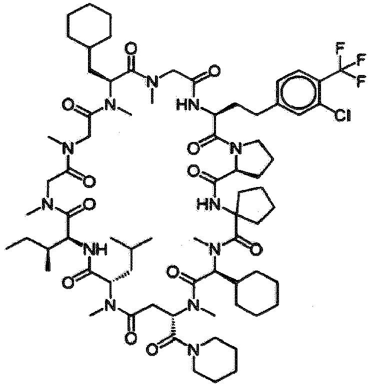 |
| Compound **2** | 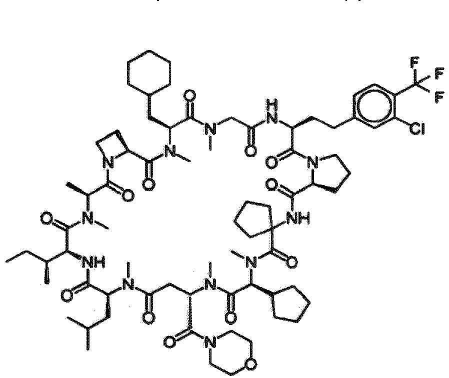 |
| Compound **3** | 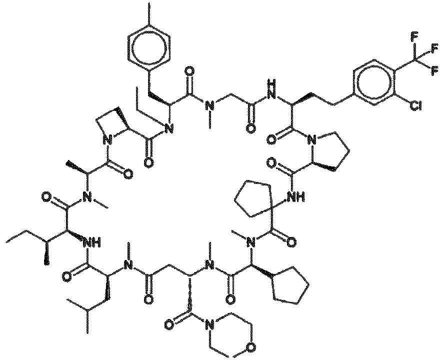 |

**[Table 3-2]**

| | |
|---|---|
| Compound **4** | 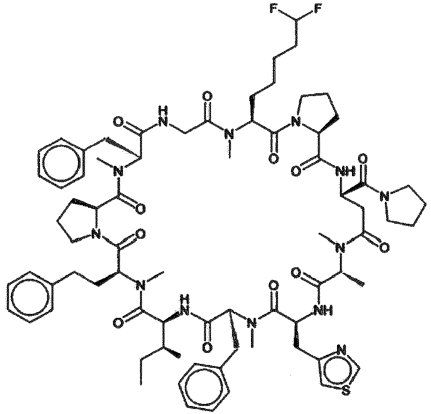 |
| Compound **5** | 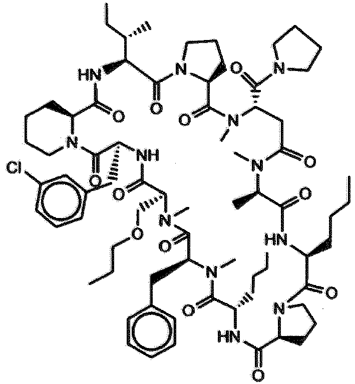 |
| Compound **6** | 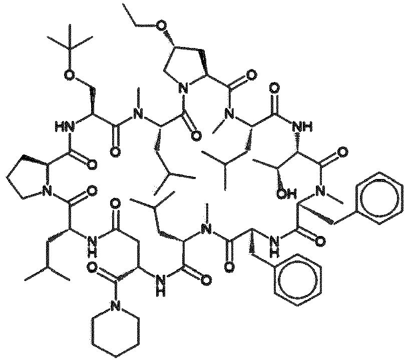 |

**[Table 3-3]**

| | |
|---|---|
| Compound **7** | 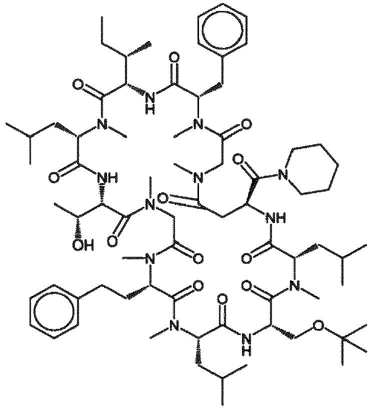 |
| Compound **8** | 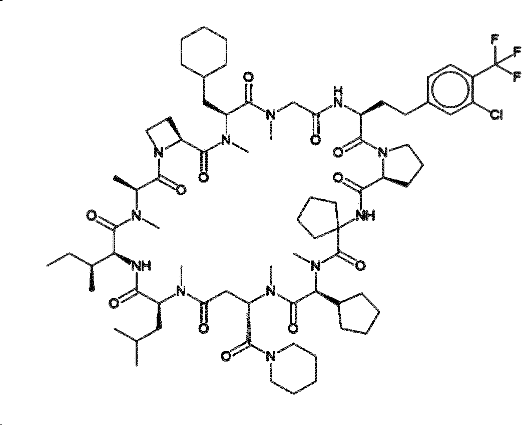 |
| Compound **9** | 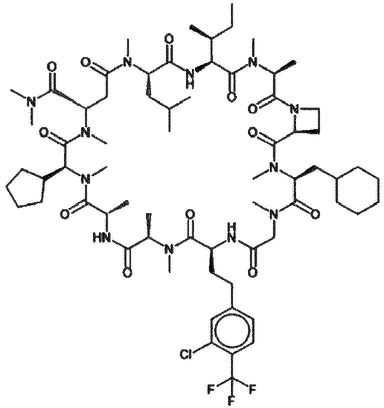 |

**[Table 3-4]**

| | |
|---|---|
| Compound **10** | 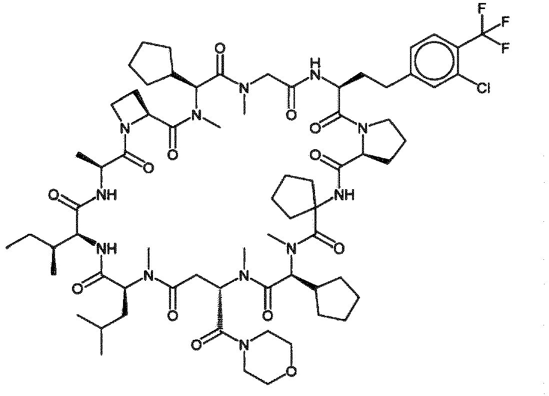 |
| Compound **11** | 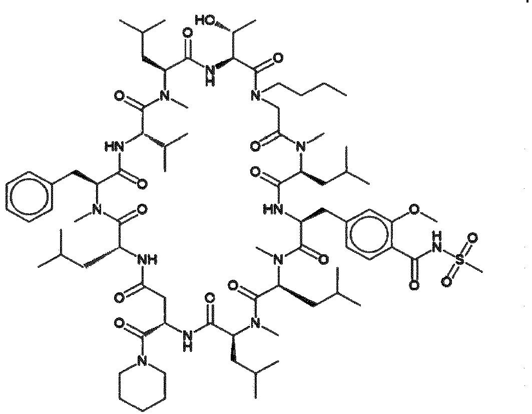 |
| Compound **12** | 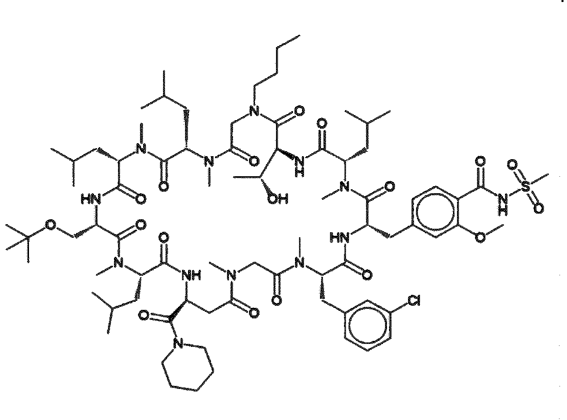 |

More specific synthetic procedures for compounds 1 to 12 are shown below.

### [Synthesis Example 1: Synthesis of compound 2]

The measurement conditions for liquid chromatography-mass spectrometry (LC/MS) are shown in Table 4.

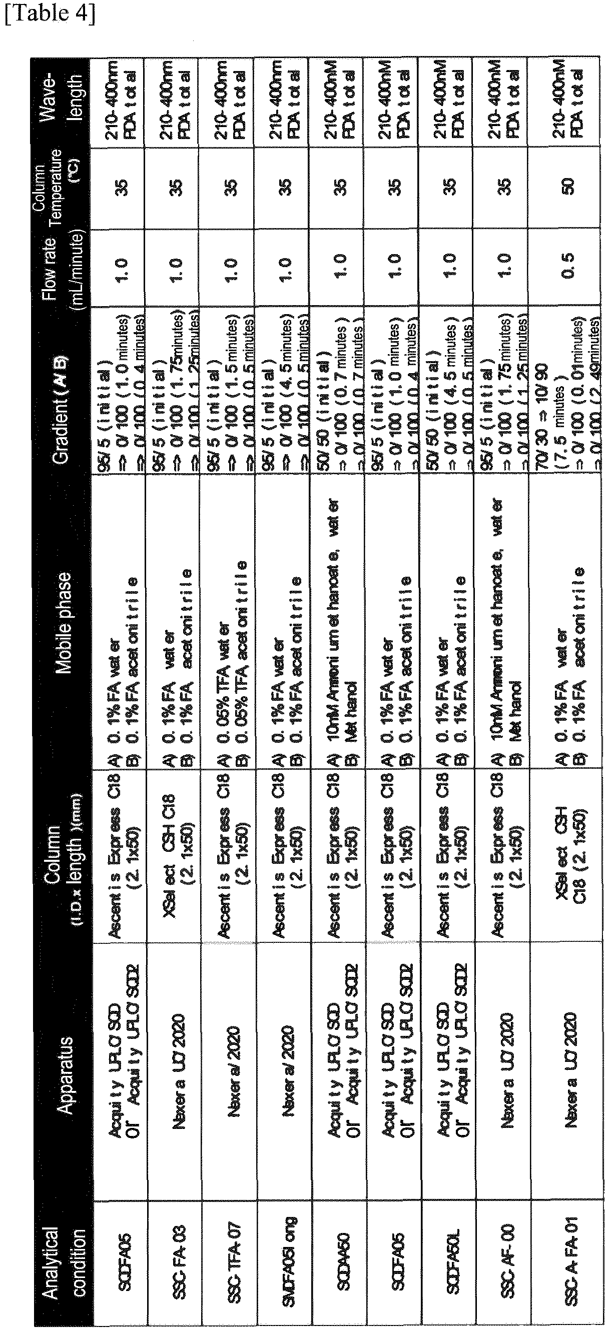

### Compound 2 was synthesized according to the following scheme.

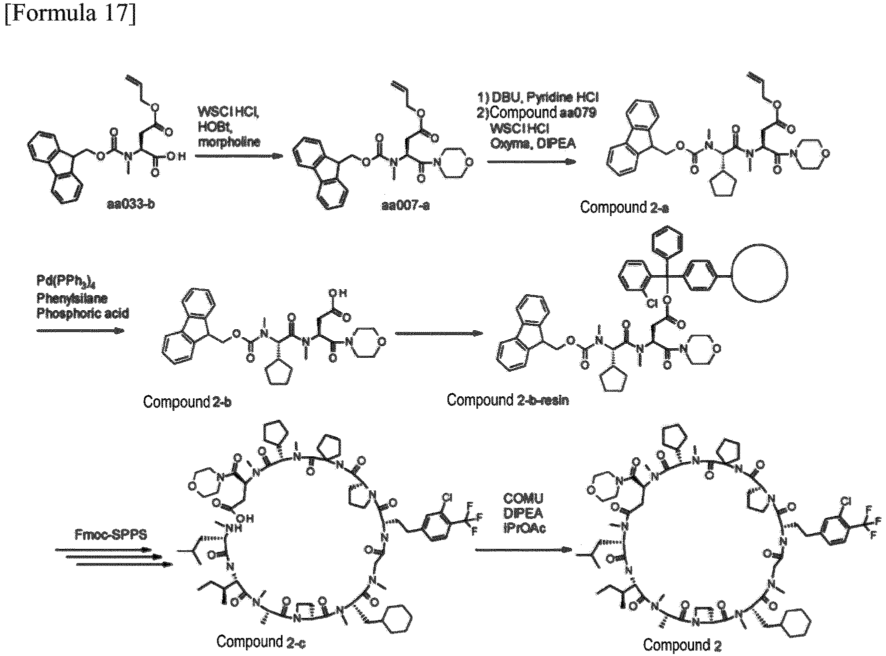

### (1) Synthesis of compound aa007-a

Under the nitrogen atmosphere, to a solution of compound aa033-b ((2S)-2-[9H-fluoren-9-ylmethoxycarbonyl(methyl)amino]-4-oxo-4-prop-2-enoxybutanoic acid, Fmoc-MeAsp(OAl)-OH (87.94 g, 215 mmol) in dimethylformamide (DMF) (430 ml) produced by the method described in WO2021/090855 at room temperature, 1-hydroxybenzotriazole (HOBt) (31.9 g, 236 mmol) and 1-ethyl-3-(3-dimethylaminopropyl)carbodiimide hydrochloride (WSCI·HCl) (49.4 g, 258 mmol) were added, and the mixture was stirred for 30 minutes. Then, the reaction solution was cooled to 0°C, and morpholine (20.44 mL, 236 mmol) was added dropwise, and the mixture was stirred at 0°C for 45 minutes. To the reaction solution, water (180 mL) was added, and the mixture was stirred at room temperature for 1 hour. Further, water (180 mL) was added, and the mixture was stirred at room temperature for 105 minutes. The precipitated solid was collected by filtration and dried under reduced pressure to obtain compound aa007-a (86.83 g, yield 84%).
LCMS(ESI) m/z = 479 (M + H)⁺

### Retention time: 2.57 minutes (analytical condition SMDFA05long)

### (2) Synthesis of compound 2-a

To a solution of compound aa079 ((2S)-2-cyclopentyl-2-[9H-fluoren-9-ylmethoxycarbonyl(methyl)amino]acetic acid) (22.6 g, 59.6 mmol) produced by the method described in WO2021/090855 and cyano(hydroxyimino)ethyl acetate (Oxyma) (10.69 g, 75 mmol) in DMF (203 mL), WSCI·HCl (16.83 g, 88 mmol) was added at room temperature, and the mixture was stirred for 30 minutes to obtain solution A.

To a solution of compound aa007-a (30 g, 62.7 mmol) in DMF (203 mL) under the nitrogen atmosphere, diazabicycloundecene (DBU) (9.45 mL, 62.7 mmol) was added dropwise at room temperature, and the mixture was stirred for 5 minutes. Pyridine hydrochloride (7.97 g, 69 mmol) was added thereto, and the mixture was stirred for 5 minutes. To the obtained reaction solution, solution A and N,N-diisopropylethylamine (DIPEA) (10.95 mL, 62.7 mmol) were added, and the mixture was stirred under the nitrogen atmosphere at room temperature for 2.5 hours. The reaction solution was diluted with ethyl acetate (300 mL), washed twice with hydrochloric acid (1 mol/L, 300 mL), and the resulting aqueous phase was extracted twice with ethyl acetate (300 mL). All organic phases were mixed, and washed sequentially with water (300 mL), twice with a mixed solution of saturated aqueous sodium hydrogen carbonate solution and water (1 : 1, 300 mL), and with a mixed solution of saturated brine and water (1 : 1, 300 mL). Then, the resulting organic phase was dried over sodium sulfate and the solvent was distilled off under reduced pressure. To the obtained residue, dichloromethane (DCM) (300 mL) was added, and the solid was removed by filtration. The solvent of the obtained solution was distilled off under reduced pressure, and the obtained residue was purified by silica gel column chromatography (hexane/ethyl acetate) to obtain compound 2-a (23.8 g, yield 61.5%).
LCMS(ESI) m/z = 640.4 (M + Na)⁺

### Retention time: 0.97 minutes (analytical condition SQDFA05)

### (3) Synthesis of compound 2-b

To a solution of compound 2-a (23.8 g, 38.5 mmol) in DCM (77 mL) under the nitrogen atmosphere, tetrakis(triphenylphosphine) palladium(0) (0.445 g, 0.385 mmol) was added at room temperature, and phenylsilane (3.32 mL, 27 mmol) was added, and the mixture was stirred for 30 minutes. The reaction solution was diluted with methyl tert-butyl ether (MTBE) (240 mL) and extracted with a mixed solution of saturated aqueous sodium bicarbonate solution and water (1 : 1, 240 mL). The resulting organic phase was extracted with water (50 mL). All aqueous phases were mixed, DCM (240 mL) was added to the mixture, and phosphoric acid (13.44 ml, 231 mmol) was then added dropwise thereto. The organic phase was separated, and the aqueous phase was extracted with DCM (240 mL). The obtained organic phases were mixed, washed with a mixed solution of saturated saline and water (1 : 1, 240 mL), and then dried over sodium sulfate, and the solvent was distilled off under reduced pressure to obtain compound 2-b (21.35 g, 96% yield).
LCMS(ESI) m/z = 578.4 (M + H)⁺

### Retention time: 0.80 minutes (analytical condition SQDFA05)

### (4) Synthesis of compound 2-b-resin

A reaction vessel with a filter was charged with 2-chlorotrityl chloride resin (1.36 mmol/g, 46.2 g, 62.8 mmol), and DCM (462 mL) was added. The vessel was shaken at room temperature for 45 minutes, and the solvent was then discharged from the filter. A solution of compound 2-b (21.35 g, 37 mmol), methanol (11.96 mL, 296 mmol) and DIPEA (30.9 mL, 177 mmol) in DCM (323 mL) was added to the reaction vessel. The vessel was shaken at room temperature for 60 minutes, and the solution was discharged from the filter. Subsequently, a solution of methanol (44.85 mL, 1.1 mol) and DIPEA (30.9 mL, 177 mmol) in DCM (323 mL) was added to the reaction vessel. The vessel was shaken at room temperature for 90 minutes, and the solution was then discharged from the filter. To the reaction vessel, DCM (323 mL) was added, and the vessel was shaken for 5 minutes, and the solvent was discharged from the filter. This washing operation of resin was repeated four more times, and the obtained resin was dried under reduced pressure to obtain compound 2-b-resin (59.1 g). The carrying amount was calculated to 0.433 mmol/g by the quantitative method of resin described in WO2013/100132, WO2018/225864, or WO2021/90855.

### (5) Synthesis of compound 2-c

Subsequent extensions of Fmoc-cLeu-OH, Fmoc-Pro-OH, Fmoc-Hph (4-CF3-3-Cl)-OH (compound aa132, produced by the method described in WO2021/090855), Fmoc-MeGly-OH, Fmoc-MeCha-OH, Fmoc-Aze(2)-OH, Fmoc-MeAla-OH, Fmoc-Ile-OH, and Fmoc-MeLeu-OH were performed by Fmoc solid-phase synthesis method.

### (5-1) Extension of Fmoc-cLeu-OH

Compound 2-b-resin (0.433 mmol/g, 59 g, 25.5 mmol) was added to a reaction vessel with a filter. DCM (600 mL) was added to this solid-phase reaction vessel, and after shaking at room temperature for 5 minutes, the solvent was discharged from the frit. DMF (420 mL) was added to this solid-phase reaction vessel, and after shaking at room temperature for 5 minutes, the solvent was discharged from the frit. This washing step of resin with DMF was repeated one more time. A DMF solution of DBU (2 v/v%, 420 mL) was added to this solid-phase reaction vessel, and after shaking at room temperature for 10 minutes, the solvent was discharged from the frit.

DMF (420 mL) was added to this solid-phase reaction vessel, and after shaking at room temperature for 5 minutes, the solution was discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, and after shaking at room temperature for 5 minutes, the solution was discharged from the frit. DMF (420 mL) was added to this solid-phase reaction vessel, and after shaking at room temperature for 5 minutes, the solvent was discharged from the frit. This washing step of resin with DMF was repeated one more time.

A solution of Fmoc-cLeu-OH (35.9 g, 102 mmol) and Oxyma (9.08 g, 63.9 mmol) in DMF (180 ml) and a solution of N,N'-diisopropylcarbodiimide (DIC) in DMF (10 v/v%, 216 mL) were mixed at room temperature, and after 2 minutes, the resulting mixture was added to the solid-phase reaction vessel obtained by the above. This solid-phase reaction vessel was shaken at 50°C for 24 hours, and the solution was then discharged from the frit. DMF (420 mL) was added to this solid-phase reaction vessel, and after shaking at room temperature for 5 minutes, the solvent was discharged from the frit. This washing step of resin with DMF was repeated four more times.

### (5-2) Extension of Fmoc-Pro-OH

A DMF solution of DBU (2 v/v%, 420 mL) was added to the solid-phase reaction vessel obtained by the above, and after shaking at room temperature for 10 minutes, the solution was discharged from the frit.

DMF (420 mL) was added to this solid-phase reaction vessel, the vessel was shaken at room temperature for 5 minutes, and the solution was then discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, and after shaking at room temperature for 5 minutes, the solution was discharged from the frit. DMF (420 mL) was added to this solid-phase reaction vessel, and after shaking at room temperature for 5 minutes, the solution was discharged from the frit. This washing step of resin with DMF was repeated one more time.

A solution ofFmoc-Pro-OH (17.24 g, 51.1 mmol) and 1-hydroxy-7-azabenzotriazole (HOAt) (4.35 g, 31.9 mmol) in DMF (240 mL) was mixed with DIC (11.54 mL, 74.1 mmol) at room temperature, and after 2 minutes, the mixture was added to the solid-phase reaction vessel obtained by the above. This solid-phase reaction vessel was shaken at 30°C for 17 hours, and the solution was then discharged from the frit. DMF (420 mL) was added to this solid-phase reaction vessel, and after shaking at room temperature for 5 minutes, the solvent was discharged from the frit. This washing step of resin with DMF was repeated four more times. DCM (420 mL) was added to this solid-phase reaction vessel, and after shaking at room temperature for 5 minutes, the solvent was discharged from the frit. This washing step of resin was repeated four more times. The obtained resin was dried under reduced pressure to obtain 63.1 g of resin.

### (5-3) Extension of Fmoc-Hph(4-CF3-3-Cl)-OH (compound aa132)

DCM (600 mL) was added to the solid-phase reaction vessel obtained by the above, and after shaking at room temperature for 5 minutes, the solvent was discharged from the frit. DMF (420 mL) was added to this solid-phase reaction vessel, and after shaking at room temperature for 5 minutes, the solvent was discharged from the frit. This washing step of resin with DMF was repeated one more time. A DMF solution of DBU (2 v/v%, 420 mL) was added thereto, and after shaking at room temperature for 10 minutes, the solution was discharged from the frit.

DMF (420 mL) was added to this solid-phase reaction vessel, and after shaking at room temperature for 5 minutes, the solution was discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, and after shaking at room temperature for 5 minutes, the solution was discharged from the frit. DMF (420 mL) was added to this solid-phase reaction vessel, and after shaking at room temperature for 5 minutes, the solvent was discharged from the frit. This washing step of resin with DMF was repeated one more time.

A solution of Fmoc-Hph(4-CF3-3-Cl)-OH (compound aa132) (25.7 g, 51.1 mmol) and HOAt (4.35 g, 31.9 mmol) in DMF (240 mL) was mixed with DIC (11.54 mL, 74.1 mmol) at room temperature, and after 2 minutes, the mixture was added to the solid-phase reaction vessel obtained by the above. This solid-phase reaction vessel was shaken at 30°C for 21 hours, and the solution was then discharged from the frit. DMF (420 mL) was added to this solid-phase reaction vessel, and after shaking at room temperature for 5 minutes, the solvent was discharged from the frit. This washing step of resin with DMF was repeated four more times.

### (5-4) Extension of Fmoc-MeGly-OH

A DMF solution of DBU (2 v/v%, 420 mL) was added to the solid-phase reaction vessel obtained by the above, and after shaking at room temperature for 10 minutes, the solution was discharged from the frit.

DMF (420 mL) was added to this solid-phase reaction vessel, and after shaking at room temperature for 5 minutes, the solution was discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, the vessel was shaken at room temperature for 5 minutes, and the solution was then discharged from the frit. DMF (420 mL) was added to this solid-phase reaction vessel, and after shaking at room temperature for 5 minutes, the solvent was discharged from the frit. This washing step of resin with DMF was repeated one more time.

A solution of Fmoc-MeGly-OH (15.91 g, 51.1 mmol) and HOAt (4.35 g, 31.9 mmol) in DMF (240 mL) was mixed with DIC (11.54 mL, 74.1 mmol) at room temperature, and after 2 minutes, the resulting mixture was added to the solid-phase reaction vessel obtained by the above. This solid-phase reaction vessel was shaken at 30°C for 32 hours, and the solution was then discharged from the frit. DMF (420 mL) was added to this solid-phase reaction vessel, and after shaking at room temperature for 5 minutes, the solution was discharged from the frit. This washing step of resin with DMF was repeated four more times.

### (5-5) Extension of Fmoc-MeCha-OH

A DMF solution of DBU (2 v/v%, 420 mL) was added to the solid-phase reaction vessel obtained by the above, and after shaking at room temperature for 10 minutes, the solution was discharged from the frit.

DMF (420 mL) was added to this solid-phase reaction vessel, and after shaking at room temperature for 5 minutes, the solution was discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, and after shaking at room temperature for 5 minutes, the solution was discharged from the frit. DMF (420 mL) was added to this solid-phase reaction vessel, and after shaking at room temperature for 5 minutes, the solvent was discharged from the frit. This washing step of resin with DMF was repeated one more time.

A solution of Fmoc-MeCha-OH (20.82 g, 51.1 mmol) and HOAt (4.35 g, 31.9 mmol) in DMF (240 mL) was mixed with DIC (11.54 mL, 74.1 mmol) at room temperature, and after 2 minutes, the resulting mixture was added to the solid-phase reaction vessel obtained by the above. This solid-phase reaction vessel was shaken at 30°C for 12 hours, and the solution was then discharged from the frit. DMF (420 mL) was added to this solid-phase reaction vessel, and after shaking at room temperature for 5 minutes, the solvent was discharged from the frit. This washing step of resin with DMF was repeated four more times. DCM (420 mL) was added to this solid-phase reaction vessel, and after shaking at room temperature for 5 minutes, the solvent was discharged from the frit. This washing step of resin with DCM was repeated four more times. The obtained resin was dried under reduced pressure to obtain 72.4 g of resin.

### (5-6) Extension of Fmoc-Aze(2)-OH

DCM (600 mL) was added to the resulting solid-phase reaction vessel, and after shaking at room temperature for 5 minutes, the solvent was discharged from the frit. DMF (420 mL) was added to this solid-phase reaction vessel, and after shaking at room temperature for 5 minutes, the solvent was discharged from the frit. This washing step of resin with DMF was repeated one more time. A DMF solution of DBU (2 v/v%, 420 mL) was added thereto, and after shaking at room temperature for 10 minutes, the solution was discharged from the frit.

DMF (420 mL) was added to this solid-phase reaction vessel, and after shaking at room temperature for 5 minutes, the solution was discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, and after shaking at room temperature for 5 minutes, the solution was discharged from the frit. DMF (420 mL) was added to this solid-phase reaction vessel, and after shaking at room temperature for 5 minutes, the solvent was discharged from the frit. This washing step of resin with DMF was repeated one more time.

A solution of Fmoc-Aze(2)-OH (16.52 g, 51.1 mmol) and HOAt (4.35 g, 31.9 mmol) in DMF (240 mL) was mixed with DIC (11.54 mL, 74.1 mmol) at room temperature and after 2 minutes, the resulting mixture was added to the solid-phase reaction vessel obtained by the above. This solid-phase reaction vessel was shaken at 30°C for 21 hours, and the solution was then discharged from the frit. DMF (420 mL) was added to this solid-phase reaction vessel, and after shaking at room temperature for 5 minutes, the solvent was discharged from the frit. This washing step of resin with DMF was repeated four more times. DCM (420 mL) was added to this solid-phase reaction vessel, and after shaking at room temperature for 5 minutes, the solvent was discharged from the frit. This washing step of resin with DCM was repeated four more times. The obtained resin was dried under reduced pressure to obtain 73.1 g of resin.

### (5-7) Extension of Fmoc-MeAla-OH

DCM (600 mL) was added to the resulting solid-phase reaction vessel, and after shaking at room temperature for 5 minutes, the solvent was discharged from the frit. DMF (420 mL) was added to this solid-phase reaction vessel, and after shaking at room temperature for 5 minutes, the solvent was discharged from the frit. This washing step of resin with DMF was repeated one more time. A DMF solution of DBU (2 v/v%, 420 mL) was added thereto, and after shaking at room temperature for 10 minutes, the solution was discharged from the frit.

DMF (420 mL) was added to this solid-phase reaction vessel, and after shaking at room temperature for 5 minutes, the solution was discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, and after shaking at room temperature for 5 minutes, the solution was discharged from the frit. DMF (420 mL) was added to this solid-phase reaction vessel, and after shaking at room temperature for 5 minutes, the solvent was discharged from the frit. This washing step of resin with DMF was repeated one more time.

A solution of Fmoc-MeAla-OH (16.63 g, 51.1 mmol) and HOAt (4.35 g, 31.9 mmol) in DMF (240 mL) was mixed with DIC (11.54 mL, 74.1 mmol), and after 2 minutes, the resulting mixture was added to the solid-phase reaction vessel obtained by the above. This solid-phase reaction vessel was shaken at 30°C for 16 hours, and the solution was then discharged from the frit. DMF (420 mL) was added to this solid-phase reaction vessel, and after shaking at room temperature for 5 minutes, the solvent was discharged from the frit. This washing step of resin with DMF was repeated four more times. DCM (420 mL) was added to this solid-phase reaction vessel, and after shaking at room temperature for 5 minutes, the solvent was discharged from the frit. This washing step of resin with DCM was repeated four more times. The obtained resin was dried under reduced pressure to obtain 76.4 g of resin.

### (5-8) Extension of Fmoc-Ile-OH

DCM (600 mL) was added to the solid-phase reaction vessel obtained by the above, and after shaking at room temperature for 5 minutes, the solvent was discharged from the frit. DMF (420 mL) was added to this solid-phase reaction vessel, and after shaking at room temperature for 5 minutes, the solvent was discharged from the frit. This washing step of resin with DMF was repeated one more time. A DMF solution of DBU (2 v/v%, 420 mL) was added thereto, and after shaking at room temperature for 10 minutes, the solution was discharged from the frit.

DMF (420 mL) was added to this solid-phase reaction vessel, and after shaking at room temperature for 5 minutes, the solution was discharged from the frit. A solution of triethylamine hydrochloride (7.03 g, 51.1 mmol) in DCM (420 mL) was added to this solid-phase reaction vessel, and after shaking at room temperature for 5 minutes, the solution was discharged from the frit. DCM (420 mL) was added to this solid-phase reaction vessel, and after shaking at room temperature for 5 minutes, the solution was discharged from the frit. DMF (420 mL) was added to this solid-phase reaction vessel, and after shaking at room temperature for 5 minutes, the solvent was discharged from the frit. This washing step of resin with DMF was repeated one more time.

A solution of Fmoc-Ile-OH (36.1 g, 102 mmol) and HOAt (8.69 g, 63.9 mmol) in DMF (180 mL) was mixed with a solution of DIC (10 v/v%) in DMF (216 mL), and after 2 minutes, the resulting mixture was added to the solid-phase reaction vessel obtained by the above. This solid-phase reaction vessel was shaken at 40°C for 8 hours, then at 30°C for 14 hours. The solution was then discharged from the frit. DMF (420 mL) was added to this solid-phase reaction vessel, and after shaking at room temperature for 5 minutes, the solvent was discharged from the frit. This washing step of resin with DMF was repeated three more times. DCM (420 mL) was added to this solid-phase reaction vessel, and after shaking at room temperature for 5 minutes, the solvent was discharged from the frit. This washing step of resin with DCM was repeated one more time. Toluene (420 mL) was further added to this solid-phase reaction vessel, and after shaking at room temperature for 5 minutes, the solvent was discharged from the frit. This washing step of resin with toluene was repeated one more time.

### (5-9) Extension of Fmoc-MeLeu-OH

A solution of DBU in toluene (2 v/v%, 420 mL) was added to the resulting solid-phase reaction vessel, and after shaking at room temperature for 10 minutes, the solution was discharged from the frit.

Toluene (420 mL) was added to this solid-phase reaction vessel, and after shaking at room temperature for 5 minutes, the solvent was discharged from the frit. This washing step of resin with toluene was repeated one more time. DCM (420 mL) was added to this solid-phase reaction vessel, and after shaking at room temperature for 5 minutes, the solvent was discharged from the frit. This washing step of resin with DCM was repeated one more time. A solution of Fmoc-MeLeu-OH (37.6 g, 102 mmol), [ethylcyano(hydroxyimino)acetat-O²]tri-1-pyrrolidinylphosphonium hexafluorophosphate (PyOxym) (53.9 g, 102 mmol) and DIPEA (26.8 mL, 153 mmol) in DCM (300 mL) was added thereto, and the vessel was shaken at 30°C for 2 hours. The solution was then discharged from the frit. DMF (420 mL) was added to this solid-phase reaction vessel, and after shaking at room temperature for 5 minutes, the solvent was discharged from the frit. This washing step of resin with DMF was repeated four more times. DCM (420 mL) was added to this solid-phase reaction vessel, and after shaking at room temperature for 5 minutes, the solvent was discharged from the frit. This washing step of resin with DCM was repeated four more times. The obtained resin was dried under reduced pressure to obtain 80.9 g of resin. Of the obtained resin, 40.4 g (equivalent to 13 mmol, converted from the carrying amount of compound 2-b-resin) was transferred to another solid-phase reaction vessel with a filter, and the following reaction was carried out.

### (6) Synthesis of compound 2-c (excision of peptide from resin)

To the resulting solid-phase reaction vessel containing 40.4 g (equivalent to 13 mmol converted from the carrying amount of compound 2-b-resin) of the resin, DCM (300 mL) was added, and after shaking at room temperature for 5 minutes, the solvent was discharged from the frit. DMF (210 mL) was further added to this solid-phase reaction vessel, and after shaking at room temperature for 5 minutes, the solvent was discharged from the frit. This washing step of resin with DMF was repeated one more time. A DMF solution of DBU (2 v/v%, 210 mL) was added thereto, and after shaking at room temperature for 10 minutes, the solution was discharged from the frit. DMF (210 mL) was added to this solid-phase reaction vessel, and after shaking at room temperature for 5 minutes, the solvent was discharged from the frit. This washing step of resin with DMF was repeated four more times. DCM (210 mL) was added to this solid-phase reaction vessel, and after shaking at room temperature for 5 minutes, the solvent was discharged from the frit. This washing step of resin with DCM was repeated four more times.

To the solid-phase reaction vessel containing resin obtained by the above, a mixed solution of 2,2,2-trifluoroethanol (TFE) (270 mL), DCM (270 mL) and DIPEA (4.01 mL, 23 mmol) was added, and the vessel was shaken at room temperature for 2 hours. The solution was then collected from the frit. To this solid-phase reaction vessel, a mixed solution of TFE (150 mL) and DCM (150 mL) was added, and after shaking at room temperature for 20 minutes, the solution was collected from the frit. Furthermore, to this solid-phase reaction vessel, a mixed solution of TFE (150 mL) and DCM (150 mL) was added, and after shaking at room temperature for 20 minutes, the solution was collected from the frit. All the collected solutions were mixed and the solvent was distilled off under reduced pressure to obtain compound 2-c as a crude product. (18.9 g)
LCMS(ESI) m/z = 1474.0 (M + H)⁺

### Retention time: 0.69 minutes (analytical condition SQDFA05)

### (7) Synthesis of compound 2 (cyclization and purification of peptide)

The compound 2-c (9.6 g) obtained above was dissolved in a mixed solution of isopropyl acetate (1246 mL) and DIPEA (1.959 mL, 11.21 mmol), and (1-cyano-2-ethoxy-2-oxoethylideneaminooxy)dimethylamino-morpholino-carbenium hexafluorophosphate (COMU) (4 g, 9.35 mmol) was added, and the mixture was stirred at room temperature for 14 hours. After that, the reaction solution was washed with a mixed solution of saturated aqueous ammonium chloride (350 mL) and water (350 mL), and then further washed with saturated saline (700 ml). The obtained organic phase was dried over sodium sulfate, and the solvent was distilled off under reduced pressure to obtain a residue of about 8 g. Further, the same operation was performed on compound 2-c (9.3 g). All the residue obtained were purified by reverse-phase silica gel column chromatography using acetonitrile containing 0.1% formic acid/water containing 0.1% formic acid as eluents) to obtain a crude product (8.1 g). Of the obtained crude product, 7.9 g was purified by silica gel column chromatography (DCM/methanol) to obtain compound 2 (6.9 g, 37%). The values in mass spectrum and retention time in liquid chromatography of the obtained compound 2 were described in Table 5.

### [Synthesis Example 2: Synthesis of compound 1]

Compound 1 (6.53 g, 56%) was obtained by a synthesis method similar to that of compound 2 using a resin (30 g) carrying a dipeptide produced by the same method as the synthesis of compound 2-b-resin as a raw material. The values in mass spectrum and retention time in liquid chromatography of the obtained compound 1 were described in Table 5.

### [Synthesis Example 3: Synthesis of compound 3]

Compound 3 (23 g, 30.5%) was obtained by a synthesis method similar to the synthesis of compound 2 using a resin (120 g) produced by the same method as compound 2-b-resin as a raw material. The values in mass spectrum and retention time in liquid chromatography of the obtained compound 3 were described in Table 5.

### [Synthesis Example 4: Synthesis of compounds 4 to 12]

Peptide extensions were performed by the following basic route according to the peptide synthesis method based on Fmoc method described in International Publication No. WO 2013/100132 or WO2018/225864. Specifically, the steps are the following 5 stages:
1) peptide extension reaction by Fmoc method from the N-terminus of Asp with its side chain carboxylic acid supported on a 2-chlorotrityl resin;
2) peptide cleavage process from the 2-chlorotrityl resin;
3) amide cyclization by condensation of the Asp side chain carboxylic acid resulting from the 2-chlorotrityl resin by the cleavage process and the N-terminal amino group of the peptide chain;
4) deprotection of the protecting group of the side chain functional group contained in the peptide chain; and
5) purification of the compound by preparative HPLC.

All starting materials and reagents were obtained from commercial suppliers or synthesized by use of methods known in the art.

Hereinafter, the synthesis of compounds 4 and 5 will be described in detail. It should be noted that compounds 6 to 12 were also synthesized according to the peptide synthesis method described above.

### <Synthesis of (S)-3-((((9H-fluoren-9-yl)methoxy)carbonyl)amino)-4-oxo-4-(pyrrolidin-1 - yl)butanoic acid-2-chlorotrityl resin (Fmoc-Asp(O-Trt(2-Cl)-Resin)-pyrro)>

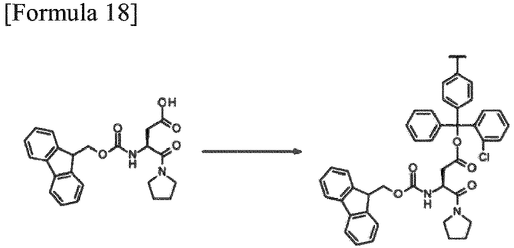

The compound was synthesized by the method described in International Publication No. WO2013/100132 from (S)-3-((((9H-fluoren-9-yl)methoxy)carbonyl)amino)-4-oxo-4-(pyrrolidin-1-yl)butanoic acid.

### <Synthesis of (S)-3-(((9H-fluoren-9-yl)methoxy)carbonyl(methyl)amino)-4-oxo-4-pyrrolidin-1 - ylbutanoic acid-2-chlorotrityl resin (Fmoc-MeAsp(O-Trt(2-Cl)-Resin)-pyrro)>

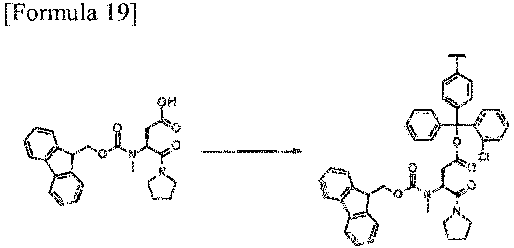

The compound was synthesized by the method described in International Publication No. WO2021/090855 from (S)-3-((((9H-fluoren-9-yl)methoxy)carbonyl(methyl)amino)-4-oxo-4-(pyrrolidin-1-yl)butanoic acid.

### <Synthesis of compound 4>

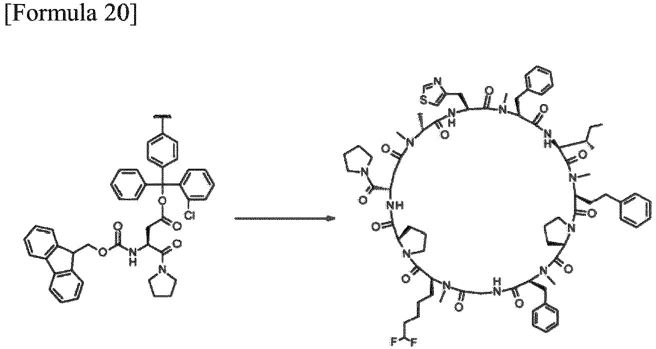

The peptide extension reaction mentioned above using the (S)-3-((((9H-fluoren-9-yl)methoxy)carbonyl)amino)-4-oxo-4-(pyrrolidin-1-yl)butanoic acid-2-chlorotrityl resin (Fmoc-Asp(O-Trt(2-Cl)-Resin)-pyrro) (0.459 mmol/g, 3.2 g, 1.469 mmol) and using Fmoc-Pro-OH, Fmoc-MeHnl(7-F2)-OH, Fmoc-Gly-OH, Fmoc-MePhe-OH, Fmoc-MeHph-OH, Fmoc-Ile-OH, Fmoc-Ala(4-Thz)-OH, and Fmoc-D-MeAla-OH, cleavage of the extended peptide from the resin, cyclization of the cleaved peptide (using O-(7-aza-1H-benzotriazol-1-yl)-N,N,N;N-tetramethyluronium hexafluorophosphate (HATU)) as a cyclization reagent), and purification of the cyclized peptide were performed to obtain compound 4 (570 mg) of interest. The values in mass spectrum and retention time in liquid chromatography of the obtained compound 4 were described in Table 5.

### <Synthesis of compound 5>

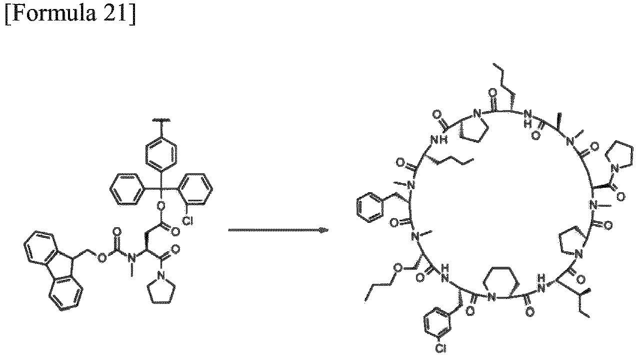

The peptide extension reaction mentioned above using the (S)-3-((((9H-fluoren-9-yl)methoxycarbonyl(methyl)amino)-4-oxo-4-(pyrrolidin-1-yl)butanoic acid-2-chlorotrityl resin (Fmoc-MeAsp(O-Trt(2-Cl)-Resin)-pyrro) (0.470 mmol/g, 1.6 g, 0.752 mmol) and using Fmoc-Pro-OH, Fmoc-Ile-OH, Fmoc-Pic(2)-OH, Fmoc-Phe(3-Cl)-OH, Fmoc-MeSer(nPr)-OH, Fmoc-MePhe-OH, Fmoc-Nle-OH, and Fmoc-D-MeAla-OH, cleavage of the extended peptide from the resin, cyclization of the cleaved peptide (using O-(7-aza-1H-benzotriazol-1-yl)-N,N,N,N-tetramethyluronium hexafluorophosphate (HATU)) as a cyclization reagent), and purification of the cyclized peptide were performed to obtain compound 5 (273 mg) of interest. The values in mass spectrum and retention time in liquid chromatography of the obtained compound 5 were described in Table 5.

**[Table 5]**

| **Compound No.** | **Measurement condition** | **Retention time (minute)** | **MS Found(m/z)** | **MS polarity** |
|---|---|---|---|---|
| Compound 1 | SSC-TFA-07 | 1.637 | 1441.8 | (M+H)+ |
| Compound 2 | SSC-FA-03 | 1.856 | 1453.9 | (M-H)- |
| Compound 3 | SQDFA05 | 1.01 | 1478.3 | (M+H)+ |
| Compound 4 | SQDFA50L | 1.21 | 1445 | (M-H)- |
| Compound 5 | SQDFA05 | 1.08 | 1396 | (M-H)- |
| Compound 6 | SQDAA50 | 0.88 | 1468 | (M+H)+ |
| Compound 7 | SQDFA05 | 1.10 | 1400 | (M+H)+ |
| Compound 8 | SSC-FA-03 | 2.020 | 1451.8 | (M-H)- |
| Compound 9 | SSC-AF-00 | 2.117 | 1359.9 | (M-H)- |
| Compound 10 | SSC-A-FA-01 | 4.709 | 1411.9 | (M-H)- |
| Compound 11 | SQDFA05 | 1.12 | 1577 | (M+H)+ |
| Compound 12 | SQDFA05 | 1.11 | 1613 | (M+H)+ |

The molecular weights of compounds 1 to 12 and cyclosporin A are as follows:
Compound 1: 1442.2 g/mol
Compound 2: 1456.2 g/mol
Compound 3: 1478.2 g/mol
Compound 4: 1446.7 g/mol
Compound 5: 1398.2 g/mol
Compound 6: 1467.9 g/mol
Compound 7: 1399.8 g/mol
Compound 8: 1454.2 g/mol
Compound 9: 1362.1 g/mol
Compound 10: 1414.1 g/mol
Compound 11: 1577.0 g/mol
Compound 12: 1613.4 g/mol
Cyclosporin A: 1202.6 g/mol

ClogP obtained using Daylight Version 4.95 (Daylight Chemical Information Systems, Inc.) in compounds 1 to 12 and cyclosporin A is as follows.
Compound 1: 16.1
Compound 2: 15.1
Compound 3: 14.9
Compound 4: 11.2
Compound 5: 13.8
Compound 6: 13.5
Compound 7: 13.7
Compound 8: 15.9
Compound 9: 14.2
Compound 10: 13.3
Compound 11: 15.1
Compound 12: 14.4
Cyclosporin A: 14.36

### (Evaluation Example 1) Evaluation of Caco-2 membrane permeability

Caco-2 cells were cultured on 96-well Transwell for 3 weeks. The permeability test was then started by adding 10 µM of any of compounds 1 to 12 and a FaSSIF/HBSS buffer (pH 6.5) containing 5 mM of lauroyl-L-carnitine (manufactured by Sigma-Aldrich or by Sinochem Japan Co., Ltd.) to the Apical side and adding an HBSS buffer (pH 7.4) containing 4% BSA to the Basal side. Each well was shaken at 5%CO₂, 37°C, 80 rpm, and at 180 minutes after the start, a sample on the Basal side was taken and the permeation amount of the compound was measured by liquid chromatography-mass spectrometry (LC/MS/MS). From the permeation amount, the permeability coefficient (Caco-2 Papp (cm/sec)) was calculated. The results are shown in Table 6.

As comparative examples, the permeation amount was measured by the same procedure as described above except that a FaSSIF/HBSS buffer (pH 6.5) without 5 mM lauroyl-L-carnitine was used. In other words, on the Apical side, any of compounds 1 to 12 and a FaSSIF/HBSS buffer (pH 6.5) were added. From the measured permeation amount, the permeability coefficient was calculated. The results are shown in Table 6.

**[Table 6]**

| Compound | With 5 mM lauroyl-L-carnitine | Without 5 mM lauroyl-L-carnitine |
|---|---|---|
| | Permeability coefficient (Caco-2 Papp) (cm/sec) | Permeability coefficient (Caco-2 Papp) (cm/sec) |
| Compound 1 | 2.53E-07 | 1.42E-08 |
| Compound 2 | 2.65E-07 | 4.91E-08 |
| Compound 3 | 3.66E-07 | 2.15E-07 |
| Compound 4 | 1.20E-08 | <1.07E-08 |
| Compound 5 | 2.64E-08 | 3.72E-09 |
| Compound 6 | 6.57E-07 | 1.27E-08 |
| Compound 7 | 5.26E-07 | <9.44E-08 |
| Compound 8 | 1.54E-07 | 4.20E-08 |
| Compound 9 | 2.14E-08 | 9.49E-09 |
| Compound 10 | 1.40E-08 | <9.76E-09 |
| Compound 11 | 3.02E-08 | 9.21E-09 |
| Compound 12 | 3.97E-08 | 2.65E-08 |

### (Evaluation Example 2) Evaluation of solubility

Evaluation of the solubility of compounds 1 to 12 was performed. To an excessive amount of lyophilized powder of the compound, 50 mM phosphate buffer (PPB: Phosphate buffer, pH 6.5) was added, and after shaking (37°C, 1 atm, 1800 rpm, for 22 to 24 hours), the mixture was filtered with a filter, and the compound concentration of the filtrate was measured by LC/MS/MS. The measurement conditions for LC/MS/MS are shown in Table 7.
Solubility (µg/mL) was calculated from the measured compound concentration. The results are shown in Table 8.

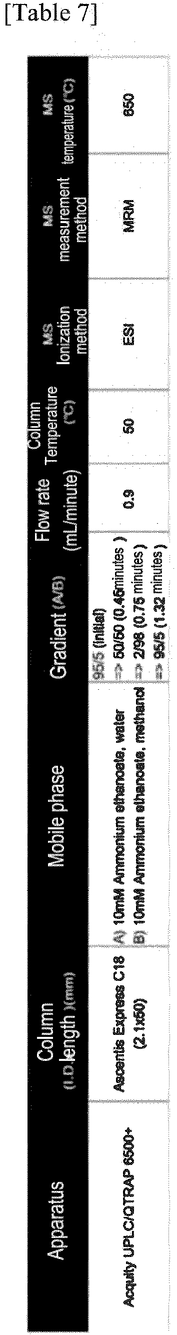

**[Table 8]**

| Compound | PPB solubility (µg/mL) |
|---|---|
| Compound 1 | 1.12 |
| Compound 2 | 1.18 |
| Compound 3 | 1.81 |
| Compound 4 | 102.94 |
| Compound 5 | <1.09 |
| Compound 6 | 20.27 |
| Compound 7 | 5.76 |
| Compound 8 | 1.13 |
| Compound 9 | 9.18 |
| Compound 10 | 101.20 |
| Compound 11 | 16.24 |
| Compound 12 | 99.16 |

### [Example 1] Preparation of absorption-promoting formulation (1)

Compound 1, lauroyl-L-carnitine (manufactured by Sigma-Aldrich Co., Ltd. or by Sinochem Japan Co., Ltd.), water for injection (manufactured by Otsuka Pharmaceutical Factory, Inc.), dimethyl sulfoxide (manufactured by Wako Pure Chemical Co., Ltd.), and cremophor EL (generic name: polyoxyethylene castor oil, the average number of moles of ethylene oxide added is 35) (manufactured by Sigma-Aldrich Co., Ltd., "Kolliphor EL"; the same holds true for the description below unless otherwise specified) were mixed to be the composition of Table 9-1 and stirred to prepare absorption-promoting formulation (1). It should be noted that compound 1 was mixed as a 60 mg/mL solution in dimethyl sulfoxide. Lauroyl-L-carnitine and cremophor EL were added as aqueous solutions, respectively, and mixed.

### [Examples 2 to 62, Comparative Examples 1 to 41, and Production Examples 1 to 19]

### Preparations of absorption-promoting formulations (2) to (62), solution formulations (1) to (41), and iv formulations (1) to (19)

The absorption-promoting formulations (2) to (62), solution formulations (1) to (41), and iv formulations (1) to (19) were prepared in the same manner as in Example 1, except that compounds 1 to 12, a surfactant having a linear alkylene structure and having 5 or more and 13 or less carbon atoms contained in the alkylene structure (lauroyl-L-carnitine, lauroyl-L-carnitine hydrochloride, sodium caprylate, sodium caprate, palmitoyl-L-carnitine hydrochloride, or sodium lauryl sulfate), water for injection or physiological saline (manufactured by Otsuka Pharmaceutical Factory, Inc.), dimethyl sulfoxide, and cremophor EL or Tween 80 (manufactured by Nacalai Tesque, Inc.) were mixed to be the composition of Table 9-1 to Table 9-4. It should be noted that compounds 1 to 12 were mixed as a solution in dimethyl sulfoxide so as to have the concentrations (Compound concentration in dimethyl sulfoxide) shown in Table 9-1 to Table 9-4. Also, in Examples 8 to 48, Comparative Examples 4 to 38, and Production Examples 3 to 19, the surfactant and cremophor EL or Tween 80 were added as a powder or a stock solution, respectively, without forming an aqueous solution, and mixed. Since Tween 80 is more easily metabolized than cremophor EL, use of Tween 80 instead of cremophor EL facilitates measuring more accurate PK profiles because Tween 80 is less likely to interact with the peptide compound used herein.

### [Examples 63 and 64] Preparation of absorption-promoting agents (63) and (64)

Compound 3 and HPMCAS (manufactured by Shin-Etsu Chemical Co., Ltd.) were added to tetrahydrofuran such that the compound 3 and HPMCAS attained the ratio described in Table 9-5 and 12 wt/vol% as a solid concentration to prepare a suspension. This suspension was spray-dried to obtain a solid dispersion. This solid dispersion was mixed with sodium lauryl sulfate (manufactured by BASF SE) or sodium lauryl sulfate and lauroyl-L-carnitine hydrochloride so as to attain the ratio described in Table 9-5 to prepare absorption-promoting agents (63) and (64).

### [Examples 65 and 66] Preparation of absorption-promoting agents (65) and (66)

Propylene glycol monocaprylate (manufactured by Nikko Chemicals Co., Ltd.), cremophor EL (manufactured by BASF SE), and oleic acid (manufactured by NOF Corp.) were mixed so as to attain the ratio described in Table 9-6, and compound 3 was dissolved therein. This solution was encapsulated by an ordinary method to prepare an absorption-promoting agent (65). Also, lauroyl-L-carnitine hydrochloride is encapsulated by an ordinary method to obtain a capsule of lauroyl-L-carnitine hydrochloride. An absorption-promoting agent (66) was prepared using a combination of the capsule of the absorption-promoting agent (65) and the capsule of lauroyl-L-carnitine hydrochloride. The ratio between the components to be combined is as described in Table 9-6.

In Table 9-1 to Table 9-6, (*1) shows a content in 1 mL of the solvent, and (*2) shows a content that occupies 100% by volume of the solvent.

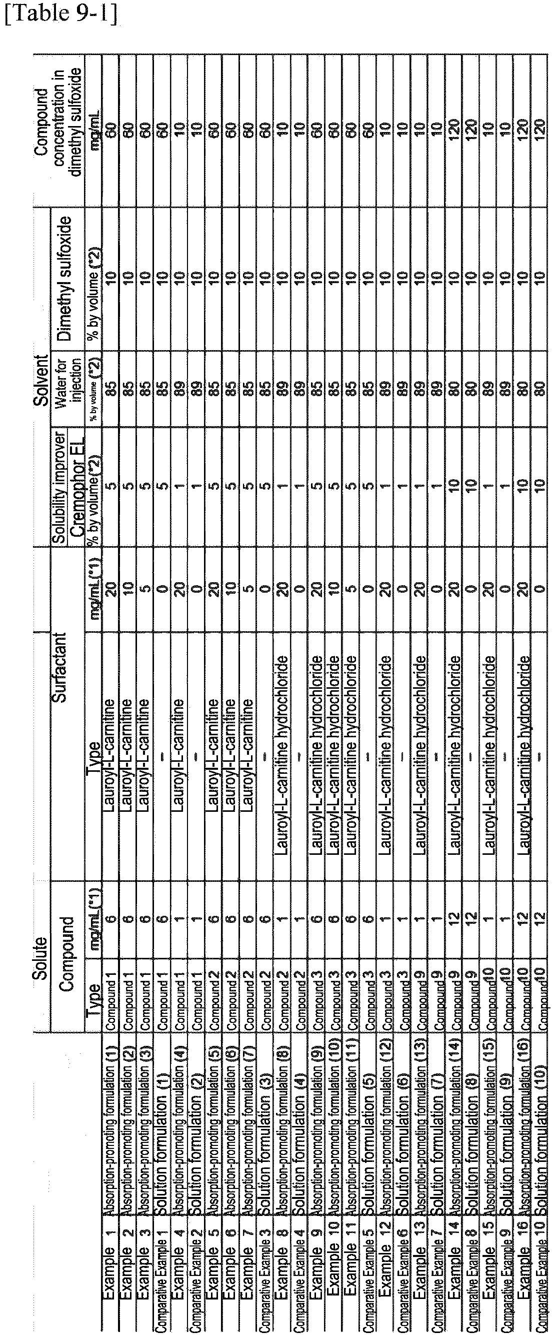

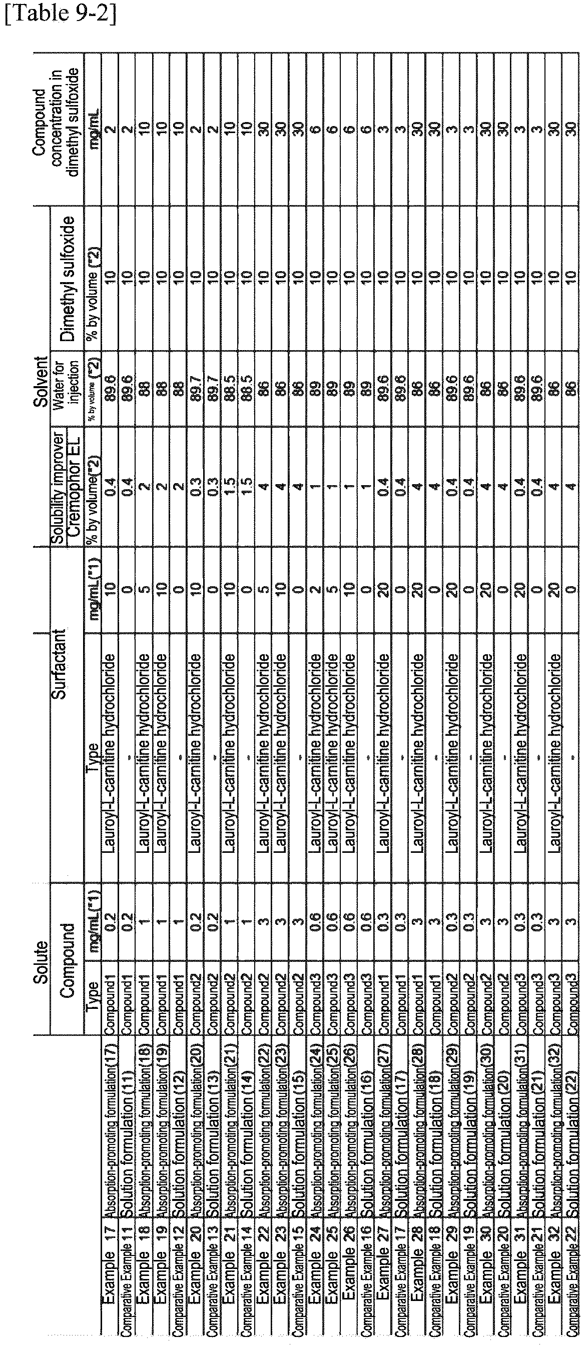

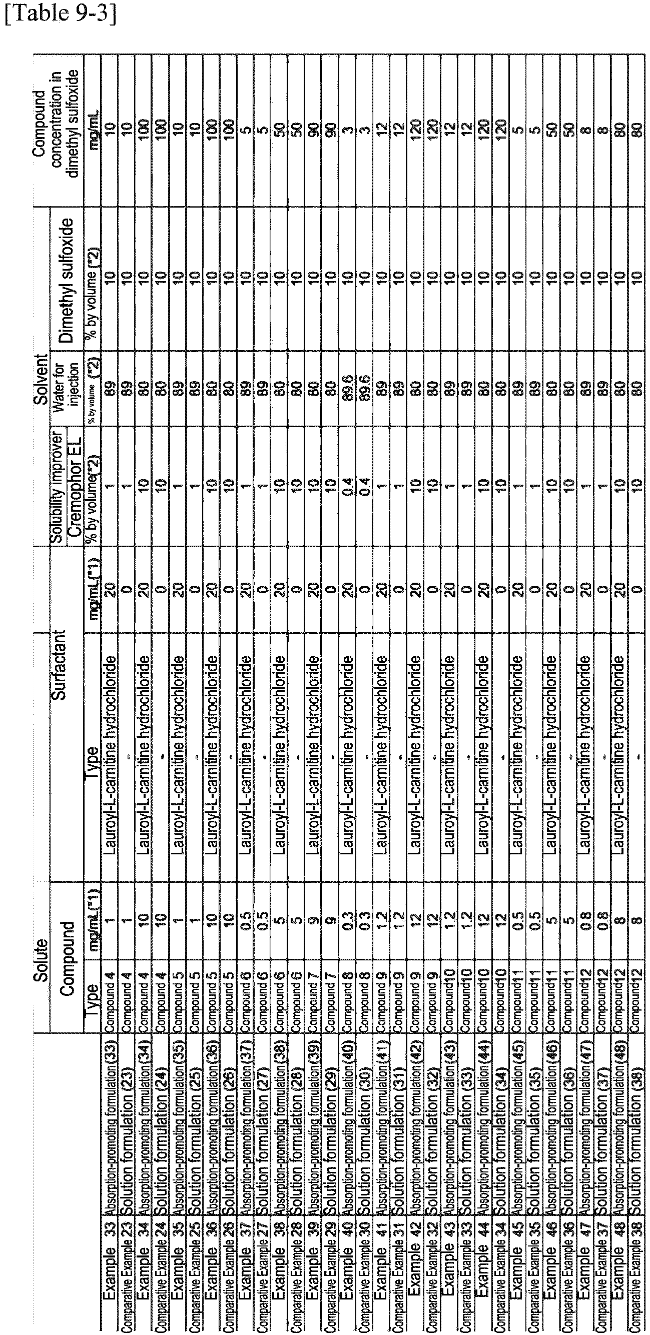

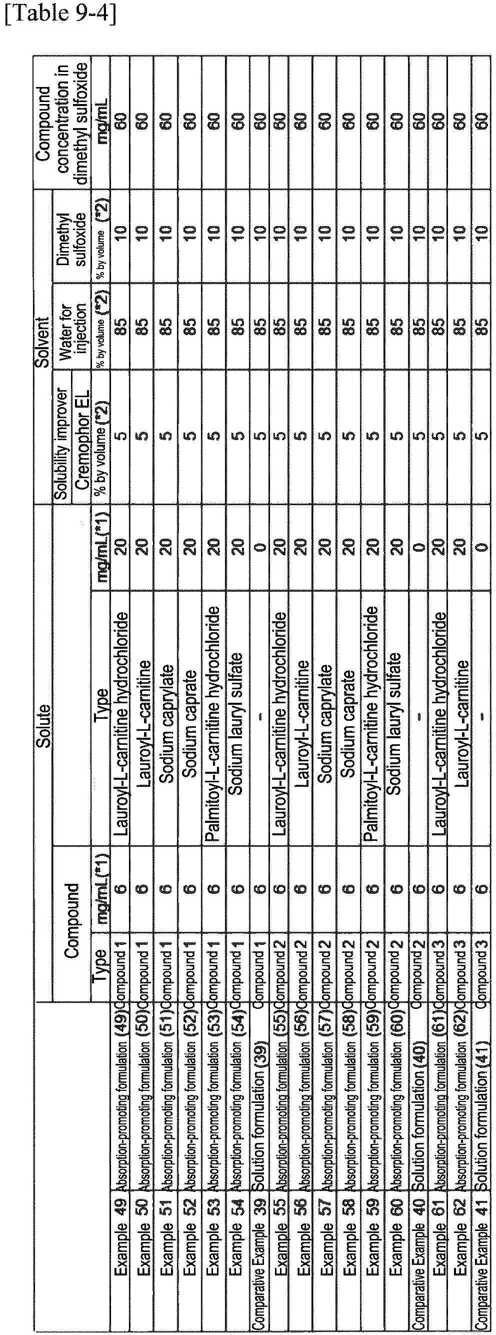

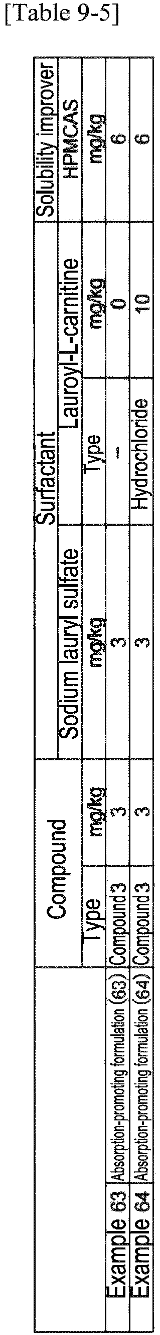

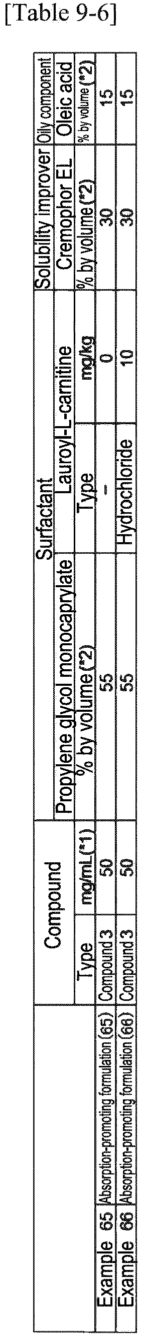

### (Evaluation Example 3) Rat PK test (30 mg/kg)

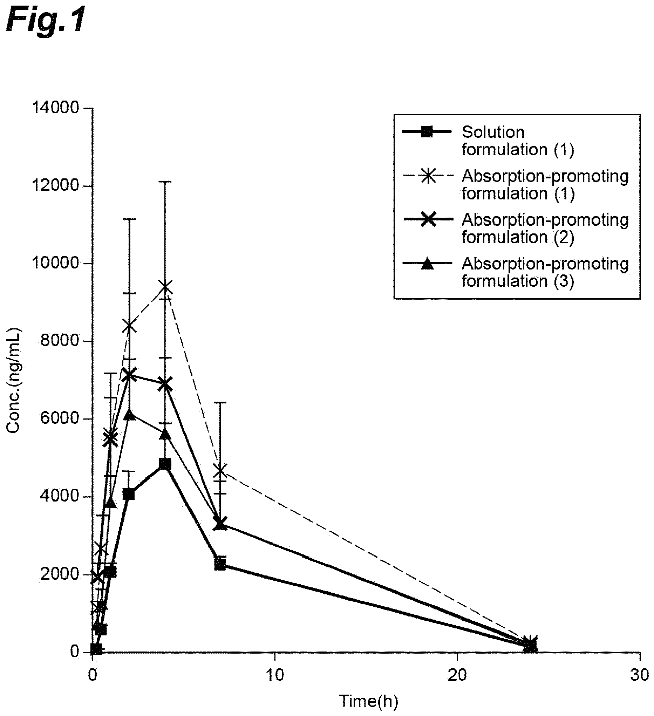

PK profile after oral administration of solution formulation (1) prepared in Comparative Example 1 and absorption-promoting formulations (1) to (3) prepared in Examples 1 to 3 in rats were evaluated. To male rats (WIST, 7 weeks of age, manufactured by Japan SLC, Inc.: 3 rats per group), any one of solution formulation (1) prepared in Comparative Example 1 and absorption-promoting formulations (1) to (3) prepared in Examples 1 to 3 was orally administered at a dose of 30 mg/kg of compound 1, and blood was collected from the jugular vein over time up to 24 hours after administration using a syringe treated with heparin as an anticoagulant. Furthermore, iv formulation (1) prepared in Production Example 1 was administered intravenously at a dose of 1 mg/kg of compound 1, and blood was collected from the jugular vein over time up to 24 hours after administration using a syringe treated with heparin as an anticoagulant. Plasma was separated from the blood by centrifugation, and after deproteinization with acetonitrile, the plasma concentration of compound 1 was measured with an LC/MS/MS device (XEVO TQ-XS, manufactured by Waters). The changes in plasma concentration of each formulation are shown in Figure 1. Furthermore, from the resulting changes in plasma concentration, the pharmacokinetic parameters were calculated by non-compartmental analysis using an analysis software Phoenix WinNonlin 8.2 (manufactured by Certara L.P.). The results are shown in Table 10.

As the pharmacokinetic parameters, area under the plasma concentration-time curve (AUC; ng·h/mL), the highest plasma concentration after oral administration (Cmax; ng/mL), bioavailability (BA), and relative bioavailability (rBA) were calculated. When a plasma concentration was equal to or below the lower limit of quantitation, the concentration was used as 0 ng/mL. For AUC, the area of the values from the time of administration to up to 24 hours was calculated, and then converted in terms of dose of 30 mg/kg from the compound concentration in the actual administration solution. BA was calculated as a ratio of AUC of solution formulation (AUCsol) or AUC of absorption-promoting formulation (AUCLC) to AUC of iv formulation (AUCiv), i.e., AUCsol/AUCiv or AUCLC/AUCiv, when the same compound is administered. rBA was calculated as the ratio (AUCLC/AUCsol) of AUC of absorption-promoting formulation (AUCLC) to AUC of solution formulation (AUCsol) when the same compound is administered. In compound 1, it was confirmed that any AUC in the administration groups of absorption-promoting formulations (1) to (3) of Examples 1 to 3 was greater than AUC in the administration group of solution formulation (1) of Comparative Example 1, and also observed that Cmax increased (Table 10). From this, it was confirmed that, with the use of lauroyl-L-carnitine, the compound of low membrane permeability shows a high absorbability compared to without lauroyl-L-carnitine.

**[Table 10]**

| Pharmacokinetic parameters of each formulation of compound 1 (rat, 30 mg/kg) | | | | |
|---|---|---|---|---|
| Formulation | AUC (ng*h/mL) | Cmax (ng/mL) | BA(%) | rBA (%) |
| Solution formulation (1) | 33800 | 4840 | 23.8 | 100 |
| Absorption-promoting formulation (1) | 74200 | 9410 | 52.3 | 220 |
| Absorption-promoting formulation (2) | 57400 | 7150 | 40.4 | 170 |
| Absorption-promoting formulation (3) | 48100 | 6450 | 33.9 | 142 |

### (Evaluation Example 4) Rat PK test (5 mg/kg)

PK profile after oral administration of solution formulation (2) prepared in Comparative Example 2 and absorption-promoting formulation (4) prepared in Example 4 in rats were evaluated. To male rats (WIST, 7 weeks of age, manufactured by Japan SLC, Inc.: 3 rats per group), solution formulation (2) prepared in Comparative Example 2 or absorption-promoting formulation (4) prepared in Example 4 was orally administered at a dose of 5 mg/kg of compound 1, and blood was collected from the jugular vein over time up to 24 hours after administration using a syringe treated with heparin as an anticoagulant. Plasma was separated from the blood by centrifugation, and after deproteinization with acetonitrile, the plasma concentration of compound 1 was measured with an LC/MS/MS device (XEVO TQ-XS, manufactured by Waters). Furthermore, from the resulting changes in plasma concentration, the pharmacokinetic parameters were calculated by non-compartmental analysis using an analysis software Phoenix WinNonlin 8.2 (manufactured by Certara L.P.). The results are shown in Table 11. As a result, it was confirmed that any AUC in the administration group of absorption-promoting formulation (4) of Example 4 was greater than the AUC in the administration group of solution formulation (2) of Comparative Example 2 (Table 11). From this, it was confirmed that, with the use of lauroyl-L-carnitine, the compound of low membrane-permeability shows a high absorbability compared to without lauroyl-L-carnitine.

**[Table 11]**

| Pharmacokinetic parameters of each formulation of compound 1 (rat, 5 mg/kg) | | |
|---|---|---|
| Formulation | AUC (ng*h/mL) | Cmax (ng/mL) |
| Solution formulation (2) | 219 | 63 |
| Absorption-promoting formulation (4) | 2790 | 398 |

### (Evaluation Example 5) Rat PK test (30 mg/kg)

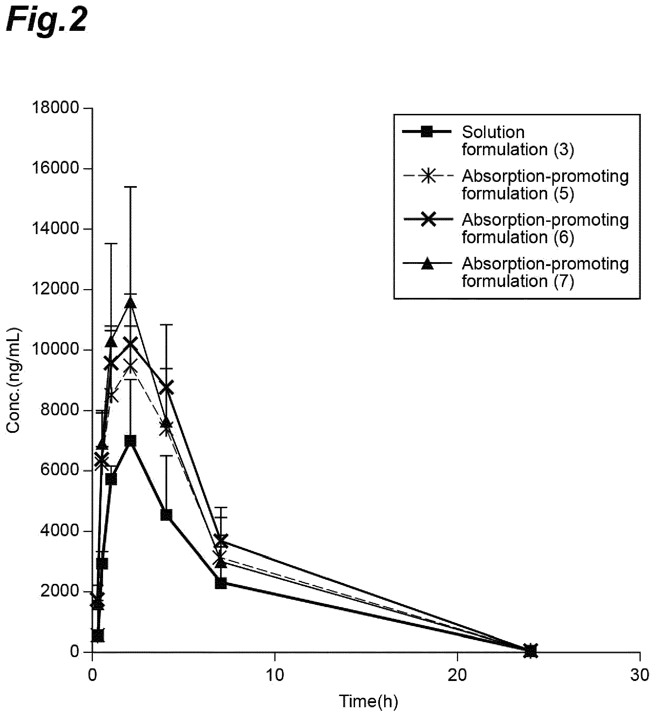

PK profile after oral administration of solution formulation (3) prepared in Comparative Example 3 and absorption-promoting formulations (5) to (7) prepared in Examples 5 to 7 and after intravenous administration of iv formulation (2) prepared in Production Example 2 in rats were evaluated in the same manner as in Evaluation Example 3. The changes in plasma concentration of each formulation are shown in Figure 2. Furthermore, from the resulting changes in plasma concentration, the pharmacokinetic parameters were calculated by the same analysis as in Evaluation Example 3. The results are shown in Table 12. As a result, it was confirmed that any AUC in the administration groups of absorption-promoting formulations (5) to (7) of Examples 5 to 7 was greater than AUC in the administration group of solution formulation (3) of Comparative Example 3, and also observed that Cmax increased (Table 12). From this, it was confirmed that, with the use of lauroyl-L-carnitine, the compound of low membrane-permeability shows a high absorbability compared to without lauroyl-L-carnitine.

**[Table 12]**

| Pharmacokinetic parameters of each formulation of compound 2 (rat, 30 mg/kg) | | | | |
|---|---|---|---|---|
| Formulation | AUC (ng*h/mL) | Cmax (ng/mL) | BA(%) | rBA (%) |
| Solution formulation (3) | 40000 | 7000 | 39.7 | 100 |
| Absorption-promoting formulation (5) | 60100 | 9470 | 59.6 | 150 |
| Absorption-promoting formulation (6) | 68300 | 10300 | 67.8 | 171 |
| Absorption-promoting formulation (7) | 63500 | 11600 | 62.9 | 159 |

### (Evaluation Example 6) Rat PK test (5 mg/kg)

PK profile after oral administration of solution formulation (4) prepared in Comparative Example 4 and absorption-promoting formulation (8) prepared in Example 8 in rats were evaluated in the same manner as in Evaluation Example 4. From the resulting changes in plasma concentration, the pharmacokinetic parameters were calculated by the same analysis as in Evaluation Example 4. The results are shown in Table 13. As a result, it was confirmed that any AUC in the administration group of absorption-promoting formulation (8) of Example 8 was greater than AUC in the administration group of solution formulation (4) of Comparative Example 4, and also observed that Cmax increased (Table 13). From this, it was confirmed that, with the use of lauroyl-L-carnitine, the compound of low membrane-permeability shows a high absorbability compared to without lauroyl-L-carnitine.

**[Table 13]**

| Pharmacokinetic parameters of each formulation of compound 2 (rat, 5 mg/kg) | | |
|---|---|---|
| Formulation | AUC (ng*h/mL) | Cmax (ng/m L) |
| Solution formulation (4) | 748 | 199 |
| Absorption-promoting formulation (8) | 2510 | 559 |

### (Evaluation Example 7) Rat PK test (30 mg/kg)

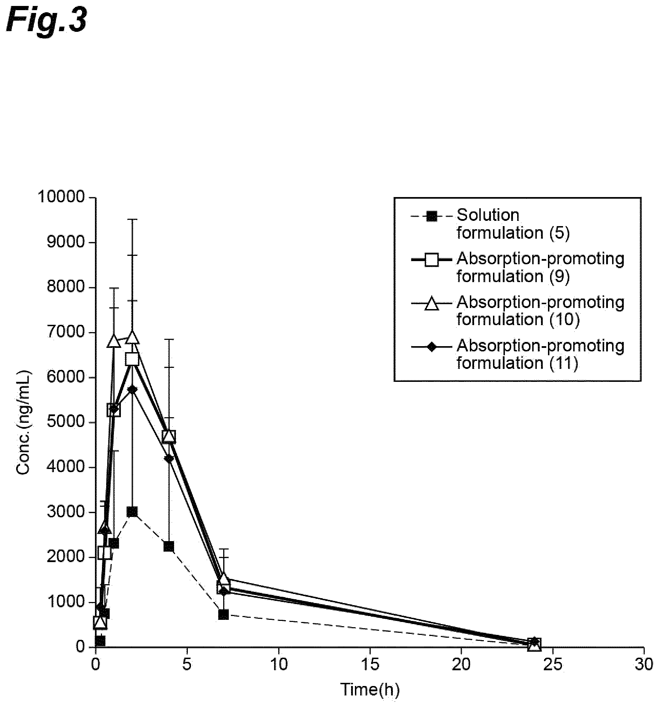

PK profile after oral administration of solution formulation (5) prepared in Comparative Example 5 and absorption-promoting formulations (9) to (11) prepared in Examples 9 to 11 and after intravenous administration of iv formulation (3) prepared in Production Example 3 in rats were evaluated in the same manner as in Evaluation Example 3. The changes in plasma concentration of each formulation are shown in Figure 3. From the resulting changes in plasma concentration, the pharmacokinetic parameters were calculated by the same analysis as in Evaluation Example 3. The results are shown in Table 14. As a result, it was confirmed that any AUC in the administration groups of absorption-promoting formulations (9) to (11) of Examples 9 to 11 was greater than AUC in the administration group of solution formulation (5) of Comparative Example 5, and also observed that Cmax increased (Table 14). From this, it was confirmed that, with the use of lauroyl-L-camitine, the compound of low membrane-permeability shows a high absorbability compared to without lauroyl-L-carnitine.

**[Table 14]**

| Pharmacokinetic parameters of each formulation of compound 3 (rat, 30 mg/kg) | | | |
|---|---|---|---|
| Formulation | AUC (ng*h/mL) | Cmax (ng/mL) BA(%) | rBA (%) |
| Solution formulation (5) | 16600 | 3010 39.0 | 100 |
| Absorption-promoting formulation (9) | 33400 | 5810 78.4 | 201 |
| Absorption-promoting formulation (10) | 37600 | 7130 88.1 | 227 |
| Absorption-promoting formulation (11) | 33700 | 6410 79.1 | 203 |

### (Evaluation Example 8) Rat PK test (5 mg/kg)

PK profile after oral administration of solution formulation (6) prepared in Comparative Example 6 and absorption-promoting formulation (12) prepared in Example 12 in rats were evaluated in the same manner as in Evaluation Example 4. From the resulting changes in plasma concentration, the pharmacokinetic parameters were calculated by the same analysis as in Evaluation Example 4. The results are shown in Table 15. As a result, it was confirmed that AUC in the administration group of absorption-promoting formulation (12) of Example 12 was greater than AUC in the administration group of solution formulation (6) of Comparative Example 6, and also observed that Cmax increased (Table 15). From this, it was confirmed that, with the use of lauroyl-L-camitine, the compound of low membrane-permeability shows a high absorbability compared to without lauroyl-L-carnitine.

**[Table 15]**

| Pharmacokinetic parameters of each formulation of compound 3 (rat, 5 mg/kg) | | |
|---|---|---|
| Formulation | AUC (ng*h/mL) | Cmax (ng/mL) |
| Solution formulation (6) | 686 | 149 |
| Absorption-promoting formulation (12) | 2780 | 591 |

### (Evaluation Example 9) Rat PK test (5 mg/kg)

PK profile after oral administration of solution formulation (7) prepared in Comparative Example 7 and absorption-promoting formulation (13) prepared in Example 13 in rats were evaluated in the same manner as in Evaluation Example 4. From the resulting changes in plasma concentration, the pharmacokinetic parameters were calculated by the same analysis as in Evaluation Example 4. The results are shown in Table 16. As a result, it was confirmed that AUC in the administration group of absorption-promoting formulation (13) of Example 13 was greater than AUC in the administration group of solution formulation (7) of Comparative Example 7, and also observed that Cmax increased (Table 16). From this, it was confirmed that, with the use of lauroyl-L-camitine, the compound of low membrane-permeability shows a high absorbability compared to without lauroyl-L-carnitine.

**[Table 16]**

| Pharmacokinetic parameters of each formulation of compound 9 (rat, 5 mg/kg) | | |
|---|---|---|
| Formulation | AUC (ng*h/mL) | Cmax (ng/mL) |
| Solution formulation (7) | 8.22 | 4.83 |
| Absorption-promoting formulation (13) | 32.4 | 12.8 |

### (Evaluation Example 10) Rat PK test (60 mg/kg)

PK profile after oral administration of solution formulation (8) prepared in Comparative Example 8 and absorption-promoting formulation (14) prepared in Example 14 in rats were evaluated in the same manner as in Evaluation Example 4. From the resulting changes in plasma concentration, the pharmacokinetic parameters were calculated by the same analysis as in Evaluation Example 4. The results are shown in Table 17. As a result, it was confirmed that AUC in the administration group of absorption-promoting formulation (14) of Example 14 was greater than AUC in the administration group of solution formulation (8) of Comparative Example 8, and also observed that Cmax and BA increased (Table 17). From this, it was confirmed that, with the use of lauroyl-L-camitine, the compound of low membrane-permeability shows a high absorbability compared to without lauroyl-L-carnitine.

**[Table 17]**

| Pharmacokinetic parameters of each formulation of compound 9 (rat, 60 mg/kg) | | | |
|---|---|---|---|
| Formulation | AUC (ng*h/mL) | Cmax (ng/mL) | BA (%) |
| Solution formulation (8) | 430 | 226 | 0.567 |
| Absorption-promoting formulation (14) | 5010 | 2040 | 7.46 |

### (Evaluation Example 11) Rat PK test (5 mg/kg)

PK profile after oral administration of solution formulation (9) prepared in Comparative Example 9 and absorption-promoting formulation (15) prepared in Example 15 in rats were evaluated in the same manner as in Evaluation Example 4. From the resulting changes in plasma concentration, the pharmacokinetic parameters were calculated by the same analysis as in Evaluation Example 4. The results are shown in Table 18. As a result, it was confirmed that AUC in the administration group of absorption-promoting formulation (15) of Example 15 was greater than AUC in the administration group of solution formulation (9) of Comparative Example 9, and also observed that Cmax increased (Table 18). From this, it was confirmed that, with the use of lauroyl-L-camitine, the compound of low membrane-permeability shows a high absorbability compared to without lauroyl-L-carnitine.

**[Table 18]**

| Pharmacokinetic parameters of each formulation of compound 10 (rat, 5 mg/kg) | | |
|---|---|---|
| Formulation | AUC (ng*h/mL) | Cmax (ng/mL) |
| Solution formulation (9) | 1.43 | 0.948 |
| Absorption-promoting formulation (15) | 61.8 | 29.5 |

### (Evaluation Example 12) Rat PK test (60 mg/kg)

PK profile after oral administration of solution formulation (10) prepared in Comparative Example 10 and absorption-promoting formulation (16) prepared in Example 16 in rats were evaluated in the same manner as in Evaluation Example 4. From the resulting changes in plasma concentration, the pharmacokinetic parameters were calculated by the same analysis as in Evaluation Example 4. The results are shown in Table 19. As a result, it was confirmed that AUC in the administration group of absorption-promoting formulation (16) of Example 16 was greater than AUC in the administration group of solution formulation (10) of Comparative Example 10, and also observed that Cmax and BA increased (Table 19). From this, it was confirmed that, with the use of lauroyl-L-camitine, the compound of low membrane-permeability shows a high absorbability compared to without lauroyl-L-carnitine.

**[Table 19]**

| Pharmacokinetic parameters of each formulation of compound 10 (rat, 60 mg/kg) | | | |
|---|---|---|---|
| Formulation | AUC (ng*h/mL) | Cmax (ng/mL) | BA (%) |
| Solution formulation (10) | 318 | 236 | 0.354 |
| Absorption-promoting formulation (16) | 8720 | 3850 | 13.0 |

### (Evaluation Example 13) Monkey PK test (1 mg/kg)

PK profile after oral administration of solution formulation (11) prepared in Comparative Example 11 and absorption-promoting formulation (17) prepared in Example 17 in monkeys were evaluated in the same manner as in Evaluation Example 4. From the resulting changes in plasma concentration, the pharmacokinetic parameters were calculated by the same analysis as in Evaluation Example 4. The results are shown in Table 20. As a result, it was confirmed that AUC in the administration group of absorption-promoting formulation (17) of Example 17 was greater than AUC in the administration group of solution formulation (11) of Comparative Example 11, and also observed that Cmax and BA increased (Table 20). Also, the value of CV (standard deviation of AUC / mean of AUC), which indicates variations in AUC of each individual, decreased. From this, it was confirmed that, with the use of lauroyl-L-camitine, the compound of low membrane-permeability shows a high absorbability and suppressed variations in amount of absorption compared to without lauroyl-L-carnitine.

**[Table 20]**

| Pharmacokinetic parameters of each formulation of compound 1 (monkey, 1 mg/kg) | | | | |
|---|---|---|---|---|
| Formulation | AUC (ng*h/mL) | Cmax (ng/mL) | BA (%) | CV (%) |
| Solution formulation (11) | 1280 | 187 | 12.4 | 70.3 |
| Absorption-promoting formulation (17) | 1880 | 291 | 14.0 | 25.0 |

### (Evaluation Example 14) Monkey PK test (5 mg/kg)

PK profile after oral administration of solution formulation (12) prepared in Comparative Example 12 and absorption-promoting formulations (18) and (19) prepared in Examples 18 and 19 in monkeys were evaluated in the same manner as in Evaluation Example 4. From the resulting changes in plasma concentration, the pharmacokinetic parameters were calculated by the same analysis as in Evaluation Example 4. The results are shown in Table 21. As a result, it was confirmed that AUC in the administration groups of absorption-promoting formulations (18) and (19) of Examples 18 and 19 was greater than AUC in the administration group of solution formulation (12) of Comparative Example 12, and also observed that Cmax and BA increased (Table 21). Also, the value of CV decreased. From this, it was confirmed that, with the use of lauroyl- L-carnitine, the compound of low membrane-permeability shows a high absorbability and suppressed variations in amount of absorption compared to without lauroyl-L-carnitine.

**[Table 21]**

| Pharmacokinetic parameters of each formulation of compound 1 (monkey, 5 mg/kg) | | | | |
|---|---|---|---|---|
| Formulation | AUC (ng*h/mL) | Cmax (ng/mL) | BA (%) | CV (%) |
| Solution formulation (12) | 5100 | 767 | 7.58 | 105 |
| Absorption-promoting formulation (18) | 13400 | 1920 | 19.8 | 48.5 |
| Absorption-promoting formulation (19) | 17500 | 2460 | 25.8 | 53.1 |

### (Evaluation Example 15) Monkey PK test (1 mg/kg)

PK profile after oral administration of solution formulation (13) prepared in Comparative Example 13 and absorption-promoting formulation (20) prepared in Example 20 in monkeys were evaluated in the same manner as in Evaluation Example 4. From the resulting changes in plasma concentration, the pharmacokinetic parameters were calculated by the same analysis as in Evaluation Example 4. The results are shown in Table 22. As a result, it was confirmed that AUC in the administration group of absorption-promoting formulation (20) of Example 20 was greater than AUC in the administration group of solution formulation (13) of Comparative Example 13, and also observed that Cmax and BA increased (Table 22). Also, the value of CV decreased. From this, it was confirmed that, with the use of lauroyl-L-camitine, the compound of low membrane-permeability shows a high absorbability and suppressed variations in amount of absorption compared to without lauroyl-L-carnitine.

**[Table 22]**

| Pharmacokinetic parameters of each formulation of compound 2 (monkey, 1 mg/kg) | | | | |
|---|---|---|---|---|
| Formulation | AUC (ng*h/mL) | Cmax (ng/mL) | BA (%) | CV (%) |
| Solution formulation (13) | 1180 | 233 | 29.6 | 94.9 |
| Absorption-promoting formulation (20) | 1680 | 387 | 32.1 | 9.52 |

### (Evaluation Example 16) Monkey PK test (5 mg/kg)

PK profile after oral administration of solution formulation (14) prepared in Comparative Example 14 and absorption-promoting formulation (21) prepared in Example 21 in monkeys were evaluated in the same manner as in Evaluation Example 4. From the resulting changes in plasma concentration, the pharmacokinetic parameters were calculated by the same analysis as in Evaluation Example 4. The results are shown in Table 23. As a result, it was confirmed that AUC in the administration group of absorption-promoting formulation (21) of Example 21 was greater than AUC in the administration group of solution formulation (14) of Comparative Example 14, and also observed that Cmax and BA increased (Table 23). Also, the value of CV decreased. From this, it was confirmed that, with the use of lauroyl-L-carnitine, the compound of low membrane-permeability shows a high absorbability and suppressed variations in amount of absorption compared to without lauroyl-L-carnitine.

**[Table 23]**

| Pharmacokinetic parameters of each formulation of compound 2 (monkey, 5 mg/kg) | | | | |
|---|---|---|---|---|
| Formulation | AUC (ng*h/mL) | Cmax (ng/mL) | BA (%) | CV (%) |
| Solution formulation (14) | 6000 | 1170 | 29.9 | 76.3 |
| Absorption-promoting formulation (21) | 21600 | 3810 | 81.9 | 31.5 |

### (Evaluation Example 17) Monkey PK test (15 mg/kg)

PK profile after oral administration of solution formulation (15) prepared in Comparative Example 15 and absorption-promoting formulations (22) and (23) prepared in Examples 22 and 23 in monkeys were evaluated in the same manner as in Evaluation Example 4. From the resulting changes in plasma concentration, the pharmacokinetic parameters were calculated by the same analysis as in Evaluation Example 4. The results are shown in Table 24. As a result, it was confirmed that AUC in the administration groups of absorption-promoting formulations (22) and (23) of Examples 22 and 23 was greater than AUC in the administration group of solution formulation (15) of Comparative Example 15, and also observed that Cmax and BA increased (Table 24). Also, the value of CV decreased. From this, it was confirmed that, with the use of lauroyl-L-carnitine, the compound of low membrane-permeability shows a high absorbability and suppressed variations in amount of absorption compared to without lauroyl-L-carnitine.

**[Table 24]**

| Pharmacokinetic parameters of each formulation of compound 2 (monkey, 15 mg/kg) | | | | |
|---|---|---|---|---|
| Formulation | AUC (ng*h/mL) | Cmax (ng/mL) | BA (%) | CV (%) |
| Solution formulation (15) | 12700 | 1950 | 16.1 | 101 |
| Absorption-promoting formulation (22) | 23600 | 3340 | 29.9 | 69.1 |
| Absorption-promoting formulation (23) | 48700 | 5880 | 61.8 | 65.7 |

### (Evaluation Example 18) Monkey PK test (3 mg/kg)

PK profile after oral administration of solution formulation (16) prepared in Comparative Example 16 and absorption-promoting formulations (24) to (26) prepared in Examples 24 to 26 in monkeys were evaluated in the same manner as in Evaluation Example 4. From the resulting changes in plasma concentration, the pharmacokinetic parameters were calculated by the same analysis as in Evaluation Example 4. The results are shown in Table 25. As a result, it was confirmed that AUC in the administration groups of absorption-promoting formulations (24) to (26) of Examples 24 to 26 was greater than AUC in the administration group of solution formulation (16) of Comparative Example 16, and also observed that BA increased (Table 25). Also, the value of CV decreased. From this, it was confirmed that, with the use of lauroyl-L-carnitine, the compound of low membrane-permeability shows a high absorbability and suppressed variations in amount of absorption compared to without lauroyl-L-carnitine.

**[Table 25]**

| Pharmacokinetic parameters of each formulation of compound 3 (monkey, 3 mg/kg) | | | |
|---|---|---|---|
| Formulation | AUC (ng*h/mL) | BA (%) | CV (%) |
| Solution formulation (16) | 1130 | 15.8 | 70.8 |
| Absorption-promoting formulation (24) | 1790 | 25.2 | 49.7 |
| Absorption-promoting formulation (25) | 2490 | 34.8 | 51.8 |
| Absorption-promoting formulation (26) | 1790 | 25.3 | 43.0 |

### (Evaluation Example 19) Mouse PK test (3 mg/kg)

PK profile after oral administration of solution formulation (17) prepared in Comparative Example 17 and absorption-promoting formulation (27) prepared in Example 27 in mice were evaluated in the same manner as in Evaluation Example 4. From the resulting changes in plasma concentration, the pharmacokinetic parameters were calculated by the same analysis as in Evaluation Example 4. The results are shown in Table 26. As a result, it was confirmed that AUC in the administration group of absorption-promoting formulation (27) of Example 27 was greater than AUC in the administration group of solution formulation (17) of Comparative Example 17, and also observed that Cmax increased (Table 26). From this, it was confirmed that, with the use of lauroyl-L-camitine, the compound of low membrane-permeability shows a high absorbability compared to without lauroyl-L-carnitine.

**[Table 26]**

| Pharmacokinetic parameters of each formulation of compound 1 (mouse, 3 mg/kg) | | |
|---|---|---|
| Formulation | AUC (ng*h/mL) | Cmax (ng/mL) |
| Solution formulation (17) | 979 | 144 |
| Absorption-promoting formulation (27) | 1630 | 193 |

### (Evaluation Example 20) Mouse PK test (30 mg/kg)

PK profile after oral administration of solution formulation (18) prepared in Comparative Example 18 and absorption-promoting formulation (28) prepared in Example 28 in mice were evaluated in the same manner as in Evaluation Example 4. From the resulting changes in plasma concentration, the pharmacokinetic parameters were calculated by the same analysis as in Evaluation Example 4. The results are shown in Table 27. As a result, it was confirmed that AUC in the administration group of absorption-promoting formulation (28) of Example 28 was greater than AUC in the administration group of solution formulation (18) of Comparative Example 18, and also observed that Cmax and BA increased (Table 27). From this, it was confirmed that, with the use of lauroyl-L-camitine, the compound of low membrane-permeability shows a high absorbability compared to without lauroyl-L-carnitine.

**[Table 27]**

| Pharmacokinetic parameters of each formulation of compound 1 (mouse, 30 mg/kg) | | | |
|---|---|---|---|
| Formulation | AUC (ng*h/mL) | Cmax (ng/mL) | BA (%) |
| Solution formulation (18) | 108000 | 11200 | 60.6 |
| Absorption-promoting formulation (28) | 149000 | 15800 | 83.9 |

### (Evaluation Example 21) Mouse PK test (3 mg/kg)

PK profile after oral administration of solution formulation (19) prepared in Comparative Example 19 and absorption-promoting formulation (29) prepared in Example 29 in mice were evaluated in the same manner as in Evaluation Example 4. From the resulting changes in plasma concentration, the pharmacokinetic parameters were calculated by the same analysis as in Evaluation Example 4. The results are shown in Table 28. As a result, it was confirmed that AUC in the administration group of absorption-promoting formulation (29) of Example 29 was greater than AUC in the administration group of solution formulation (19) of Comparative Example 19, and also observed that Cmax increased (Table 28). From this, it was confirmed that, with the use of lauroyl-L-camitine, the compound of low membrane-permeability shows a high absorbability compared to without lauroyl-L-carnitine.

**[Table 28]**

| Pharmacokinetic parameters of each formulation of compound 2 (mouse, 3 mg/kg) | | |
|---|---|---|
| Formulation | AUC (ng*h/mL) | Cmax (ng/mL) |
| Solution formulation (19) | 890 | 193 |
| Absorption-promoting formulation (29) | 1800 | 293 |

### (Evaluation Example 22) Mouse PK test (30 mg/kg)

PK profile after oral administration of solution formulation (20) prepared in Comparative Example 20 and absorption-promoting formulation (30) prepared in Example 30 in mice were evaluated in the same manner as in Evaluation Example 4. From the resulting changes in plasma concentration, the pharmacokinetic parameters were calculated by the same analysis as in Evaluation Example 4. The results are shown in Table 29. As a result, it was confirmed that AUC in the administration group of absorption-promoting formulation (30) of Example 30 was greater than AUC in the administration group of solution formulation (20) of Comparative Example 20, and also observed that Cmax and BA increased (Table 29). From this, it was confirmed that, with the use of lauroyl-L-carnitine, the compound of low membrane-permeability shows a high absorbability compared to without lauroyl-L-carnitine.

**[Table 29]**

| Pharmacokinetic parameters of each formulation of compound 2 (mouse, 30 mg/kg) | | | |
|---|---|---|---|
| Formulation | AUC (ng*h/mL) | Cmax (ng/mL) | BA (%) |
| Solution formulation (20) | 56000 | 6610 | 71.9 |
| Absorption-promoting formulation (30) | 109000 | 13400 | 140 |

### (Evaluation Example 23) Mouse PK test (3 mg/kg)

PK profile after oral administration of solution formulation (21) prepared in Comparative Example 21 and absorption-promoting formulation (31) prepared in Example 31 in mice were evaluated in the same manner as in Evaluation Example 4. From the resulting changes in plasma concentration, the pharmacokinetic parameters were calculated by the same analysis as in Evaluation Example 4. The results are shown in Table 30. As a result, it was confirmed that AUC in the administration group of absorption-promoting formulation (31) of Example 31 was greater than AUC in the administration group of solution formulation (21) of Comparative Example 21, and also observed that Cmax increased (Table 30). From this, it was confirmed that, with the use of lauroyl-L-camitine, the compound of low membrane-permeability shows a high absorbability compared to without lauroyl-L-carnitine.

**[Table 30]**

| Pharmacokinetic parameters of each formulation of compound 3 (mouse, 3 mg/kg) | | |
|---|---|---|
| Formulation | AUC (ng*h/mL) | Cmax (ng/mL) |
| Solution formulation (21) | 1150 | 154 |
| Absorption-promoting formulation (31) | 3230 | 437 |

### (Evaluation Example 24) Mouse PK test (30 mg/kg)

PK profile after oral administration of solution formulation (22) prepared in Comparative Example 22 and absorption-promoting formulation (32) prepared in Example 32 in mice were evaluated in the same manner as in Evaluation Example 4. From the resulting changes in plasma concentration, the pharmacokinetic parameters were calculated by the same analysis as in Evaluation Example 4. The results are shown in Table 31. As a result, it was confirmed that AUC in the administration group of absorption-promoting formulation (32) of Example 32 was greater than AUC in the administration group of solution formulation (22) of Comparative Example 22, and also observed that Cmax and BA increased (Table 31). From this, it was confirmed that, with the use of lauroyl-L-camitine, the compound of low membrane-permeability shows a high absorbability compared to without lauroyl-L-carnitine.

**[Table 31]**

| Pharmacokinetic parameters of each formulation of compound 3 (mouse, 30 mg/kg) | | | |
|---|---|---|---|
| Formulation | AUC (ng*h/mL) | Cmax (ng/mL) | BA (%) |
| Solution formulation (22) | 42500 | 5900 | 74.2 |
| Absorption-promoting formulation (32) | 77900 | 8120 | 136 |

### (Evaluation Example 25) Mouse PK test (10 mg/kg)

PK profile after oral administration of solution formulation (23) prepared in Comparative Example 23 and absorption-promoting formulation (33) prepared in Example 33 in mice were evaluated in the same manner as in Evaluation Example 4. From the resulting changes in plasma concentration, the pharmacokinetic parameters were calculated by the same analysis as in Evaluation Example 4. The results are shown in Table 32. As a result, it was confirmed that AUC in the administration group of absorption-promoting formulation (33) of Example 33 was greater than AUC in the administration group of solution formulation (23) of Comparative Example 23, and also observed that Cmax and BA increased (Table 32). From this, it was confirmed that, with the use of lauroyl-L-camitine, the compound of low membrane-permeability shows a high absorbability compared to without lauroyl-L-carnitine.

**[Table 32]**

| Pharmacokinetic parameters of each formulation of compound 4 (mouse, 10 mg/kg) | | | |
|---|---|---|---|
| Formulation | AUC (ng*h/mL) | Cmax (ng/mL) | BA (%) |
| Solution formulation (23) | 57.8 | 13.8 | 0.340 |
| Absorption-promoting formulation (33) | 308 | 112 | 0.968 |

### (Evaluation Example 26) Mouse PK test (100 mg/kg)

PK profile after oral administration of solution formulation (24) prepared in Comparative Example 24 and absorption-promoting formulation (34) prepared in Example 34 in mice were evaluated in the same manner as in Evaluation Example 4. From the resulting changes in plasma concentration, the pharmacokinetic parameters were calculated by the same analysis as in Evaluation Example 4. The results are shown in Table 33. As a result, it was confirmed that AUC in the administration group of absorption-promoting formulation (34) of Example 34 was greater than AUC in the administration group of solution formulation (24) of Comparative Example 24, and also observed that Cmax and BA increased (Table 33). From this, it was confirmed that, with the use of lauroyl-L-camitine, the compound of low membrane-permeability shows a high absorbability compared to without lauroyl-L-carnitine.

**[Table 33]**

| Pharmacokinetic parameters of each formulation of compound 4 (mouse, 100 mg/kg) | | | |
|---|---|---|---|
| Formulation | AUC (ng*h/mL) | Cmax (ng/mL) | BA (%) |
| Solution formulation (24) | 1160 | 445 | 0.420 |
| Absorption-promoting formulation (34) | 5020 | 1780 | 3.37 |

### (Evaluation Example 27) Mouse PK test (10 mg/kg)

PK profile after oral administration of solution formulation (25) prepared in Comparative Example 25 and absorption-promoting formulation (35) prepared in Example 35 in mice were evaluated in the same manner as in Evaluation Example 4. From the resulting changes in plasma concentration, the pharmacokinetic parameters were calculated by the same analysis as in Evaluation Example 4. The results are shown in Table 34. As a result, it was confirmed that AUC in the administration group of absorption-promoting formulation (35) of Example 35 was greater than AUC in the administration group of solution formulation (25) of Comparative Example 25, and also observed that Cmax increased (Table 34). From this, it was confirmed that, with the use of lauroyl-L-camitine, the compound of low membrane-permeability shows a high absorbability compared to without lauroyl-L-carnitine.

**[Table 34]**

| Pharmacokinetic parameters of each formulation of compound 5 (mouse, 10 mg/kg) | | |
|---|---|---|
| Formulation | AUC (ng*h/mL) | Cmax (ng/mL) |
| Solution formulation (25) | 117 | 38.9 |
| Absorption-promoting formulation (35) | 850 | 263 |

### (Evaluation Example 28) Mouse PK test (100 mg/kg)

PK profile after oral administration of solution formulation (26) prepared in Comparative Example 26 and absorption-promoting formulation (36) prepared in Example 36 in mice were evaluated in the same manner as in Evaluation Example 4. From the resulting changes in plasma concentration, the pharmacokinetic parameters were calculated by the same analysis as in Evaluation Example 4. The results are shown in Table 35. As a result, it was confirmed that AUC in the administration group of absorption-promoting formulation (36) of Example 36 was greater than AUC in the administration group of solution formulation (26) of Comparative Example 26, and also observed that Cmax and BA increased (Table 35). From this, it was confirmed that, with the use of lauroyl-L-camitine, the compound of low membrane-permeability shows a high absorbability compared to without lauroyl-L-carnitine.

**[Table 35]**

| Pharmacokinetic parameters of each formulation of compound 5 (mouse, 100 mg/kg) | | | |
|---|---|---|---|
| Formulation | AUC (ng*h/mL) | Cmax (ng/mL) | BA (%) |
| Solution formulation (26) | 7920 | 2720 | 4.98 |
| Absorption-promoting formulation (36) | 20100 | 4080 | 12.6 |

### (Evaluation Example 29) Mouse PK test (5 mg/kg)

PK profile after oral administration of solution formulation (27) prepared in Comparative Example 27 and absorption-promoting formulation (37) prepared in Example 37 in mice were evaluated in the same manner as in Evaluation Example 4. From the resulting changes in plasma concentration, the pharmacokinetic parameters were calculated by the same analysis as in Evaluation Example 4. The results are shown in Table 36. As a result, it was confirmed that AUC in the administration group of absorption-promoting formulation (37) of Example 37 was greater than AUC in the administration group of solution formulation (27) of Comparative Example 27, and also observed that Cmax increased (Table 36). From this, it was confirmed that, with the use of lauroyl-L-camitine, the compound of low membrane-permeability shows a high absorbability compared to without lauroyl-L-carnitine.

**[Table 36]**

| Pharmacokinetic parameters of each formulation of compound 6 (mouse, 5 mg/kg) | | |
|---|---|---|
| Formulation | AUC (ng*h/mL) | Cmax (ng/mL) |
| Solution formulation (27) | 1030 | 229 |
| Absorption-promoting formulation (37) | 1350 | 236 |

### (Evaluation Example 30) Mouse PK test (50 mg/kg)

PK profile after oral administration of solution formulation (28) prepared in Comparative Example 28 and absorption-promoting formulation (38) prepared in Example 38 in mice were evaluated in the same manner as in Evaluation Example 4. From the resulting changes in plasma concentration, the pharmacokinetic parameters were calculated by the same analysis as in Evaluation Example 4. The results are shown in Table 37. As a result, it was confirmed that AUC in the administration group of absorption-promoting formulation (38) of Example 38 was greater than AUC in the administration group of solution formulation (28) of Comparative Example 28, and also observed that Cmax and BA increased (Table 37). From this, it was confirmed that, with the use of lauroyl-L-camitine, the compound of low membrane-permeability shows a high absorbability compared to without lauroyl-L-carnitine.

**[Table 37]**

| Pharmacokinetic parameters of each formulation of compound 6 (mouse, 50 mg/kg) | | | |
|---|---|---|---|
| Formulation | AUC (ng*h/mL) | Cmax (ng/mL) | BA (%) |
| Solution formulation (28) | 37000 | 6470 | 40.5 |
| Absorption-promoting formulation (38) | 65300 | 10200 | 71.7 |

### (Evaluation Example 31) Mouse PK test (90 mg/kg)

PK profile after oral administration of solution formulation (29) prepared in Comparative Example 29 and absorption-promoting formulation (39) prepared in Example 39 in mice were evaluated in the same manner as in Evaluation Example 4. From the resulting changes in plasma concentration, the pharmacokinetic parameters were calculated by the same analysis as in Evaluation Example 4. The results are shown in Table 38. As a result, it was confirmed that AUC in the administration group of absorption-promoting formulation (39) of Example 39 was greater than AUC in the administration group of solution formulation (29) of Comparative Example 29, and also observed that Cmax and BA increased (Table 38). From this, it was confirmed that, with the use of lauroyl-L-camitine, the compound of low membrane-permeability shows a high absorbability compared to without lauroyl-L-carnitine.

**[Table 38]**

| Pharmacokinetic parameters of each formulation of compound 7 (mouse, 90 mg/kg) | | | |
|---|---|---|---|
| Formulation | AUC (ng*h/mL) | Cmax (ng/mL) | BA (%) |
| Solution formulation (29) | 132000 | 15300 | 58.6 |
| Absorption-promoting formulation (39) | 195000 | 20800 | 82.7 |

### (Evaluation Example 32) Mouse PK test (3 mg/kg)

PK profile after oral administration of solution formulation (30) prepared in Comparative Example 30 and absorption-promoting formulation (40) prepared in Example 40 in mice were evaluated in the same manner as in Evaluation Example 4. From the resulting changes in plasma concentration, the pharmacokinetic parameters were calculated by the same analysis as in Evaluation Example 4. The results are shown in Table 39. As a result, it was confirmed that AUC in the administration group of absorption-promoting formulation (40) of Example 40 was greater than AUC in the administration group of solution formulation (30) of Comparative Example 30, and also observed that Cmax increased (Table 39). From this, it was confirmed that, with the use of lauroyl-L-camitine, the compound of low membrane-permeability shows a high absorbability compared to without lauroyl-L-carnitine.

**[Table 39]**

| Pharmacokinetic parameters of each formulation of compound 8 (mouse, 3 mg/kg) | | |
|---|---|---|
| Formulation | AUC (ng*h/mL) | Cmax (ng/mL) |
| Solution formulation (30) | 2200 | 293 |
| Absorption-promoting formulation (40) | 3850 | 466 |

### (Evaluation Example 33) Mouse PK test (12 mg/kg)

PK profile after oral administration of solution formulation (31) prepared in Comparative Example 31 and absorption-promoting formulation (41) prepared in Example 41 in mice were evaluated in the same manner as in Evaluation Example 4. From the resulting changes in plasma concentration, the pharmacokinetic parameters were calculated by the same analysis as in Evaluation Example 4. The results are shown in Table 40. As a result, it was confirmed that AUC in the administration group of absorption-promoting formulation (41) of Example 41 was greater than AUC in the administration group of solution formulation (31) of Comparative Example 31, and also observed that Cmax increased (Table 40). From this, it was confirmed that, with the use of lauroyl-L-camitine, the compound of low membrane-permeability shows a high absorbability compared to without lauroyl-L-carnitine.

**[Table 40]**

| Pharmacokinetic parameters of each formulation of compound 9 (mouse, 12 mg/kg) | | |
|---|---|---|
| Formulation | AUC (ng*h/mL) | Cmax (ng/mL) |
| Solution formulation (31) | 155 | 42.0 |
| Absorption-promoting formulation (41) | 728 | 228 |

### (Evaluation Example 34) Mouse PK test (120 mg/kg)

PK profile after oral administration of solution formulation (32) prepared in Comparative Example 32 and absorption-promoting formulation (42) prepared in Example 42 in mice were evaluated in the same manner as in Evaluation Example 4. From the resulting changes in plasma concentration, the pharmacokinetic parameters were calculated by the same analysis as in Evaluation Example 4. The results are shown in Table 41. As a result, it was confirmed that AUC in the administration group of absorption-promoting formulation (42) of Example 42 was greater than AUC in the administration group of solution formulation (32) of Comparative Example 32, and also observed that Cmax and BA increased (Table 41). From this, it was confirmed that, with the use of lauroyl-L-carnitine, the compound of low membrane-permeability shows a high absorbability compared to without lauroyl-L-carnitine.

**[Table 41]**

| Pharmacokinetic parameters of each formulation of compound 9 (mouse, 120 mg/kg) | | | |
|---|---|---|---|
| Formulation | AUC (ng*h/mL) | Cmax (ng/mL) | BA (%) |
| Solution formulation (32) | 17600 | 6420 | 4.18 |
| Absorption-promoting formulation (42) | 40300 | 10400 | 9.56 |

### (Evaluation Example 35) Mouse PK test (12 mg/kg)

PK profile after oral administration of solution formulation (33) prepared in Comparative Example 33 and absorption-promoting formulation (43) prepared in Example 43 in mice were evaluated in the same manner as in Evaluation Example 4. From the resulting changes in plasma concentration, the pharmacokinetic parameters were calculated by the same analysis as in Evaluation Example 4. The results are shown in Table 42. As a result, it was confirmed that AUC in the administration group of absorption-promoting formulation (43) of Example 43 was greater than AUC in the administration group of solution formulation (33) of Comparative Example 33, and also observed that Cmax and BA increased (Table 42). From this, it was confirmed that, with the use of lauroyl-L-camitine, the compound of low membrane-permeability shows a high absorbability compared to without lauroyl-L-camitine.

**[Table 42]**

| Pharmacokinetic parameters of each formulation of compound 10 (mouse, 12 mg/kg) | | | |
|---|---|---|---|
| Formulation | AUC (ng*h/mL) | Cmax (ng/mL) | BA (%) |
| Solution formulation (33) | 27.9 | 10.8 | 0.187 |
| Absorption-promoting formulation (43) | 251 | 107 | 1.69 |

### (Evaluation Example 36) Mouse PK test (120 mg/kg)

PK profile after oral administration of solution formulation (34) prepared in Comparative Example 34 and absorption-promoting formulation (44) prepared in Example 44 in mice were evaluated in the same manner as in Evaluation Example 4. From the resulting changes in plasma concentration, the pharmacokinetic parameters were calculated by the same analysis as in Evaluation Example 4. The results are shown in Table 43. As a result, it was confirmed that AUC in the administration group of absorption-promoting formulation (44) of Example 44 was greater than AUC in the administration group of solution formulation (34) of Comparative Example 34, and also observed that Cmax and BA increased (Table 43). From this, it was confirmed that, with the use of lauroyl-L-camitine, the compound of low membrane-permeability shows a high absorbability compared to without lauroyl-L-carnitine.

**[Table 43]**

| Pharmacokinetic parameters of each formulation of compound 10 (mouse, 120 mg/kg) | | | |
|---|---|---|---|
| Formulation | AUC (ng*h/mL) | Cmax (ng/mL) | BA (%) |
| Solution formulation (34) | 572 | 297 | 0.386 |
| Absorption-promoting formulation (44) | 20600 | 7810 | 13.9 |

### (Evaluation Example 37) Mouse PK test (5 mg/kg)

PK profile after oral administration of solution formulation (35) prepared in Comparative Example 35 and absorption-promoting formulation (45) prepared in Example 45 in mice were evaluated in the same manner as in Evaluation Example 4. From the resulting changes in plasma concentration, the pharmacokinetic parameters were calculated by the same analysis as in Evaluation Example 4. The results are shown in Table 44. As a result, it was confirmed that AUC in the administration group of absorption-promoting formulation (45) of Example 45 was greater than AUC in the administration group of solution formulation (35) of Comparative Example 35, and also observed that Cmax and BA increased (Table 44). From this, it was confirmed that, with the use of lauroyl-L-camitine, the compound of low membrane-permeability shows a high absorbability compared to without lauroyl-L-camitine.

**[Table 44]**

| Pharmacokinetic parameters of each formulation of compound 11 (mouse, 5 mg/kg) | | | |
|---|---|---|---|
| Formulation | AUC (ng*h/mL) | Cmax (ng/mL) | BA (%) |
| Solution formulation (35) | 18.4 | 6.32 | 0.102 |
| Absorption-promoting formulation (45) | 1510 | 648 | 8.39 |

### (Evaluation Example 38) Mouse PK test (50 mg/kg)

PK profile after oral administration of solution formulation (36) prepared in Comparative Example 36 and absorption-promoting formulation (46) prepared in Example 46 in mice were evaluated in the same manner as in Evaluation Example 4. From the resulting changes in plasma concentration, the pharmacokinetic parameters were calculated by the same analysis as in Evaluation Example 4. The results are shown in Table 45. As a result, it was confirmed that AUC in the administration group of absorption-promoting formulation (46) of Example 46 was greater than AUC in the administration group of solution formulation (36) of Comparative Example 36, and also observed that Cmax and BA increased (Table 45). From this, it was confirmed that, with the use of lauroyl-L-camitine, the compound of low membrane-permeability shows a high absorbability compared to without lauroyl-L-camitine.

**[Table 45]**

| Pharmacokinetic parameters of each formulation of compound 11 (mouse, 50 mg/kg) | | | |
|---|---|---|---|
| Formulation | AUC (ng*h/mL) | Cmax (ng/mL) | BA (%) |
| Solution formulation (36) | 10500 | 3000 | 5.82 |
| Absorption-promoting formulation (46) | 52000 | 12800 | 32.5 |

### (Evaluation Example 39) Mouse PK test (8 mg/kg)

PK profile after oral administration of solution formulation (37) prepared in Comparative Example 37 and absorption-promoting formulation (47) prepared in Example 47 in mice were evaluated in the same manner as in Evaluation Example 4. From the resulting changes in plasma concentration, the pharmacokinetic parameters were calculated by the same analysis as in Evaluation Example 4. The results are shown in Table 46. As a result, it was confirmed that AUC in the administration group of absorption-promoting formulation (47) of Example 47 was greater than AUC in the administration group of solution formulation (37) of Comparative Example 37, and also observed that Cmax and BA increased (Table 46). From this, it was confirmed that, with the use of lauroyl-L-carnitine, the compound of low membrane-permeability shows a high absorbability compared to without lauroyl-L-camitine.

**[Table 46]**

| Pharmacokinetic parameters of each formulation of compound 12 (mouse, 8 mg/kg) | | | |
|---|---|---|---|
| Formulation | AUC (ng*h/mL) | Cmax (ng/mL) | BA (%) |
| Solution formulation (37) | 640 | 298 | 2.06 |
| Absorption-promoting formulation (47) | 1350 | 604 | 4.32 |

### (Evaluation Example 40) Mouse PK test (80 mg/kg)

PK profile after oral administration of solution formulation (38) prepared in Comparative Example 38 and absorption-promoting formulation (48) prepared in Example 48 in mice were evaluated in the same manner as in Evaluation Example 4. From the resulting changes in plasma concentration, the pharmacokinetic parameters were calculated by the same analysis as in Evaluation Example 4. The results are shown in Table 47. As a result, it was confirmed that AUC in the administration group of absorption-promoting formulation (48) of Example 48 was greater than AUC in the administration group of solution formulation (38) of Comparative Example 38, and also observed that Cmax and BA increased (Table 47). From this, it was confirmed that, with the use of lauroyl-L-carnitine, the compound of low membrane-permeability shows a high absorbability compared to without lauroyl-L-camitine.

**[Table 47]**

| Pharmacokinetic parameters of each formulation of compound 12 (mouse, 80 mg/kg) | | | |
|---|---|---|---|
| Formulation | AUC (ng*h/mL) | Cmax (ng/mL) | BA (%) |
| Solution formulation (38) | 71700 | 19300 | 23.1 |
| Absorption-promoting formulation (48) | 103000 | 21800 | 33.2 |

### (Evaluation Example 41) Rat PK test (30 mg/kg)

PK profile after oral administration of solution formulation (39) prepared in Comparative Example 39 and absorption-promoting formulations (49) to (54) prepared in Examples 49 to 54 in rats were evaluated in the same manner as in Evaluation Example 4. From the resulting changes in plasma concentration, the pharmacokinetic parameters were calculated by the same analysis as in Evaluation Example 4. The results are shown in Table 48. As a result, it was confirmed that AUC in the administration groups of absorption-promoting formulations (49) to (54) of Examples 49 to 54 was greater than AUC in the administration group of solution formulation (39) of Comparative Example 39, and also observed that Cmax increased (Table 48). From this, it was confirmed that, with the use of a surfactant having a linear alkylene structure and having 5 or more and 13 or less carbon atoms in the alkylene structure, a high absorbability compared to without these surfactants is shown. It was confirmed that, particularly, with the use of lauroyl-L-camitine among these surfactants, the compound of low membrane-permeability shows a particularly high absorbability compared to without lauroyl-L-carnitine.

**[Table 48]**

| Pharmacokinetic parameter of formulation of compound 1 (rat, 30 mg/kg) | | |
|---|---|---|
| Formulation | AUC (ng*h/mL) | Cmax (ng/mL) |
| Solution formulation (39) | 25200 | 4320 |
| Absorption-promoting formulation (49) | 58700 | 9520 |
| Absorption-promoting formulation (50) | 56600 | 8060 |
| Absorption-promoting formulation (51) | 37000 | 6140 |
| Absorption-promoting formulation (52) | 31100 | 4880 |
| Absorption-promoting formulation (53) | 41500 | 6520 |
| Absorption-promoting formulation (54) | 38900 | 7240 |

### (Evaluation Example 42) Rat PK test (30 mg/kg)

PK profile after oral administration of solution formulation (40) prepared in Comparative Example 40 and absorption-promoting formulations (55) to (60) prepared in Examples 55 to 60 in rats were evaluated in the same manner as in Evaluation Example 4. From the resulting changes in plasma concentration, the pharmacokinetic parameters were calculated by the same analysis as in Evaluation Example 4. The results are shown in Table 49. As a result, it was confirmed that AUC in the administration groups of absorption-promoting formulations (55) to (60) of Examples 55 to 60 was greater than AUC in the administration group of solution formulation (40) of Comparative Example 40, and the values of Cmax were also equivalent or more (Table 49). From this, it was confirmed that, with the use of a surfactant having a linear alkylene structure and having 5 or more and 13 or less carbon atoms in the alkylene structure, a high absorbability compared to without these surfactants is shown. It was confirmed that, particularly, with the use of lauroyl-L-camitine among these surfactants, the compound of low membrane-permeability shows a particularly high absorbability compared to without lauroyl-L-carnitine.

**[Table 49]**

| Pharmacokinetic parameter of formulation of compound 2 (rat, 30 mg/kg) | | |
|---|---|---|
| Formulation | AUC (ng*h/mL) | Cmax (ng/mL) |
| Solution formulation (40) | 30800 | 5850 |
| Absorption-promoting formulation (55) | 43800 | 8040 |
| Absorption-promoting formulation (56) | 54300 | 9400 |
| Absorption-promoting formulation (57) | 34000 | 7460 |
| Absorption-promoting formulation (58) | 33400 | 5820 |
| Absorption-promoting formulation (59) | 33800 | 6170 |
| Absorption-promoting formulation (60) | 32300 | 6000 |

### (Evaluation Example 43) Rat PK test (30 mg/kg)

PK profile after oral administration of solution formulation (41) prepared in Comparative Example 41 and absorption-promoting formulations (61) and (62) prepared in Examples 61 and 62 in rats were evaluated in the same manner as in Evaluation Example 4. From the resulting changes in plasma concentration, the pharmacokinetic parameters were calculated by the same analysis as in Evaluation Example 4. The results are shown in Table 50. As a result, it was confirmed that AUC in the administration groups of absorption-promoting formulations (61) and (62) of Examples 61 and 62 was greater than AUC in the administration group of solution formulation (41) of Comparative Example 41, and also observed that Cmax increased (Table 50). From this, it was confirmed that, with the use of lauroyl-L-camitine, the compound of low membrane-permeability shows a high absorbability compared to without lauroyl-L-carnitine.

**[Table 50]**

| Pharmacokinetic parameter of formulation of compound 3 (rat, 30 mg/kg) | | |
|---|---|---|
| Formulation | AUC (ng*h/mL) | Cmax (ng/mL) |
| Solution formulation (41) | 17800 | 3660 |
| Absorption-promoting formulation (61) | 26500 | 5030 |
| Absorption-promoting formulation (62) | 31400 | 6690 |

### (Evaluation Example 44) Monkey PK test (3 mg/kg)

Absorption-promoting formulations (63) and (64) prepared in Examples 63 and 64 were respectively placed in separate capsulates, and PK profile after oral administration in monkeys were evaluated in the same manner as in Evaluation Example 4. From the resulting changes in plasma concentration, the pharmacokinetic parameters were calculated by the same analysis as in Evaluation Example 4. The results are shown in Table 51. As a result, sufficient AUC, Cmax, and BA were confirmed in the administration group of absorption-promoting agent (63) of Example 63. Further, it was confirmed that AUC in the administration group of absorption-promoting formulation (64) of Example 64 was greater than AUC in the administration group of absorption-promoting agent (63) of Example 63, and also observed that Cmax and BA further increased (Table 51). Also, the value of CV, which indicates variations in AUC of each individual, decreased. From this, it was confirmed that, with the use of a surfactant having a linear alkylene structure and having 5 or more and 13 or less carbon atoms in the alkylene structure, a sufficiently high absorbability is shown. Further, it was confirmed that, with the use of lauroyl-L-camitine, the compound of low membrane-permeability shows a higher absorbability and more suppressed variations in amount of absorption compared to without lauroyl-L-camitine.

**[Table 51]**

| Pharmacokinetic parameter of formulation of compound 3 (monkey, 3 mg/kg) | | | | |
|---|---|---|---|---|
| Formulation | AUC (ng*h/mL) | Cmax (ng/mL) | BA (%) | CV (%) |
| Absorption-promoting formulation (63) | 1230 | 222 | 17.2 | 86.2 |
| Absorption-promoting formulation (64) | 2870 | 602 | 40.2 | 42.2 |

### (Evaluation Example 45) Monkey PK test (3 mg/kg)

Two capsules containing absorption-promoting formulation (65) prepared in Example 65 were provided. In evaluation of Example 65, only one of the capsules was orally administered to a monkey, while in evaluation of Example 66, a total of two capsules, one of the capsules and one capsule containing lauroyl-L-camitine hydrochloride, were orally administered at the same time to a monkey, and PK profile after oral administration in monkeys were evaluated in the same manner as in Evaluation Example 4. The ratio between the absorption-promoting agent (65) and lauroyl-L-camitine hydrochloride in the total weight of 3 mg of these components in the evaluation of Example 66 is as shown in Table 9-6. From the resulting changes in plasma concentration, the pharmacokinetic parameters were calculated by the same analysis as in Evaluation Example 4. The results are shown in Table 52. As a result, sufficient AUC, Cmax, and BA were confirmed in the administration group of absorption-promoting agent (65) of Example 65. Further, it was confirmed that AUC in the administration group of absorption-promoting formulation (66) of Example 66 was greater than AUC in the administration group of absorption-promoting agent (65) of Example 65, and also observed that Cmax and BA further increased (Table 52). Also, the value of CV, which indicates variations in AUC of each individual, decreased. From this, it was confirmed that, with the use of a surfactant having a linear alkylene structure and having 5 or more and 13 or less carbon atoms in the alkylene structure, a sufficiently high absorbability is shown. Further, it was confirmed that, with the use of lauroyl-L-camitine, the compound of low membrane-permeability shows a higher absorbability and more suppressed variations in amount of absorption compared to without lauroyl-L-camitine.

**[Table 52]**

| Pharmacokinetic parameter of formulation of compound 3 (monkey, 3 mg/kg) | | | | |
|---|---|---|---|---|
| Formulation | AUC (ng*h/mL) | Cmax (ng/mL) | BA (%) | CV (%) |
| Absorption-promoting formulation (65) | 1820 | 465 | 25.6 | 54.4 |
| Absorption-promoting formulation (66) | 2840 | 725 | 39.6 | 44.4 |

Although the reason why the absorbability of the peptide compound is improved by the addition of lauroyl-L-camitine is uncertain, it is presumed that the absorbability is improved both in promoting absorption via the paracellular pathway by expanding the tight junction and in promoting absorption via the transcellular pathway by improving the flowability of the cell membrane.

In other words, since it has been reported that lauroyl-L-camitine suppresses the expression of claudin protein and thus expands the tight junction (Drug Metab. Pharmacokinet., 26 (2): 162170 (2011)), it is believed that lauroyl-L-camitine assists the intracellular uptake of the peptide compounds through the tight junction. Furthermore, since lauroyl-L-camitine has a function as a surfactant, it is believed that it makes the peptide compound easier to pass through the cell membrane by pulling phospholipids, lipid-soluble components present in the cell membrane, out and making the cell membrane surface a "sparse" state, or by entering into phospholipid membranes.

However, it is known that even when lauroyl-L-camitine is added to other peptides, the bioavailability of the other peptides remains only about one order of magnitude% (Pharmaceutics, 2019, 11(1), 41). In contrast, as shown in the present Examples, when lauroyl-L-carnitine is added to the peptide compound according to the present invention, the bioavailability is greatly improved by the range of about tens of percent. From this, it can be understood that the combination of the peptide compound according to the present invention and the surfactant according to the present invention exhibits a particularly remarkable effect on the membrane permeability and absorbability of the peptide compound. It is also possible that a special mechanism of action other than described above, which has not yet been found, is working. Likewise, it can be understood that combined use of the peptide compound according to the present invention and lauroyl-L-camitine produces distinct effects in view of conventional techniques, because variations in the amount of absorption of the peptide compound are suppressed by the addition of lauroyl-L-carnitine.

## Claims

1. A composition comprising a component (1) below, for combined use of a component (2) below:
(1) at least one or more peptide compounds selected from the group consisting of (i), (ii), and (iii) below:
(i) a peptide compound containing one or more N-substituted amino acid residues,
(ii) a peptide compound having ClogP of 4 or more and 25 or less, and
(iii) a peptide compound having a solubility of 10 mg/mL or less at 37°C and 1 atm in 50 mM phosphate buffer (pH 6.5); and
(2) at least one or more surfactants selected from the group consisting of (iv) and (v) below:
(iv) a surfactant having a linear alkylene structure and having 5 or more and 13 or less carbon atoms in the alkylene structure, and
(v) a surfactant having a carnitine residue.

2. The composition according to claim 1, further comprising (3) a solubility improver.

3. The composition according to claim 2, wherein a content of the solubility improver is 0.05% by volume or more and 100% by volume or less based on 100% by volume of a liquid component contained in the composition.

4. The composition according to claim 2 or 3, wherein the solubility improver contains a polyoxyethylene structure.

5. The composition according to any one of claims 2 to 4, wherein the solubility improver is a polyoxyethylene castor oil.

6. The composition according to any one of claims 1 to 5, wherein an amount of the surfactant to be used in combination is 0.05 parts by mass or more and 300 parts by mass or less based on 1 part by mass of the peptide compound.

7. The composition according to any one of claims 1 to 6, wherein a substituent on a nitrogen atom of the N-substituted amino acid residue is a C₁-C₆ alkyl group.

8. The composition according to any one of claims 1 to 7, wherein the peptide compound is a cyclic peptide compound.

9. The composition according to any one of claims 1 to 8, wherein a molecular weight of the peptide compound is 5,000 g/mol or less.

10. The composition according to any one of claims 1 to 9, wherein the surfactant is represented by any one of general formulae (a1) to (a3) below: 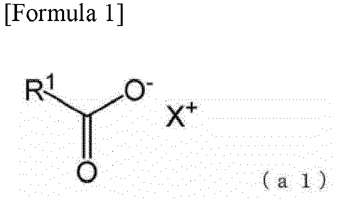 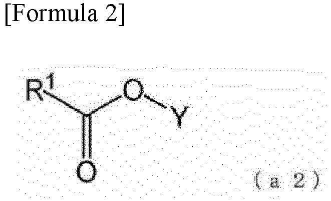 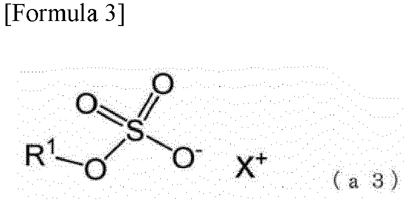
wherein R¹ represents an optionally substituted, saturated or unsaturated, linear alkyl group having 5 or more and 13 or less carbon atoms, X represents sodium or potassium, and Y represents a group represented by formula (a4) below or a stereoisomer thereof: 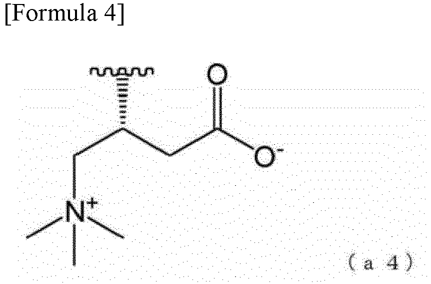
wherein 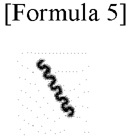 represents a bond.

11. The composition according to any one of claims 1 to 10, wherein the surfactant is a medium-chain fatty acid ester, a sodium salt of medium-chain fatty acid, or a potassium salt of medium-chain fatty acid.

12. The composition according to any one of claims 1 to 11, wherein the surfactant is acylcarnitine.

13. The composition according to any one of claims 1 to 12, wherein the surfactant is lauroyl-L-camitine.

14. A method for improving absorbability of a peptide compound (1) below, comprising
using in combination a composition containing the peptide compound (1) below and a surfactant (2) below:
(1) at least one or more peptide compounds selected from the group consisting of (i), (ii), and (iii) below:
(i) a peptide compound containing one or more N-substituted amino acid residues,
(ii) a peptide compound having ClogP of 4 or more and 25 or less, and
(iii) a peptide compound having a solubility of 10 mg/mL or less at 37°C and 1 atm in 50 mM phosphate buffer (pH 6.5); and
(2) at least one or more surfactants selected from the group consisting of (iv) and (v) below:
(iv) a surfactant having a linear alkylene structure and having 5 or more and 13 or less carbon atoms in the alkylene structure, and
(v) a surfactant having a carnitine residue.

15. A method for improving absorbability of a peptide compound (1) below, comprising
using in combination the peptide compound (1) below and a surfactant (2) below:
(1) at least one or more peptide compounds selected from the group consisting of (i), (ii), and (iii) below:
(i) a peptide compound containing one or more N-substituted amino acid residues,
(ii) a peptide compound having ClogP of 4 or more and 25 or less, and
(iii) a peptide compound having a solubility of 10 mg/mL or less at 37°C and 1 atm in 50 mM phosphate buffer (pH 6.5); and
(2) at least one or more surfactants selected from the group consisting of (iv) and (v) below:
(iv) a surfactant having a linear alkylene structure and having 5 or more and 13 or less carbon atoms in the alkylene structure, and
(v) a surfactant having a carnitine residue.
